# EUROPEAN PATENT SPECIFICATION

(11) **EP 4 003 427 B1**
(45) Date of publication and mention of the grant of the patent: **17.09.2025**
(21) Application number: 20746624.4
(22) Date of filing: 24.07.2020
(51) Int. Cl.: A61K 31/56, A61K 31/704, A61K 31/7056

(54) **SAPONIN DERIVATIVES WITH IMPROVED THERAPEUTIC WINDOW**
SAPONINDERIVATE MIT VERBESSERTEM THERAPEUTISCHEM FENSTER
DÉRIVÉS DE SAPONINE À FENÊTRE THÉRAPEUTIQUE AMÉLIORÉE

(30) Priority: 25.07.2019 NL 2023568; 09.12.2019 WO PCT/EP2019/084210; 09.12.2019 WO PCT/EP2019/084290; 09.12.2019 WO PCT/EP2019/084292; 24.06.2020 NL 2025904
(43) Date of publication of application: 01.06.2022
(60) Divisional application: 25195993.8
(73) Proprietor: Sapreme Technologies B.V., 3584 CM Utrecht (NL)
(72) Inventor: POSTEL, Ruben, 3584 CM Utrecht (NL); HERMANS, Guy, 9820 Merelbeke (BE); FUCHS, Hendrik, 13353 Berlin (DE)
(74) Representative: Nederlandsch Octrooibureau
(86) International application number: PCT/EP2020/071045
(87) International publication number: WO 2021/014019

(56) References cited:
- WO-A1-2015/184451
- WO-A1-2018/057031
- WO-A1-2019/011914
- WO-A1-93/05789
- WO-A2-2004/092329
- HENDRIK FUCHS ET AL: "Glycosylated Triterpenoids as Endosomal Escape Enhancers in Targeted Tumor Therapies", BIOMEDICINES, vol. 5, no. 2, 29 March 2017 (2017-03-29), pages 14, XP055429235, DOI: 10.3390/biomedicines5020014

## Description

### TECHNOLOGICAL FIELD

The invention relates to a saponin derivative based on a saponin comprising a triterpene aglycone and a first saccharide chain and/or a second saccharide chain, and comprising: an aglycone core structure comprising an aldehyde group which has been derivatised; and/or the first saccharide chain wherein the first saccharide chain comprises a carboxyl group, which has been derivatised; and/or the second saccharide chain wherein the second saccharide chain comprises at least one acetoxy group which has been derivatised. The invention also relates to a first pharmaceutical composition comprising the saponin derivative of the invention. In addition, the invention relates to a pharmaceutical combination comprising the first pharmaceutical composition of the invention and a second pharmaceutical composition comprising any one or more of an antibody-toxin conjugate, a receptor-ligand - toxin conjugate, an antibody-drug conjugate, a receptor-ligand - drug conjugate, an antibody-oligonucleotide conjugate or a receptor-ligand - oligonucleotide conjugate. The invention also relates to the first pharmaceutical composition or the pharmaceutical combination of the invention, for use as a medicament, or use in the treatment or prophylaxis of a cancer, an infectious disease, viral infection, hypercholesterolemia, primary hyperoxaluria, haemophilia A, haemophilia B, alpha-1 antitrypsin related liver disease, acute hepatic porphyria, transthyretin-mediated amyloidosis, or an auto-immune disease. Furthermore, the invention relates to an *in vitro* or *ex vivo* method for transferring a molecule from outside a cell to inside said cell, comprising contacting said cell with the molecule and with a saponin derivative of the invention.

### BACKGROUND OF THE INVENTION

Targeted tumor therapy is a cancer treatment that uses drugs to target specific genes and proteins that are involved in the growth and survival of cancer cells. Immunotoxins, which are targeted toxins that contain an antibody as targeting moiety, are very promising because they combine the specificity of an antibody against tumor-specific antigens, which enables them to channel the toxin to the aimed point of action, and can introduce additionally cell killing mechanisms such as antibody-dependent cell-mediated cytotoxicity and complement-dependent cytotoxicity. To exhibit its effect, the toxin needs to be released into the cytosol after internalization. A major drawback is that the targeting moiety which bears the payload is often not fully internalized, directly recycled to the surface after internalization, or degraded in lysosomes, therewith hampering the sufficient delivery of the payload into the cell cytosol. To ensure a toxic payload concentration for tumor cells and to overcome insufficient cytosolic entry, high serum levels of the targeted toxin are required often resulting in severe side effects, in particular including immunogenicity and vascular leak syndrome. Thus, a sufficiently wide therapeutic window remains a concern when treating cancer patients with antibody-drug conjugates (ADCs).

WO 93/05789 discloses saponin/antigen conjugates based on purified Quillaja saponins such as QA-7, QA-17, QA-18, and QA-21 and the use thereof for enhancing immune responses in individuals.

To cope with the drawback of insufficient cytosolic entry, several strategies were developed relating to for example the redirection of toxins to endogenous cellular membrane transport complexes of the biosynthetic pathway, disruption of endosomes, attenuation of the membrane integrity of endosomal membranes, or use of cell penetrating peptides.

For example, glycosylated triterpenes such as saponins were found to act as endosomal escape enhancers for targeted toxins, such as ribosome-inactivating proteins (RIPs), in tumor therapy. Structural-activity relationship analysis of saponins revealed that the presence of the following core structural elements appear to be beneficial for the ability of saponins to enhance the cytotoxicity of RIPs (see Formula (I) with X¹ = H or OH and X² = a polysaccharide moiety):
- a branched tri-saccharide at C-3 containing a glucuronic acid
- an aldehyde at C-4
- a carboxy group at C-28
- a polysaccharide moiety (R²) linked to the C-28 position of at least four sugar moieties containing an acetyl group.

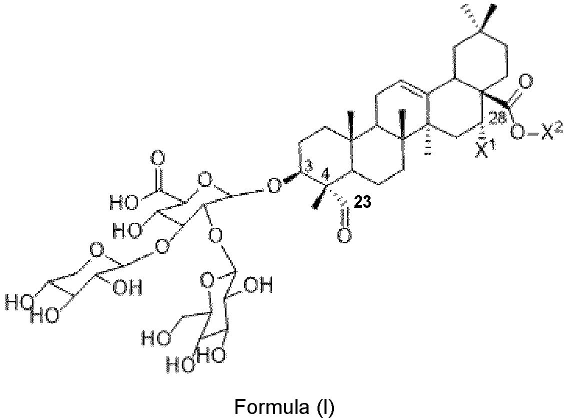

Especially, SO1861 (Formula II, sometimes also referred to as SPT001), a triterpenoid saponin, was identified as a potent molecule in order to enhance the endosomal escape of tumor-cell targeted toxins. A dual effect for the enhancer mechanism is postulated: first, a direct increase of the endosomal escape resulting in caspase-dependent apoptosis that is, second, combined with lysosomal-mediated cell death pathways, which are triggered after the release of cathepsins and other hydrolytic enzymes following destruction of lysosomal membranes.

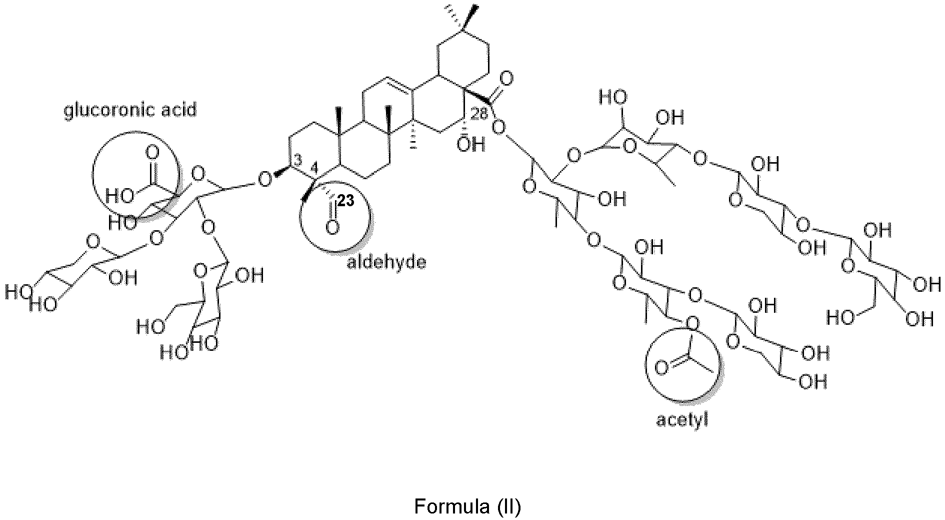

The application of saponins as endosomal escape enhancers is based on the recognition that these saponins have the ability to rupture erythrocyte membranes. However, at the very same time, cell rupturing activity of saponins contribute to (the risk for) side effects when a subject is treated with such saponins, therewith influencing optimal therapeutic windows in view of limiting therapeutic index. Indeed, toxicity of such saponins, extracellularly and/or intracellularly, when administered to a patient in need of anti-tumor therapy, is of concern when for example the optimal dosing regimen and route and frequency of administration are considered.

All characteristics of the chemical composition of the saponins themselves, including the structure of the triterpene backbone, a pentacyclic C30 terpene skeleton (also known as sapogenin or aglycone), number and length of saccharide side chains as well as type and linkage variants of the sugar residues linked to the backbone, contribute to the hemolytic potential and/or cytotoxicity of such saponins.

The saponins are per se not target-specific when the endosome and the cytosol of cells are considered, and saponins expectedly and most often distribute in a (human) subject with other kinetics than the targeted toxins, even when the same route of administration would be considered. Thus, after application to a patient in need thereof of a therapeutic combination comprising e.g. an ADC and a saponin, the saponin molecules can be found in any organ connoting that specificity is only mediated by the targeted toxin. Distribution of saponins in the whole body requires higher concentrations for a successful treatment when compared to specific accumulation in target cells. Hence, the toxicity of the modified saponins needs to be low enough for a successful application in view of the systemic application of saponins in the body, in order to achieve a suitable therapeutic window.

Therefore, there is a still a need to improve the therapeutic index when co-administration of a saponin together with e.g. an ADC is considered: need for better controlling (or better: lower) the cytotoxicity of saponins while at the same time maintaining sufficient efficacy when potentiation of the cytotoxic effect of an ADC is considered.

### SUMMARY

Surprisingly, the inventors have found that modified saponins, i.e. saponin derivatives, having
- a branched tri-saccharide moiety bound at C-3 of the aglycone of the saponin and containing a modified glucuronic acid; and/or
- a modified aldehyde at C-4 of the aglycone of the saponin; and/or
- a polysaccharide moiety bound at C-28 position of the aglycone of the saponin, and containing a modified acetoxy group in said polysaccharide moiety;
have a reduced toxicity when cell viability is considered of cells contacted with the saponin derivatives, have activity when potentiation of e.g. toxin cytotoxicity or BNA mediated gene silencing is considered (without wishing to be bound by any theory: relating to similar or improved endosomal escape enhancing activity of the modified saponin) and/or have reduced hemolytic activity, when compared with the toxicity, activity and haemolytic activity of unmodified saponin. Therewith, the inventors provide saponin derivatives according to claim 1 with an improved therapeutic window, since the ratio between IC50 values for cell toxicity and e.g. toxin potentiation or gene silencing is increased, and/or since the ratio between IC50 values for saponin haemolytic activity and e.g. toxin potentiation or gene silencing is increased.

A first aspect of the invention relates to a saponin derivative according to claim 1. Said saponin derivative is based on a SO1861 saponin comprising a triterpene aglycone core structure and a first saccharide chain and a second saccharide chain linked to the aglycone core structure, wherein the saponin derivative is a SO1861 derivative having a quillaic acid aglycone core structure, wherein the saponin derivative corresponds to the saponin represented by Molecule 1, wherein the aglycone core structure of Molecule 1 is quillaic acid and R is defined as hydroxyl; wherein the first saccharide chain A1 is Gal-(1→2)-[Xyl-(1→3)]-GlcA- and the second saccharide chain A2 is Glc-(1→3)-Xyl-(1→4)-Rha-(1→2)-[Xyl-(1→3)-4-OAc-Qui-(1→4)]-Fuc-;
and wherein:
i. the saponin derivative comprises said aglycone core structure comprising an aldehyde group at position C23 of the quillaic acid which has been derivatised; or
ii. the saponin derivative comprises the first saccharide chain A1 wherein the first saccharide chain comprises a carboxyl group of a glucuronic acid moiety, which has been derivatised; or
iii. the saponin derivative comprises the second saccharide chain A2 wherein the second saccharide chain comprises an acetoxy (Me(CO)O-) group which has been derivatised; or
iv. the saponin derivative comprises any combination of derivatisations i., ii. and iii., preferably any combinations of two derivatisations i., ii. and iii..

The saponin derivative according to the invention is a bidesmosidic triterpene glycoside.

A second aspect of the invention relates to a first pharmaceutical composition comprising the saponin derivative according to the invention and optionally a pharmaceutically acceptable excipient and/or diluent.

A third aspect of the invention relates to a pharmaceutical combination comprising:
- the first pharmaceutical composition of the invention; and
- a second pharmaceutical composition comprising any one or more of an antibody-toxin conjugate, a receptor-ligand - toxin conjugate, an antibody-drug conjugate, a receptor-ligand - drug conjugate, an antibody-oligonucleotide conjugate or a receptor-ligand - oligonucleotide conjugate, and optionally comprising a pharmaceutically acceptable excipient and/or diluent.

A fourth aspect of the invention relates to a third pharmaceutical composition comprising the saponin derivative of the invention and further comprising any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate, and optionally comprising a pharmaceutically acceptable excipient and/or diluent.

A fifth aspect of the invention relates to the first pharmaceutical composition of the invention, the pharmaceutical combination of the invention or the third pharmaceutical composition of the invention, for use as a medicament.

A sixth aspect of the invention relates to the first pharmaceutical composition of the invention, the pharmaceutical combination of the invention or the third pharmaceutical composition of the invention, for use in the treatment or prophylaxis of a cancer, an infectious disease, viral infection, hypercholesterolemia, primary hyperoxaluria, haemophilia A, haemophilia B, alpha-1 antitrypsin related liver disease, acute hepatic porphyria, transthyretin-mediated amyloidosis, or an auto-immune disease.

A seventh aspect of the invention relates to an *in vitro* or ex *vivo* method for transferring a molecule from outside a cell to inside said cell, preferably into the cytosol of said cell, comprising the steps of:
a) providing a cell;
b) providing the molecule for transferring from outside the cell into the cell provided in step a);
c) providing a saponin derivative according to the invention;
d) contacting the cell of step a) *in vitro* or *ex vivo* with the molecule of step b) and the saponin derivative of step c), therewith establishing the transfer of the molecule from outside the cell into said cell.

### DEFINITIONS

The term "saponin" has its regular scientific meaning and here refers to a group of amphipatic glycosides which comprise one or more hydrophilic glycone moieties combined with a lipophilic aglycone core which is a sapogenin. The saponin may be naturally occurring or synthetic (i.e. non-naturally occurring). The term "saponin" includes naturally-occurring saponins, derivatives of naturally-occurring saponins as well as saponins synthesized de novo through chemical and/or biotechnological synthesis routes.

The term "modified saponin" has its regular scientific meaning and here refers to a saponin, i.e. a saponin derivative, which has one or more chemical modifications at positions where previously any of an aldehyde group, a carboxyl group, an acetate group and/or an acetyl group was present in the non-derivatised saponin before being subjected to chemical modification for provision of the modified saponin. For example, the modified saponin is provided by chemical modification of any one or more of an aldehyde group, a carboxyl group, an acetate group and/or an acetyl group in a saponin upon which the modified saponin is based, i.e. the saponin is provided and any of an aldehyde group, a carboxyl group, an acetate group and/or an acetyl group is chemically modified therewith providing the modified saponin. For example, the saponin that is modified for provision of the modified saponin is a naturally occurring saponin. Typically, the modified saponin is a synthetic saponin, typically the modified saponin is a modification of a natural saponin, and is thus derived from a natural saponin, although a modified saponin can also be derived from a synthetic saponin which may or may not have a natural counterpart. Typically, the modified saponin has not a natural counterpart, i.e. the modified saponin is not produced naturally by e.g. plants or trees.

The term "aglycone core structure" has its regular scientific meaning and here refers to the aglycone core of a saponin without the one or two carbohydrate antenna or saccharide chains (glycans) bound thereto. For example, quillaic acid is the aglycone core structure for SO1861, QS-7 and QS21. Typically, the glycans of a saponin are mono-saccharides or oligo-saccharides, such as linear or branched glycans.

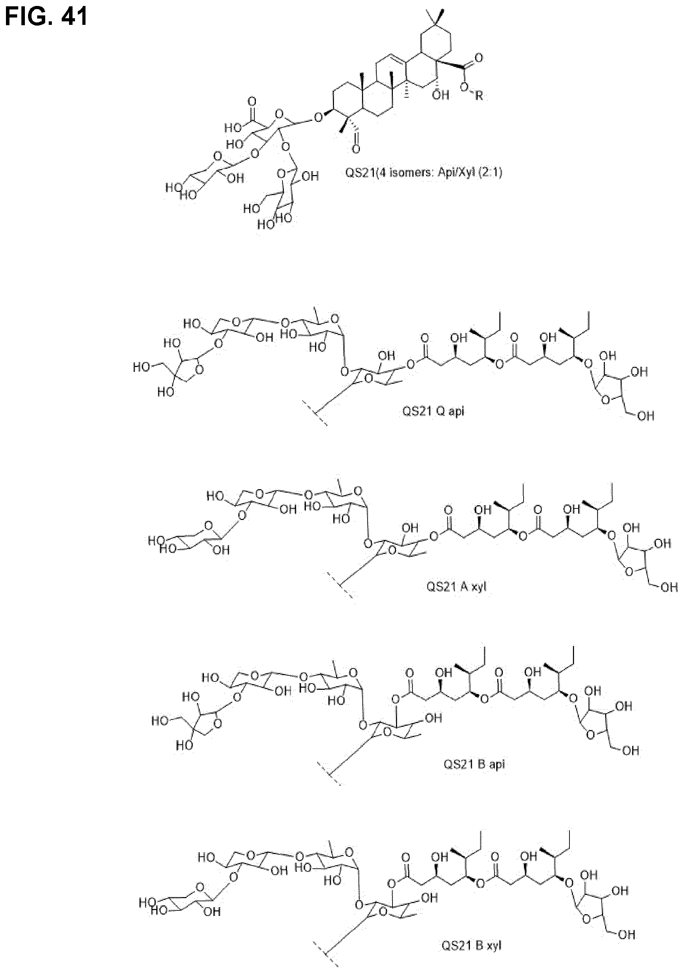

The term "QS21", unless further specified, refers to any one of the isomers of QS21, which have the structural formula shown in Figure 41, as well as to a mixture of two or more, such as all of the isomers shown in Figure 41. As will be understood by the skilled person, a typical natural extract comprising QS21 will comprise a mixture of the different isomers of QS21. However, through purification or (semi-)synthetic routes, a single isomer can be isolated.

The term "saccharide chain" has its regular scientific meaning and here refers to any of a glycan, a carbohydrate antenna, a single saccharide moiety (mono-saccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "chemically modified" has its regular scientific meaning and here refers to the chemical modification of a first chemical group or first chemical moiety such that a second chemical group or second chemical moiety is provided. Examples are the chemical modification of a carbonyl group into a - (H)C-OH group, the chemical modification of an acetate group into a hydroxyl group, the provision of a saponin conjugated at its aldehyde group with an N-ε-maleimidocaproic acid hydrazide (EMCH) moiety via a chemical reaction, etc.

The term "chemically modified aldehyde group" has its regular scientific meaning and here refers to the chemical reaction product obtained by the chemical reaction involving the aldehyde group of a saponin resulting in replacement of the initial aldehyde group by a new chemical group. For example, the formation of a - (H)C-OH group from the initial aldehyde group of a saponin.

The term "chemically modified carboxyl group" has its regular scientific meaning and here refers to the chemical reaction product obtained by the chemical reaction involving the carboxyl group of a saponin, such as the carboxyl group of a glucuronic acid moiety, and a further molecule, resulting in replacement of the initial carboxyl group by a new chemical group. For example, the formation of the conjugate between a saponin and any one of 2-amino-2-methyl-1,3-propanediol (AMPD), N-(2-aminoethyl)maleimide (AEM) or 1-[Bis(dimethylamino)methylene]-1H-1,2,3-triazolo[4,5-b]pyridinium 3-oxid hexafluorophosphate (HATU), involving the carboxyl group of the glucuronic acid of the saponin.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

The term "saponin on which the modified saponin is based" has its regular scientific meaning and here refers to a saponin that has been modified in order to provide the modified saponin. Typically, the saponin on which the modified saponin is based is a naturally occurring saponin, which is subjected to chemical modification for the provision of the modified saponin.

The term "modified saponin based on a saponin" has its regular scientific meaning and here refers to a saponin that has been subjected to a chemical modification step such that the modified saponin is provided, wherein the saponin from which the modified saponin has been made is typically a naturally occurring saponin.

The term "oligonucleotide" has its regular scientific meaning and here refers to amongst others any natural or synthetic string of nucleic acids encompassing DNA, modified DNA, RNA, mRNA, modified RNA, synthetic nucleic acids, presented as a single-stranded molecule or a double-stranded molecule, such as a BNA, an antisense oligonucleotide (ASO), a short or small interfering RNA (siRNA; silencing RNA), an anti-sense DNA, anti-sense RNA, etc.

The term "antibody-drug conjugate" or "ADC" has its regular scientific meaning and here refers to any conjugate of an antibody such as an IgG, a Fab, an scFv, an immunoglobulin, an immunoglobulin fragment, one or multiple V_{H} domains, single-domain antibodies, a V_{HH}, a camelid V_{H}, etc., and any molecule that can exert a therapeutic effect when contacted with cells of a subject such as a human patient, such as an active pharmaceutical ingredient, a toxin, an oligonucleotide, an enzyme, a small molecule drug compound, etc.

The term "antibody-oligonucleotide conjugate" or "AOC" has its regular scientific meaning and here refers to any conjugate of an antibody such as an IgG, a Fab, an scFv, an immunoglobulin, an immunoglobulin fragment, one or multiple V_{H} domains, single-domain antibodies, a V_{HH}, a camelid V_{H}, etc., and any oligonucleotide molecule that can exert a therapeutic effect when contacted with cells of a subject such as a human patient, such as an oligonucleotide selected from a natural or synthetic string of nucleic acids encompassing DNA, modified DNA, RNA, mRNA, modified RNA, synthetic nucleic acids, presented as a single-stranded molecule or a double-stranded molecule, such as a BNA, an antisense oligonucleotide (ASO), a short or small interfering RNA (siRNA; silencing RNA), an anti-sense DNA, anti-sense RNA, etc.

The term "effector molecule", or "effector moiety" when referring to the effector molecule as part of e.g. a covalent conjugate, has its regular scientific meaning and here refers to a molecule that can selectively bind to for example any one or more of the target molecules: a protein, a peptide, a carbohydrate, a saccharide such as a glycan, a (phospho)lipid, a nucleic acid such as DNA, RNA, an enzyme, and regulates the biological activity of such one or more target molecule(s). The effector molecule is for example a molecule selected from any one or more of a small molecule such as a drug molecule, a toxin such as a protein toxin, an oligonucleotide such as a BNA, a xeno nucleic acid or an siRNA, an enzyme, a peptide, a protein, or any combination thereof. Thus, for example, an effector molecule or an effector moiety is a molecule or moiety selected from any one or more of a small molecule such as a drug molecule, a toxin such as a protein toxin, an oligonucleotide such as a BNA, a xeno nucleic acid or an siRNA, an enzyme, a peptide, a protein, or any combination thereof, that can selectively bind to any one or more of the target molecules: a protein, a peptide, a carbohydrate, a saccharide such as a glycan, a (phospho)lipid, a nucleic acid such as DNA, RNA, an enzyme, and that upon binding to the target molecule regulates the biological activity of such one or more target molecule(s). Typically, an effector molecule can exert a biological effect inside a cell such as a mammalian cell such as a human cell, such as in the cytosol of said cell. Typical effector molecules are thus drug molecules, plasmid DNA, toxins such as toxins comprised by antibody-drug conjugates (ADCs), oligonucleotides such as siRNA, BNA, nucleic acids comprised by an antibody-oligonucleotide conjugate (AOC). For example, an effector molecule is a molecule which can act as a ligand that can increase or decrease (intracellular) enzyme activity, gene expression, or cell signalling.

The term "HSP27" relates to a BNA molecule which silences the expression of HSP27 in the cells.

The term "bridged nucleic acid", or "BNA" in short, or "locked nucleic acid" or "LNA" in short, has its regular scientific meaning and here refers to a modified RNA nucleotide. A BNA is also referred to as 'constrained RNA molecule' or 'inaccessible RNA molecule'. A BNA monomer can contain a five-membered, six-membered or even a seven-membered bridged structure with a "fixed" C₃'-endo sugar puckering. The bridge is synthetically incorporated at the 2', 4'-position of the ribose to afford a 2', 4'-BNA monomer. A BNA monomer can be incorporated into an oligonucleotide polymeric structure using standard phosphoramidite chemistry known in the art. A BNA is a structurally rigid oligonucleotide with increased binding affinity and stability.

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments of the invention described herein can operate in combination and cooperation, unless specified otherwise.

Furthermore, the various embodiments, although referred to as "preferred" or "e.g." or "for example" or "in particular" and the like are to be construed as exemplary manners in which the invention may be implemented rather than as limiting the scope of the invention.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

### BRIEF DESCRIPTION OF THE DRAWINGS

**Figure 1****:** Synthesis of molecule **3A**
**Figure 2****:** Synthesis of molecule **6**
**Figure 3****:** Synthesis of molecule **8**
**Figure 4****:** Synthesis of molecule **9**
**Figure 5****:** Synthesis of molecule **10**
**Figure 6****:** Synthesis of molecule **11**
**Figure 7****:** Synthesis of molecule **12**
**Figure 8****:** Synthesis of molecule **14**
**Figure 9****:** Synthesis of molecule **15**
**Figure 10****:** Synthesis of molecule **16**
**Figure 11****:** Synthesis of molecule **18**
**Figure 12****:** Synthesis of molecule **19**
**Figure 13****:** Synthesis of molecule **20**
**Figure 14****:** Synthesis of molecule **21**
**Figure 15****:** Mass-chromatogram of molecule **6**
**Figure 16****:** Detail of the mass-chromatogram of the synthesis of molecule **6** starting from SO1861
**Figure 17****:** Detail of the mass-chromatogram of molecule **9** starting from molecule **6**
**Figure 18A****:** IC50-curve for the endosomal escape enhancing activity of saponin derivatives in the presence of a non-effective fixed concentration of 5 pM EGF-dianthin on EGFR expressing cells (HeLa)
**Figure 18B****:** IC50-curve for the endosomal escape enhancing activity of saponin derivatives in the presence of a non-effective fixed concentration of 5 pM EGF-dianthin on EGFR expressing cells (A431)
**Figure 19A****:** IC50-curve for the endosomal escape enhancing activity of saponin derivatives in the presence of a non-effective fixed concentration of 5 pM EGF-dianthin on EGFR expressing cells (HeLa)
**Figure 19B****:** IC50-curve for the endosomal escape enhancing activity of saponin derivatives in the presence of a non-effective fixed concentration of 5 pM EGF-dianthin on EGFR expressing cells (A431)
**Figure 20A****:** IC50-curve for the toxicity of saponin derivatives in the presence of a non-effective fixed concentration of 5 pM EGF-dianthin on EGFR expressing cells (HeLa)
**Figure 20B****:** IC50-curve for the toxicity of saponin derivatives in the presence of a non-effective fixed concentration of 5 pM EGF-dianthin on EGFR expressing cells (A431)
**Figure 21A****:** IC50-curve for the toxicity of saponin derivatives on EGFR expressing cells (HeLa)

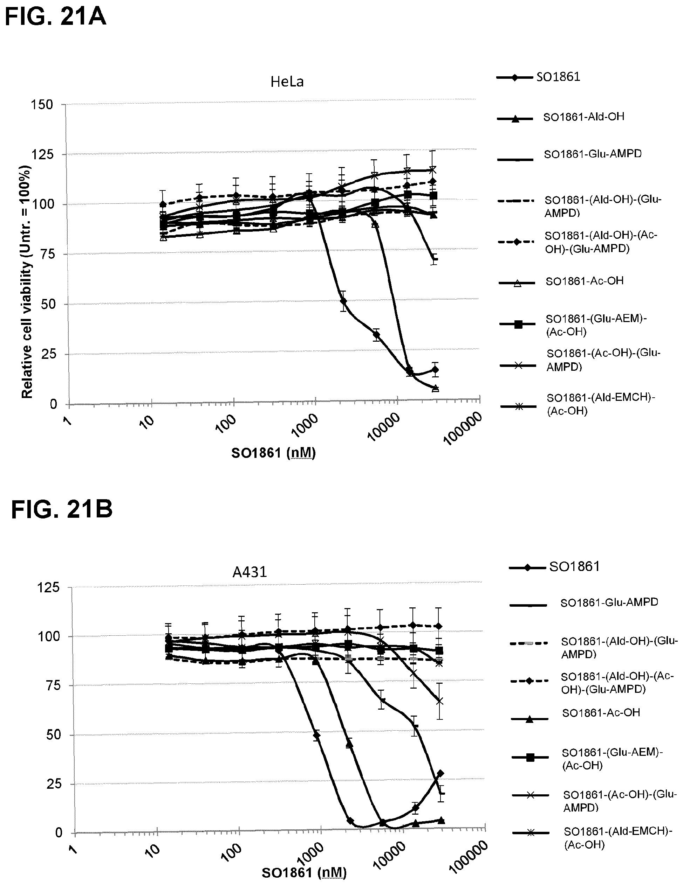

**Figure 21** **B:** IC50-curve for the toxicity of saponin derivatives on EGFR expressing cells (A431)

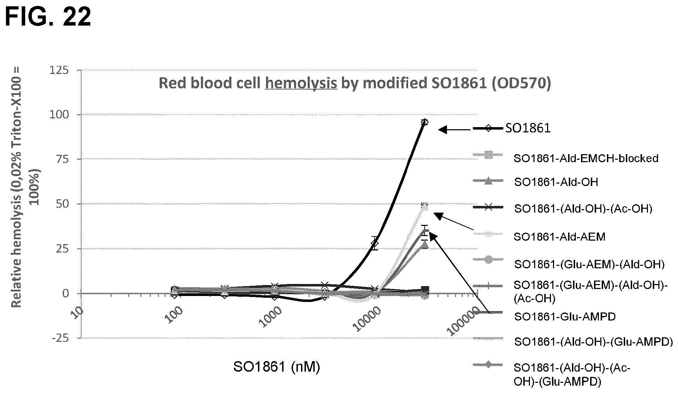

**Figure 22****:** hemolysis activity of the saponin derivatives determined by a human red blood cell hemolysis assay

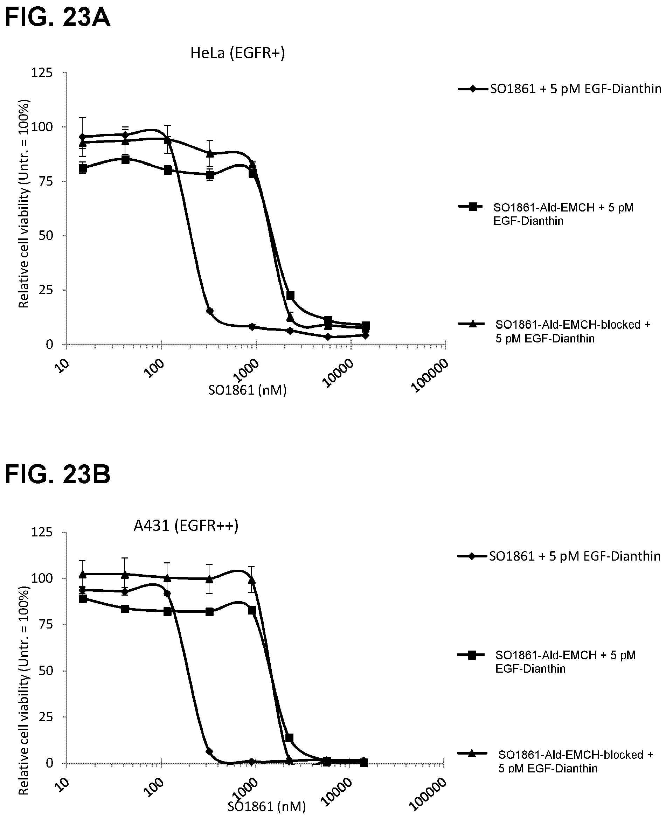

**Figure 23A****:** IC50-curve for the endosomal escape enhancing activity of saponin derivatives in the presence of a non-effective fixed concentration of 5 pM EGF-dianthin on EGFR expressing cells (HeLa)
**Figure 23B****:** IC50-curve for the endosomal escape enhancing activity of saponin derivatives in the presence of a non-effective fixed concentration of 5 pM EGF-dianthin on EGFR expressing cells (A431)

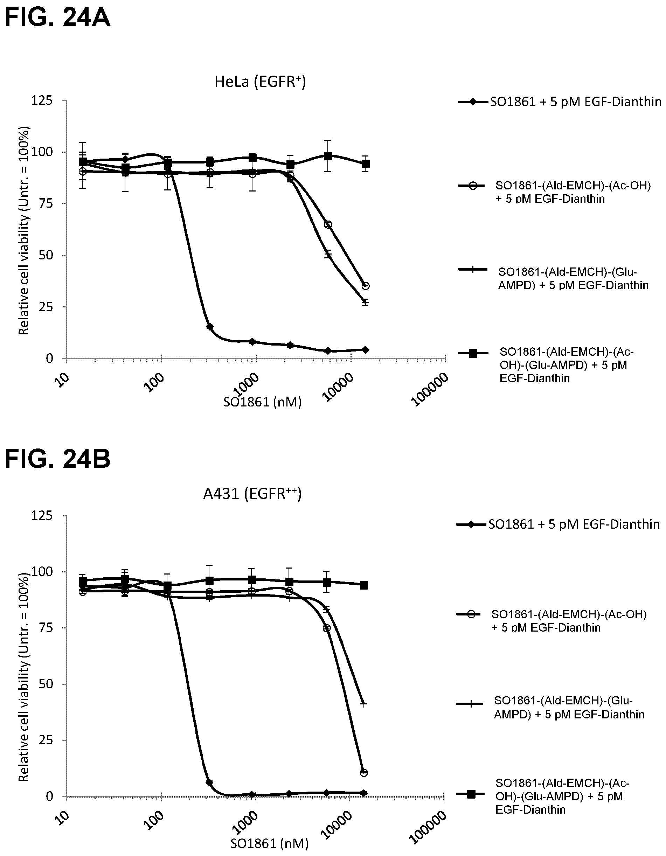

**Figure 24A****:** IC50-curve for the endosomal escape enhancing activity of saponin derivatives in the presence of a non-effective fixed concentration of 5 pM EGF-dianthin on EGFR expressing cells (HeLa)
**Figure 24B****:** IC50-curve for the endosomal escape enhancing activity of saponin derivatives in the presence of a non-effective fixed concentration of 5 pM EGF-dianthin on EGFR expressing cells (A431)

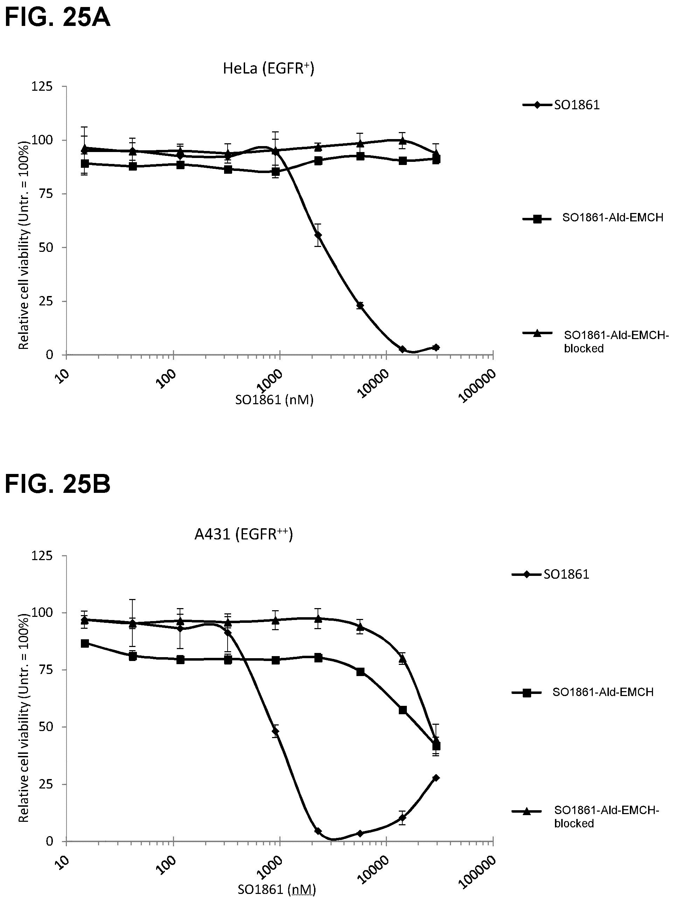

**Figure 25A****:** IC50-curve for the toxicity of saponin derivatives on EGFR expressing cells (HeLa)
**Figure 25B****:** IC50-curve for the toxicity of saponin derivatives on EGFR expressing cells (A431)

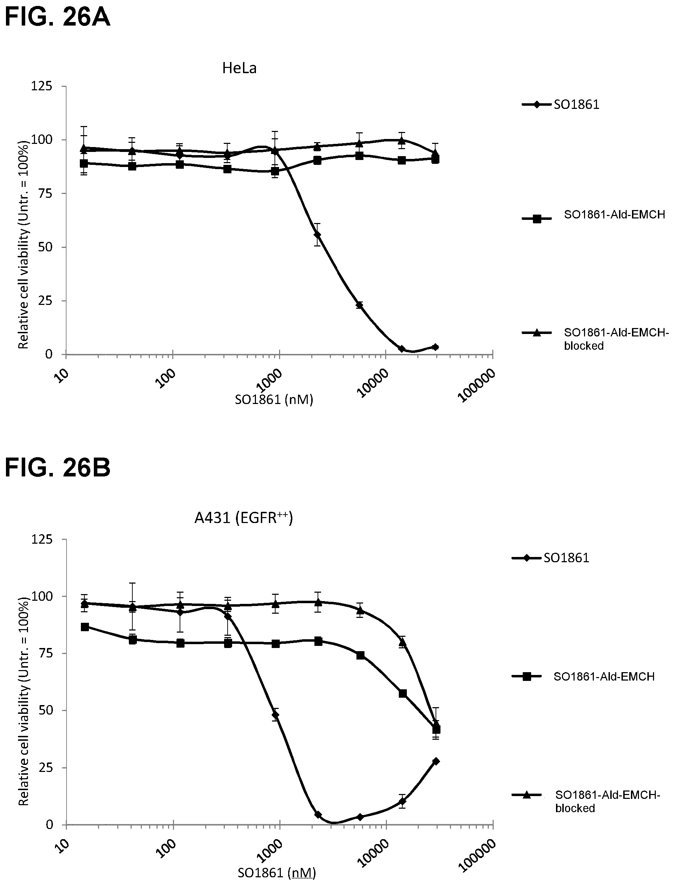

**Figure 26A****:** IC50-curve for the toxicity of saponin derivatives on EGFR expressing cells (HeLa)
**Figure 26B****:** IC50-curve for the toxicity of saponin derivatives on EGFR expressing cells (A431)

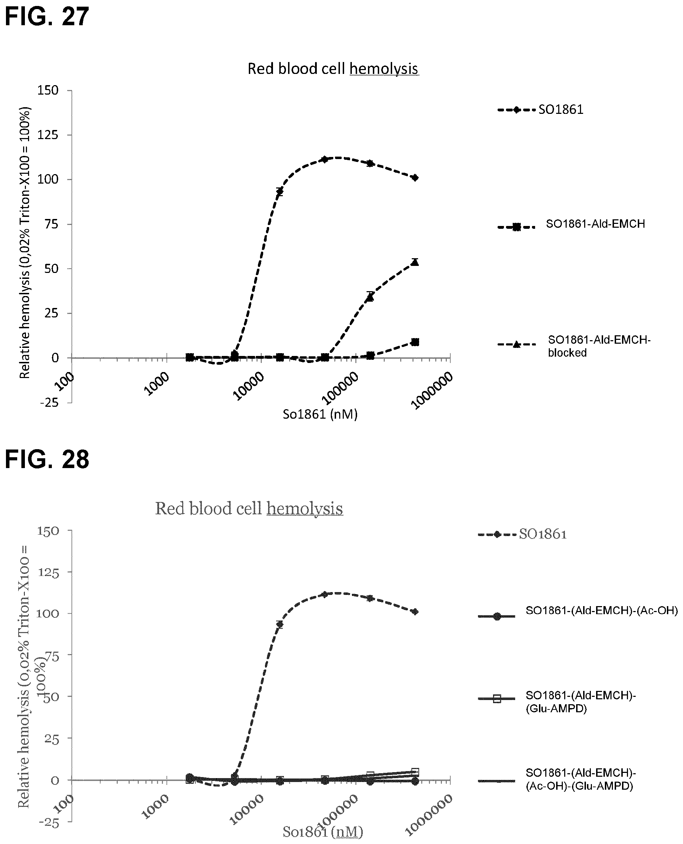

**Figure 27****:** hemolysis activity of the saponin derivatives determined by a human red blood cell hemolysis assay.
**Figure 28****:** hemolysis activity of saponin derivatives determined by a human red blood cell hemolysis assay

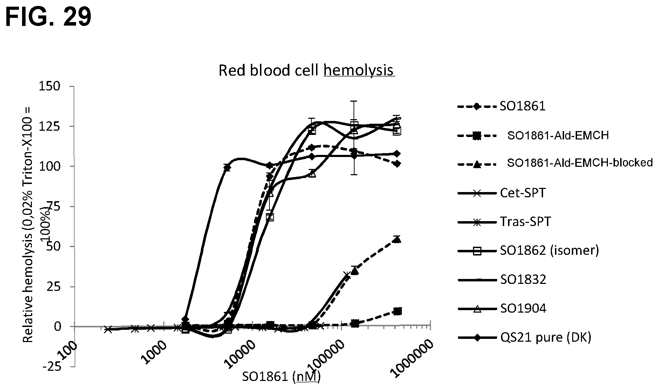

**Figure 29****:** hemolysis activity of saponin derivatives determined by a human red blood cell hemolysis assay

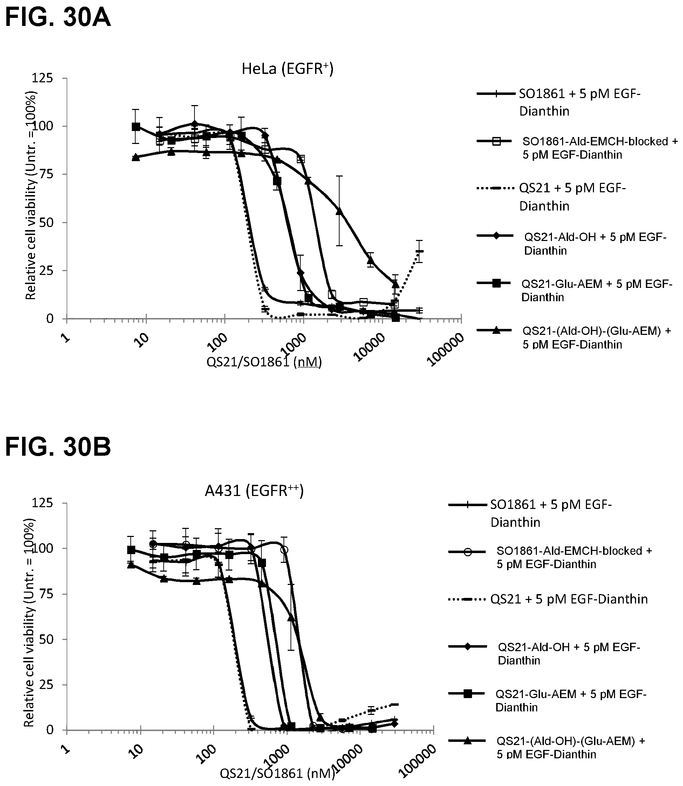

**Figure 30A****:** IC50-curve for the activity of saponin derivatives in the presence of a non-effective fixed concentration of 5 pM EGF-dianthin on EGFR expressing cells on EGFR expressing cells (HeLa)
**Figure 30B****:** IC50-curve for the activity of saponin derivatives in the presence of a non-effective fixed concentration of 5 pM EGF-dianthin on EGFR expressing cells on EGFR expressing cells (A431)

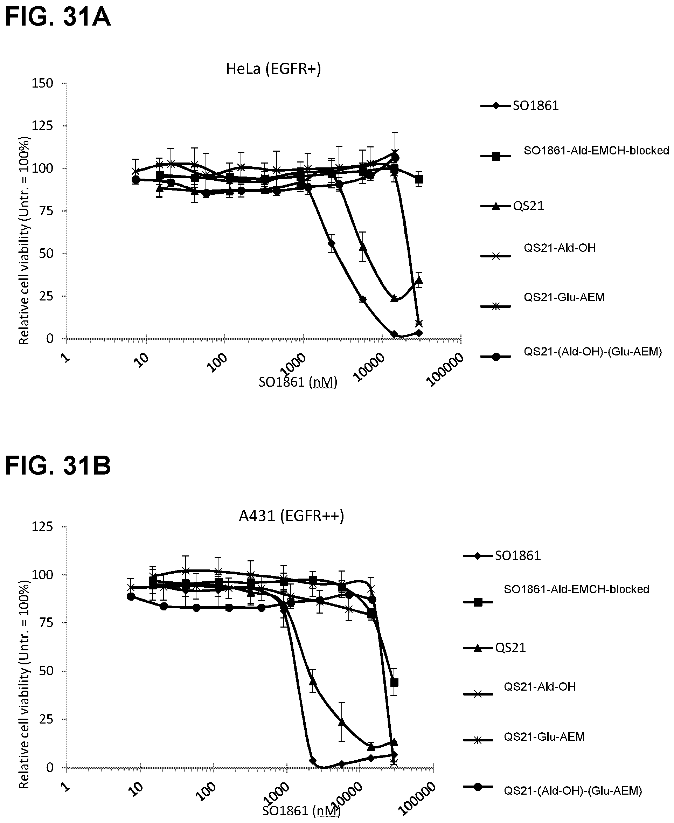

**Figure 31A****:** IC50-curve for the toxicity of saponin derivatives on EGFR expressing cells (HeLa)
**Figure 31B****:** IC50-curve for the toxicity of saponin derivatives on EGFR expressing cells (A431)

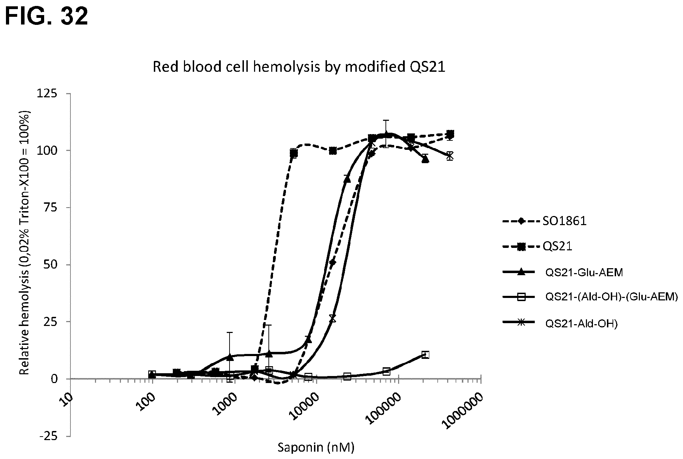

**Figure 32****:** hemolysis activity of the saponin derivatives determined by a human red blood cell hemolysis assay

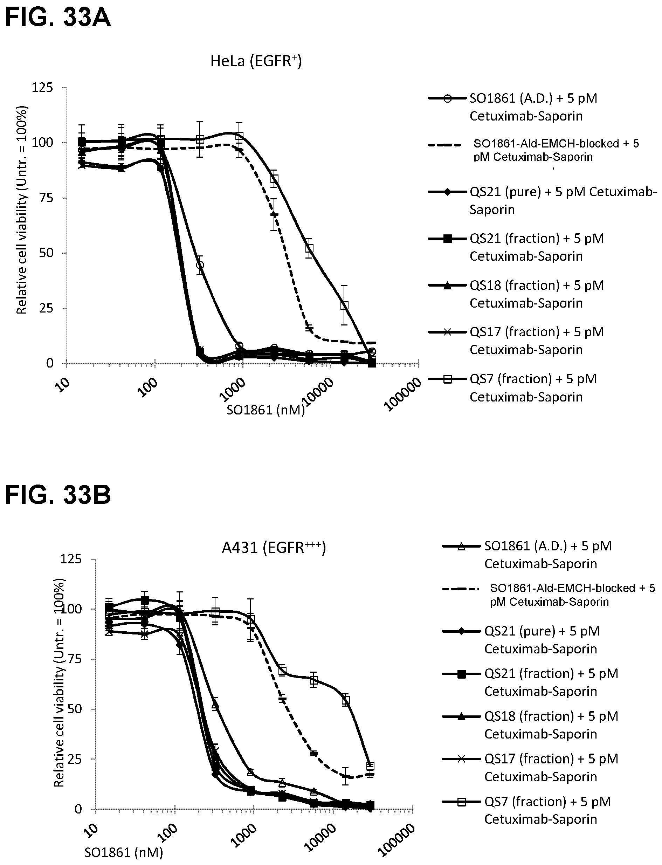

**Figure 33A****:** IC50-curve for the endosomal escape enhancing activity of various QS saponins fractions in the presence of a concentration of 5 pM cetuximab-Saporin on EGFR expressing cells (HeLa)
**Figure 33B****:** IC50-curve for the endosomal escape enhancing activity of various QS saponins fractions in the presence of a concentration of 5 pM cetuximab-Saporin on EGFR expressing cells (A431)

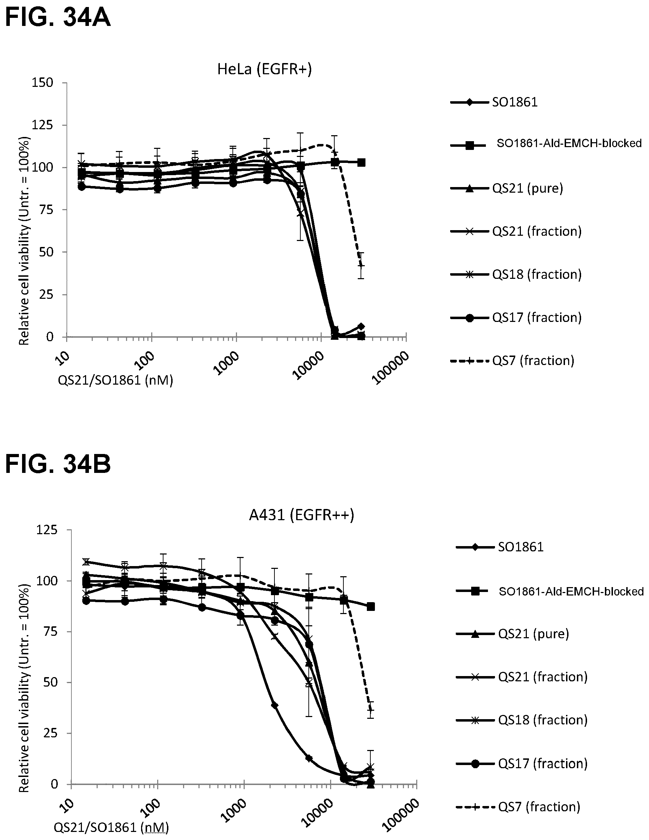

**Figure 34A****:** IC50-curve for the toxicity of QS saponins fractions on EGFR expressing cells (HeLa)
**Figure 34B****:** IC50-curve for the toxicity of QS saponins fractions on EGFR expressing cells (A431)

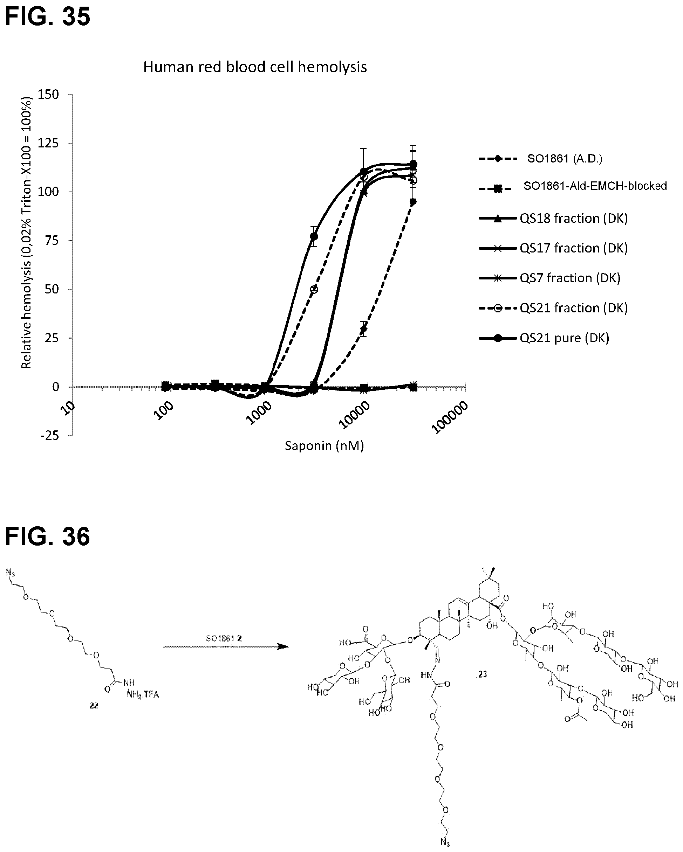

**Figure 35****:** hemolysis activity of QS saponins fractions determined by a human red blood cell hemolysis assay
**Figure 36****:** Synthesis of molecule **23**

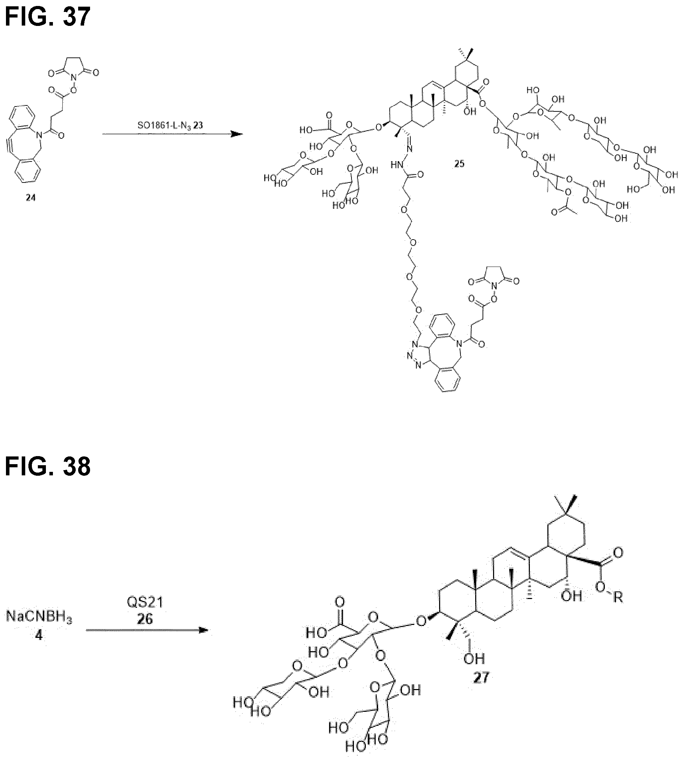

**Figure 37****:** Synthesis of molecule **25**
**Figure 38****:** Synthesis of molecule **27**

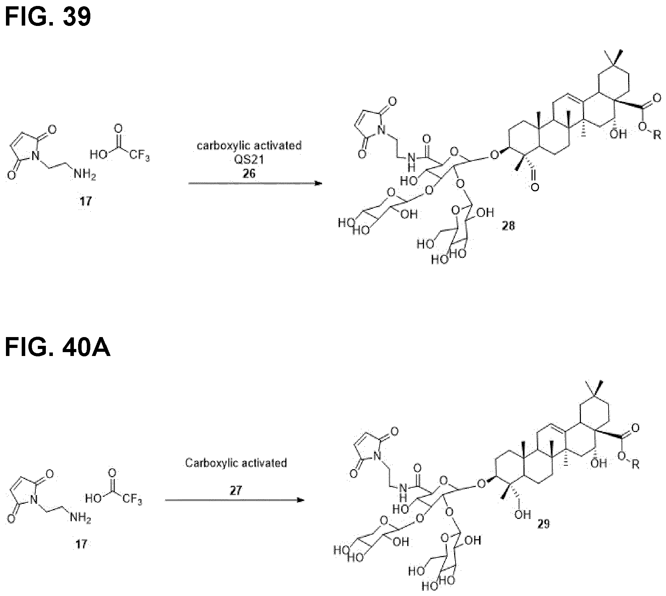

**Figure 39****:** Synthesis of molecule **28**
**Figure 40A:** Synthesis of molecule **29**

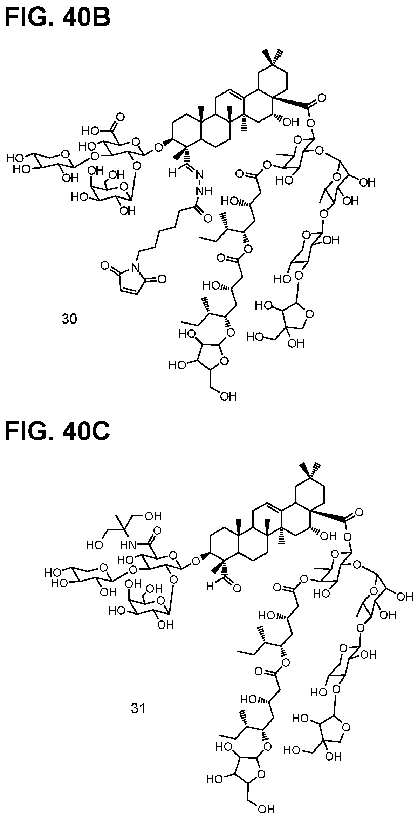

**Figure 40B****:** QS21-Ald-EMCH (molecule **30)**
**Figure 40C****:** QS21-Glu-AMPD (molecule **31)**

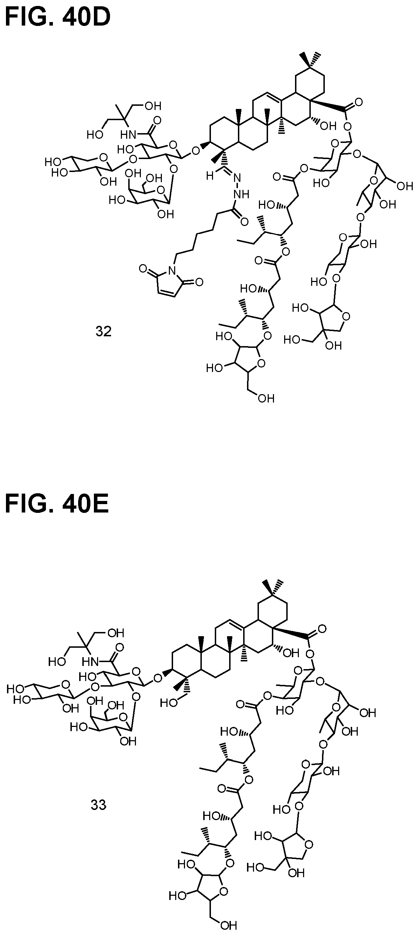

**Figure 40D****:** QS21-(Ald-EMCH)-(Glu-AMPD) (molecule **32)**
**Figure 40E****:** QS21-(Ald-OH)-(Glu-AMPD) (molecule **33)**
**Figure 41****:** structure of four QS-21 isomers.

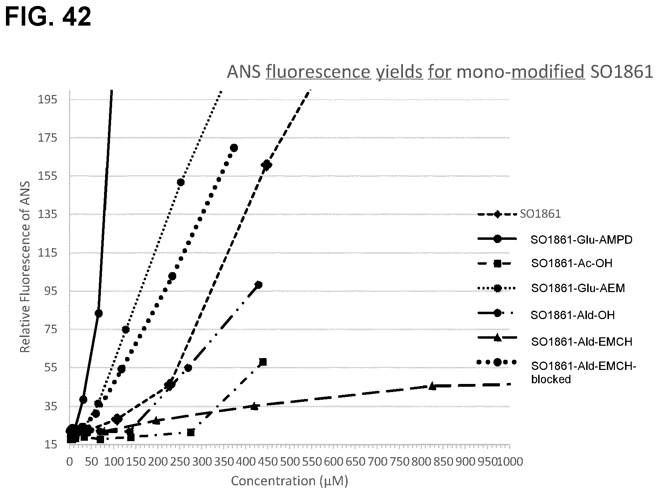

**Figure 42****:** Determining critical micelle concentrations: ANS fluorescence yields for mono-modified SO1861.

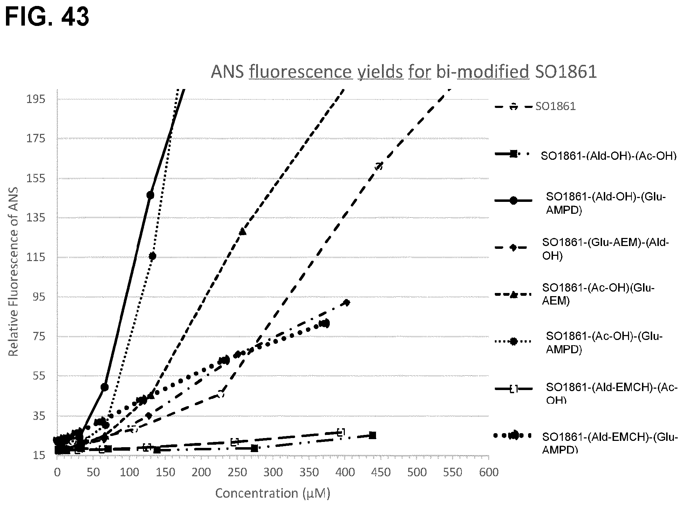

**Figure 43****:** Determining critical micelle concentrations: ANS fluorescence yields for bi-modified SO1861.

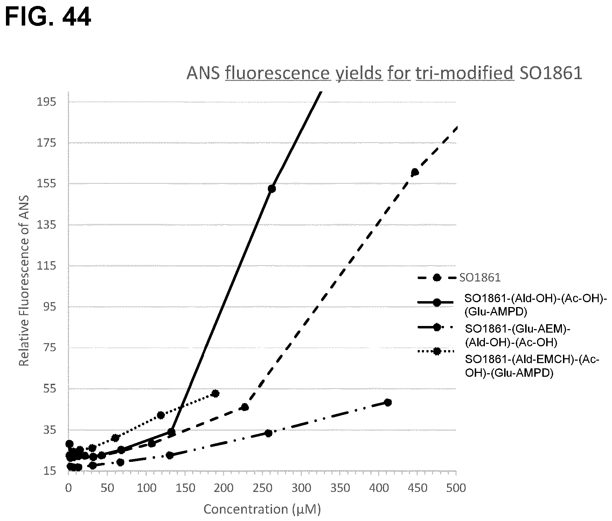

**Figure 44****:** Determining critical micelle concentrations: ANS fluorescence yields for tri-modified SO1861.

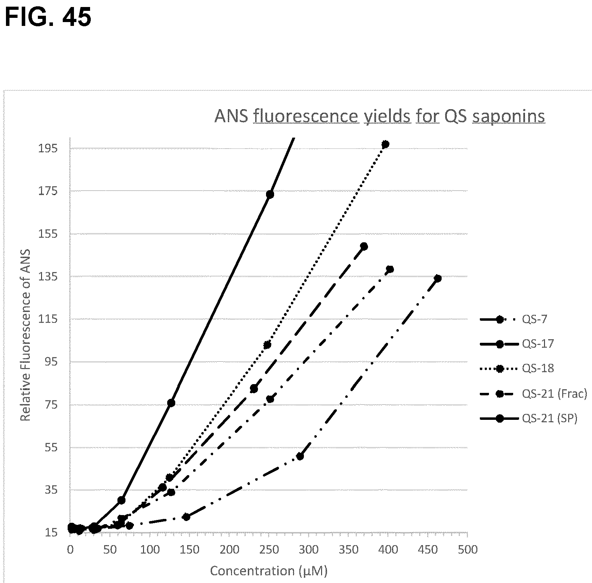

**Figure 45****:** Determining critical micelle concentrations: ANS fluorescence yields for QS saponins.

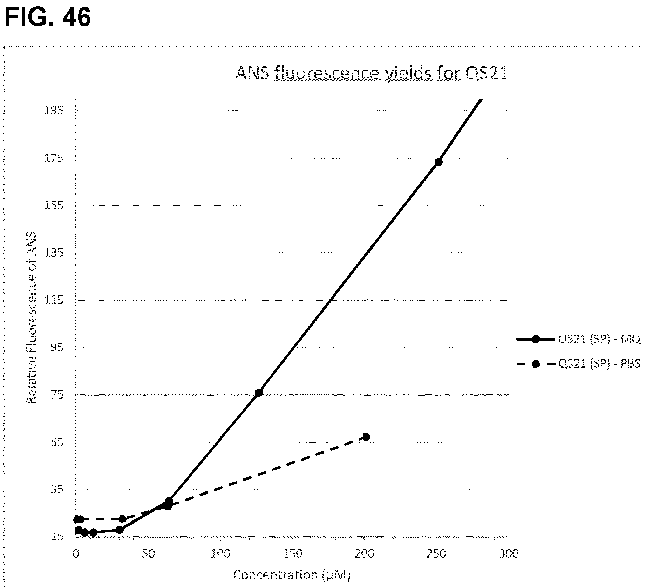

**Figure 46****:** Determining critical micelle concentrations: ANS fluorescence yields for QS21.

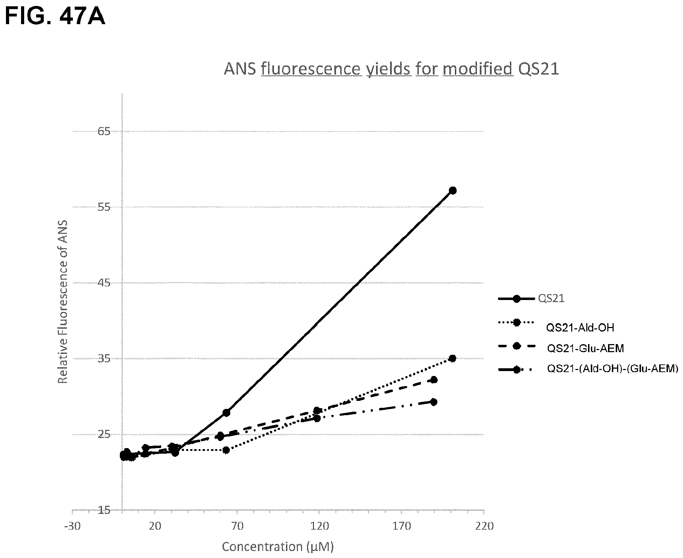

**Figure 47A****:** Determining critical micelle concentrations: ANS fluorescence yields for modified QS21.

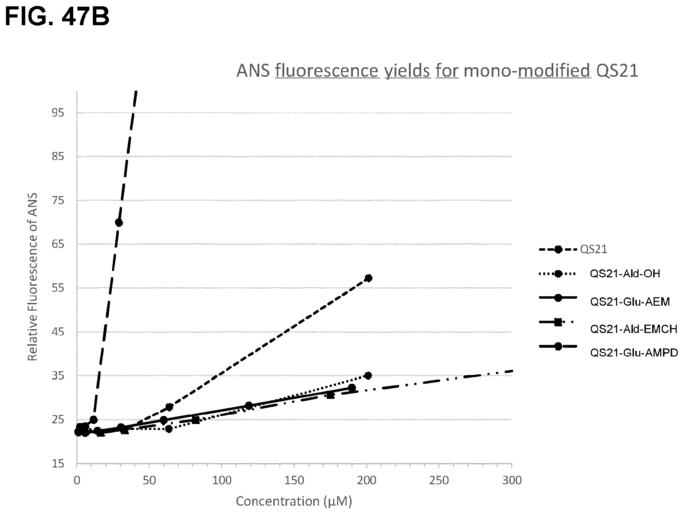

**Figure 47B****:** Determining critical micelle concentrations: ANS fluorescence yields for mono-modified QS21.

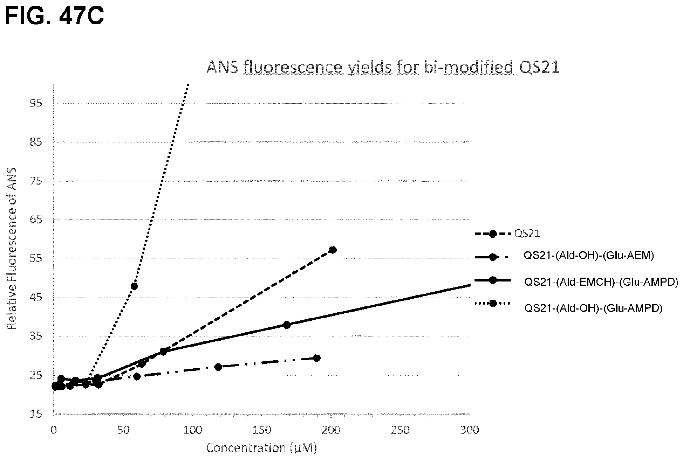

**Figure 47C****:** Determining critical micelle concentrations: ANS fluorescence yields for bi-modified QS21.

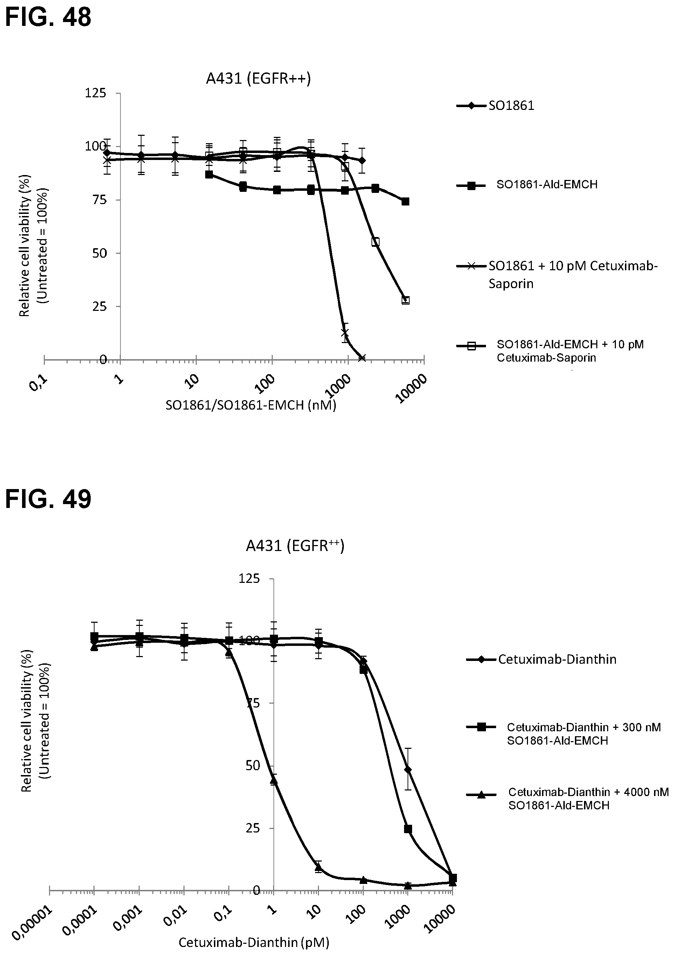

**Figure 48****:** Cell viability assay (MTS) of SO1861 or SO1861-EMCH + 10 pM Cetuximab-saporin on A431 cells.
**Figure 49****:** Cell viability assay (MTS) of cetuximab-dianthin + 300 nM and 4000 nM SO1861-EMCH on A431 cells.

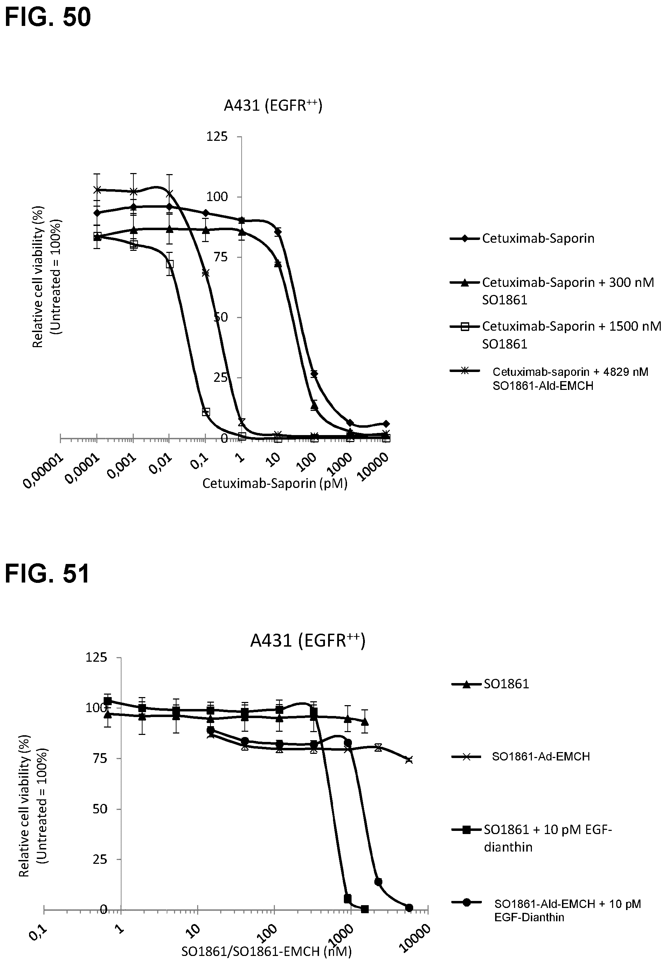

**Figure 50****:** Cell viability assay (MTS) of cetuximab-saporin + 300 nM and 1500 nM SO1861 or 4000 nM SO1861-EMCH on A431 cells.
**Figure 51****:** Cell viability assay (MTS) of SO1861 or SO1861-EMCH + 10 pM EGFdianthin on A431 cells.

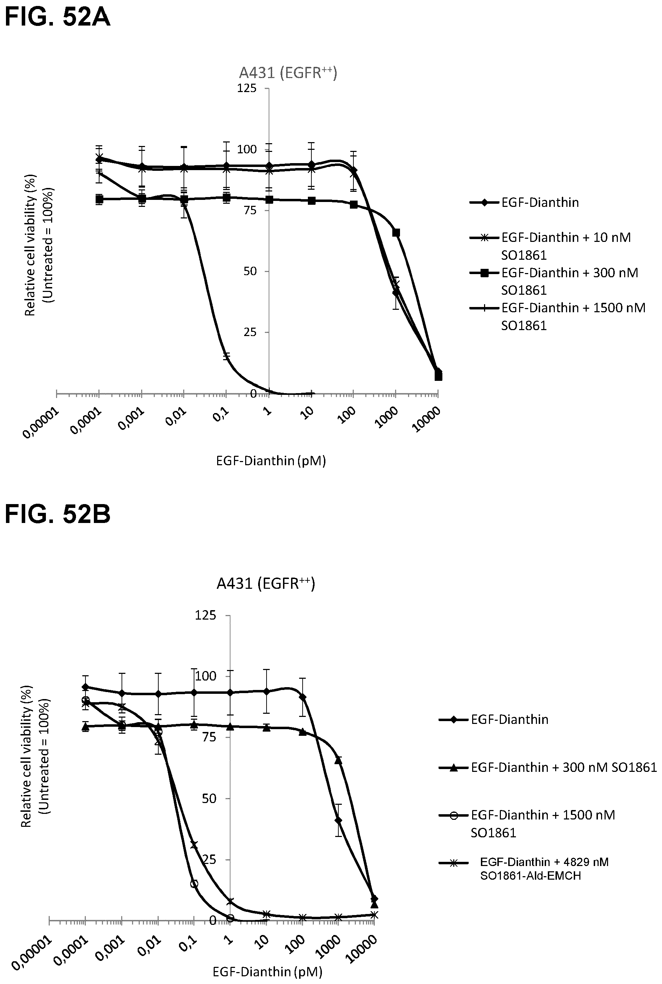

**Figure 52A****, B:** Cell viability assay (MTS) of EGFdianthin + 10 nM, 300nM and 1500 nM SO1861 or 4829 nM SO1861-EMCH on A431 cells.

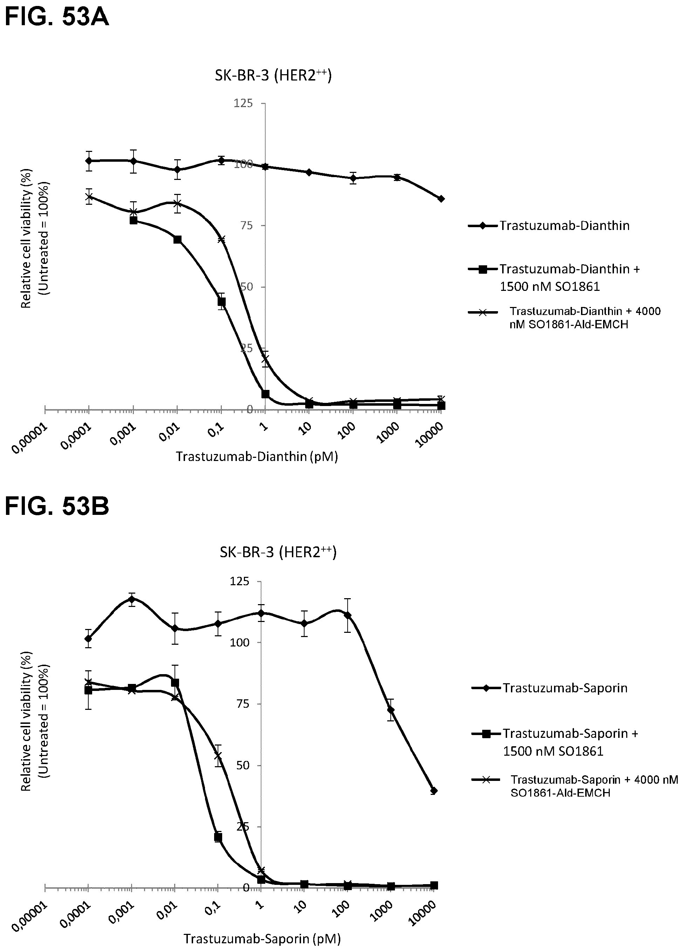

**Figure 53A****, B:** Cell viability assay (MTS) of trastuzumab-dianthin or trastuzumab-saporin + 1500 nM SO1861 or 4000 nM SO1861-EMCH on A431 cells.

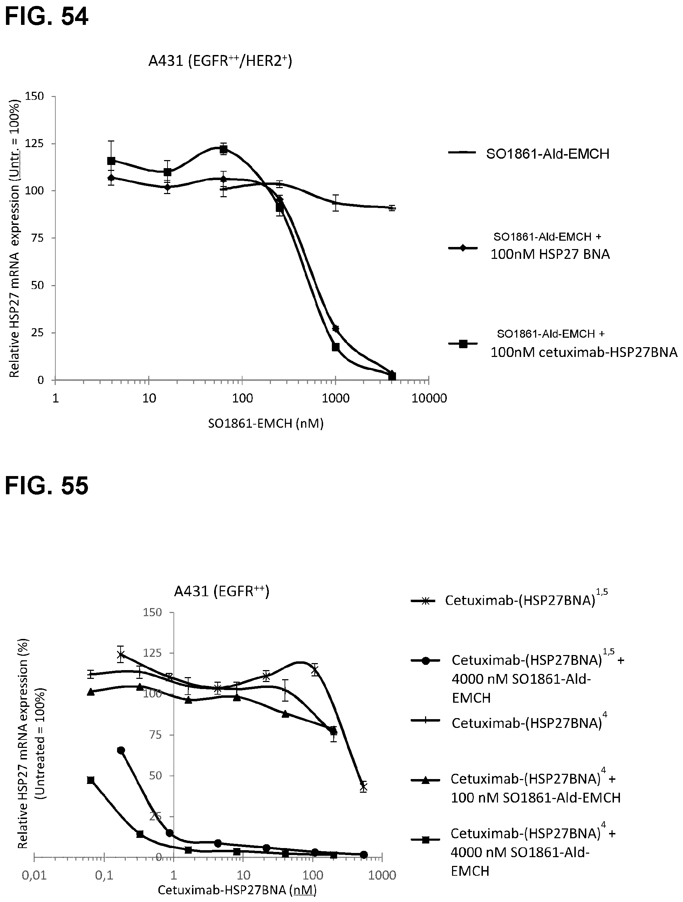

**Figure 54****:** HSP27 mRNA gene silencing analysis of SO1861-EMCH + 100 nM HSP27BNA, 100 nM cetuximab-HSP27BNA on A431 cells.
**Figure 55****:** HSP27 mRNA gene silencing analysis of cetuximab-HSP27BNA conjugate (DAR1.5 or DAR4) + 100 nM SO1861-EMCH or 4000 nM SO1861-EMCH on A431 cells.

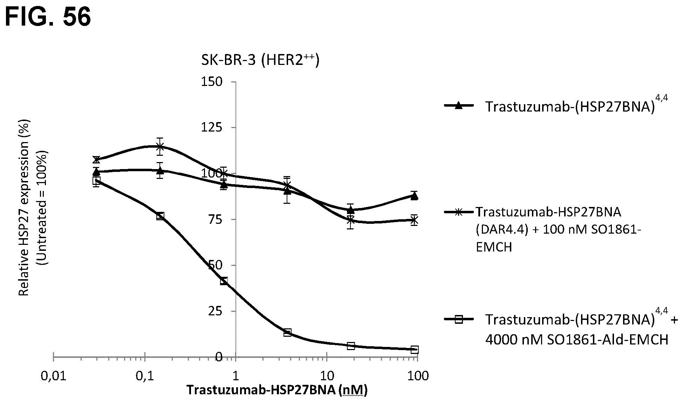

**Figure 56****:** HSP27 mRNA gene silencing analysis of trastuzumab-HSP27BNA conjugate (DAR4.4) + 100 nM SO1861-EMCH or 4000 nM SO1861-EMCH on SK-BR-3 cells.

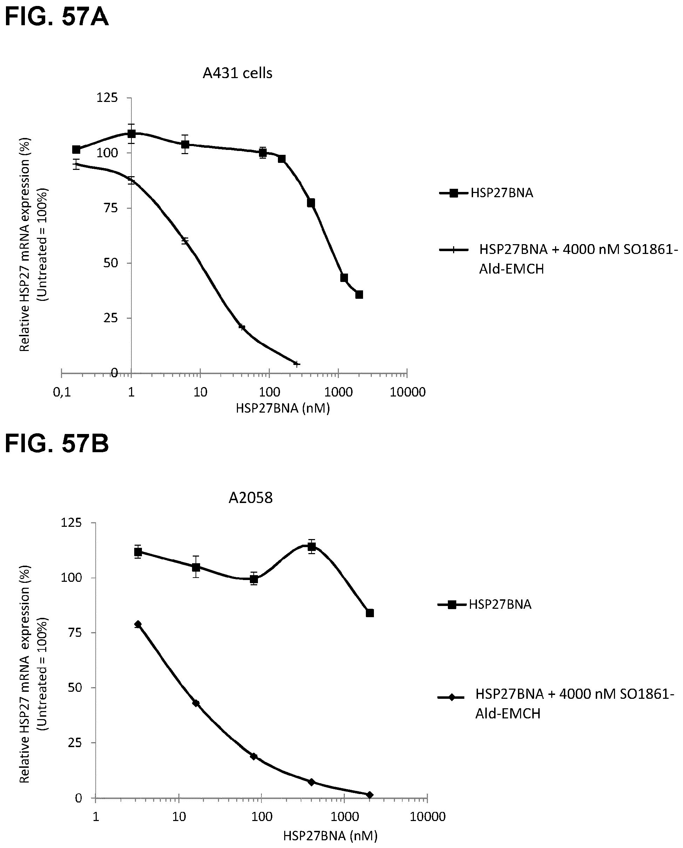

**Figure 57A****, B:** HSP27 mRNA gene silencing analysis of HSP27BNA + 4000 nM SO1861-EMCH on A431 cells and A2058 cells.

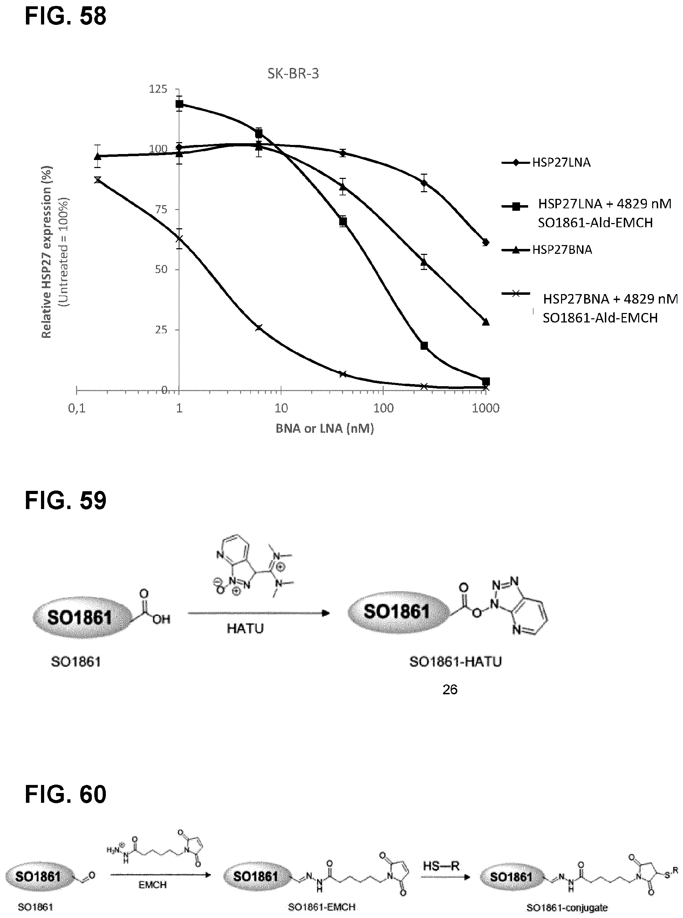

**Figure 58****:** HSP27 mRNA gene silencing analysis of HSP27BNA or HSP27LNA + 4829 nM SO1861-EMCH on SK-BR-3 cells.
**Figure 59****:** Synthesis of molecule **26.**
**Figure 60****:** General reaction scheme of the Michael addition reaction of the EMCH maleimide group with thiols (if R = CH₂-CH₂-OH then Figure 60 describes the synthesis of SO1861-Ald-EMCH-blocked (SO1861-Ald-EMCH-mercaptoethanol)).

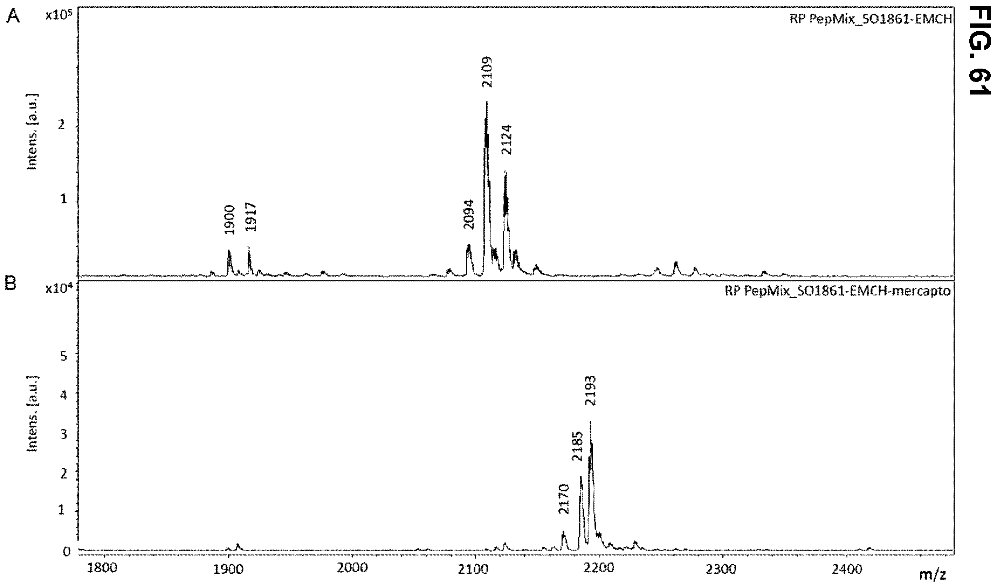

**Figure 61****:** (A) MALDI-TOF-MS spectrum of SO1861-Ald-EMCH and (B) SO1861-Ald-EMCH-mercaptoethanol. (A) RP mode: *m*/*z* 2124 Da ([M+K]⁺, saponin-Ald-EMCH), *m*/*z* 2109 Da ([M+K]⁺, SO1861-Ald-EMCH), *m*/*z* 2094 Da ([M+Na]⁺, SO1861-EMCH). (B) RP mode: *m*/*z* 2193 Da ([M+K]⁺, saponin-Ald-EMCH-mercaptoethanol), *m*/*z* 2185 Da ([M+K]⁺, SO1861-Ald-EMCH-mercaptoethanol), *m*/*z* 2170 Da ([M+Na]⁺, SO1861-Ald-EMCH-mercaptoethanol).

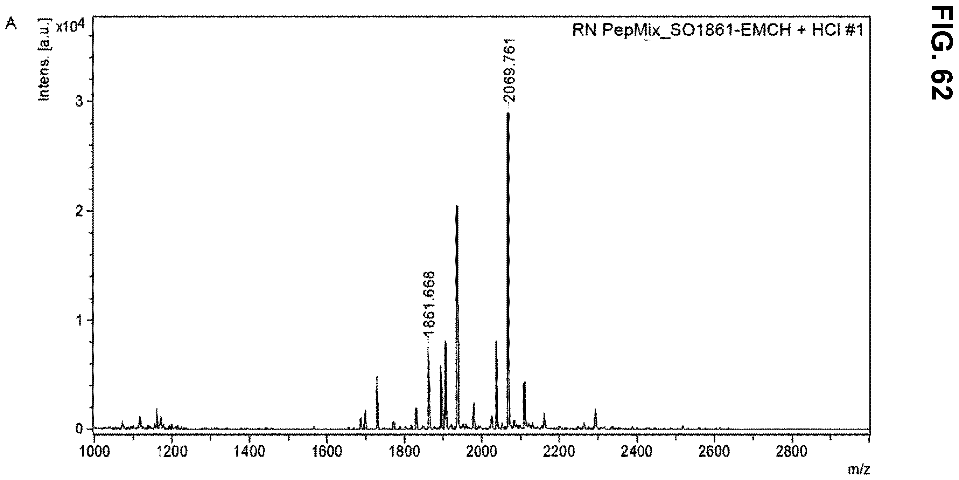

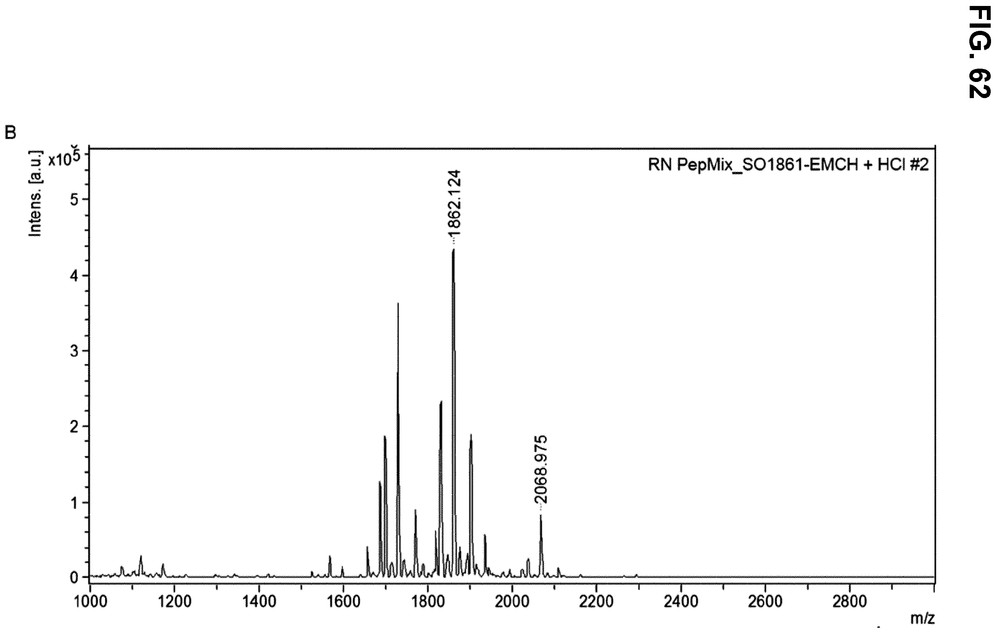

**Figure 62****:** MALDI-TOF-MS spectra of SO1861-EMCH (A) before and (B) after hydrolysis in HCl solution at pH 3.

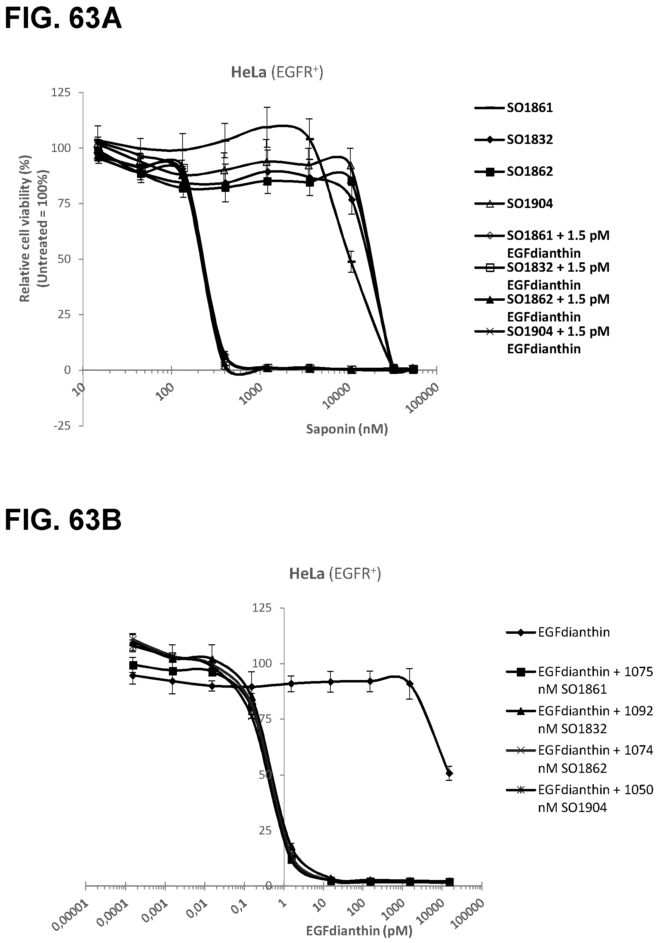

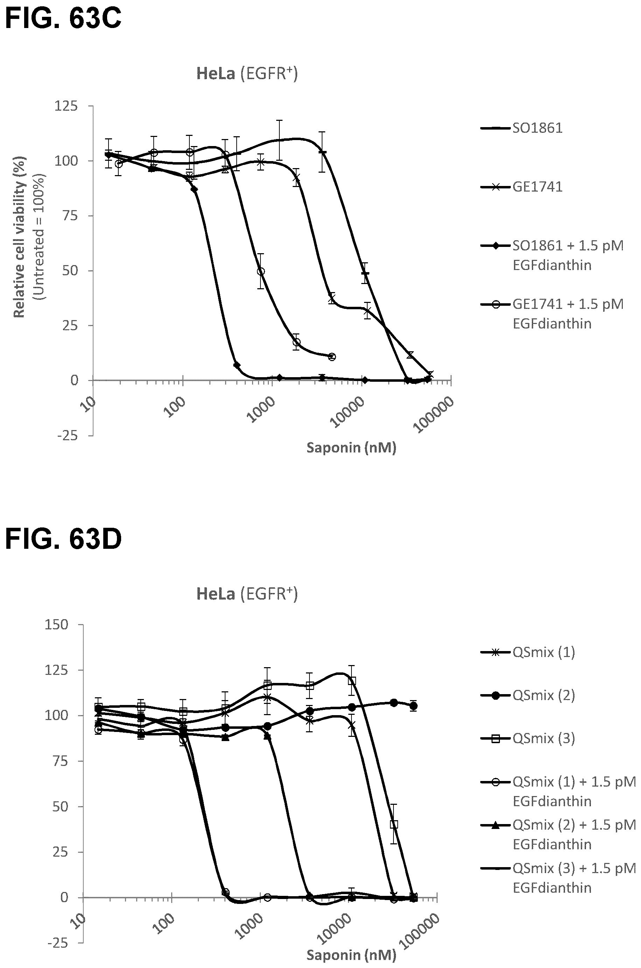

**Figure 63****.** *unconjugated saponin-mediated endosomal escape and target cell killing enhancement. A)* Cell viability analyses of HeLa cells (EGFR⁺) treated with SO1861, SO1832, SO1862 (isomer of SO1861) or SO1904 with or without 1.5 pM EGFdianthin B) Cell viability analyses of HeLa cells (EGFR⁺) treated with EGFdianthin and fixed concentrations of SO1861, SO1832, SO1862 (isomer of SO1861) or SO1904. C) Cell viability analyses of HeLa cells (EGFR⁺) treated with SO1861 or GE1741 with or without 1.5 pM EGFdianthin. D) Cell viability analyses of HeLa cells (EGFR⁺) treated with various QSmix (saponin mixture from Quillaia Saponaria) with or without 1.5 pM EGFdianthin.

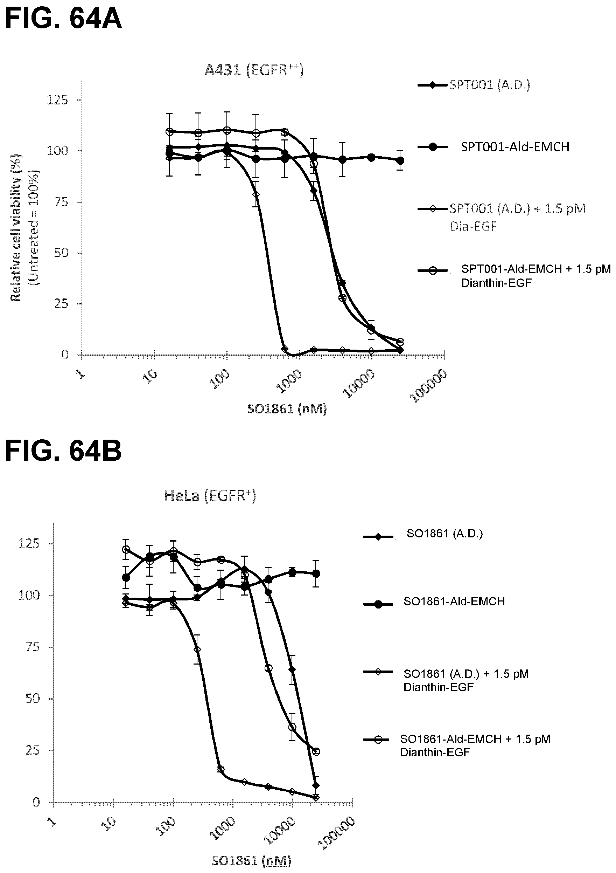

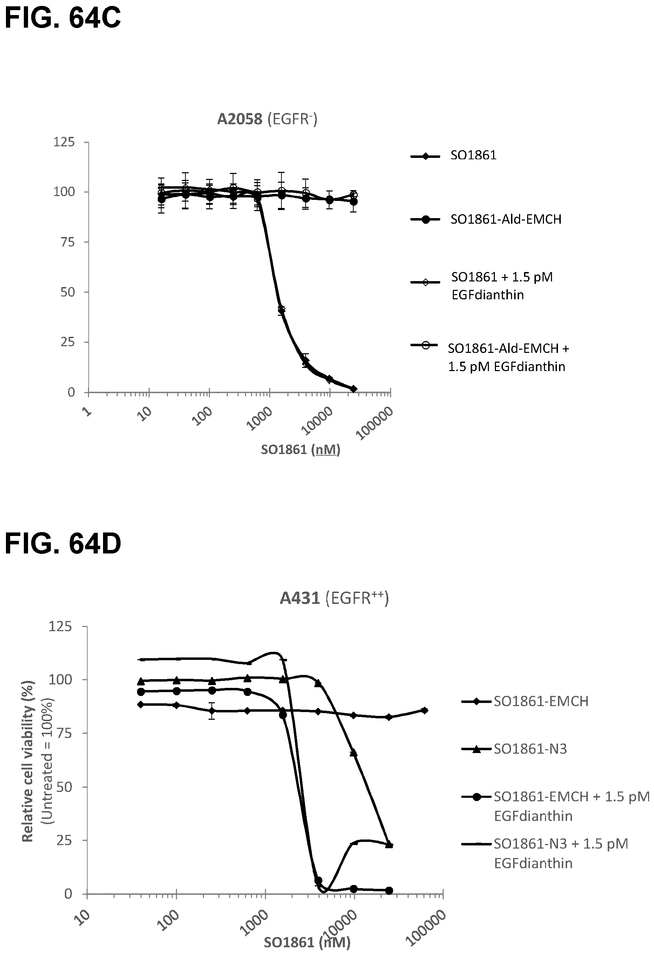

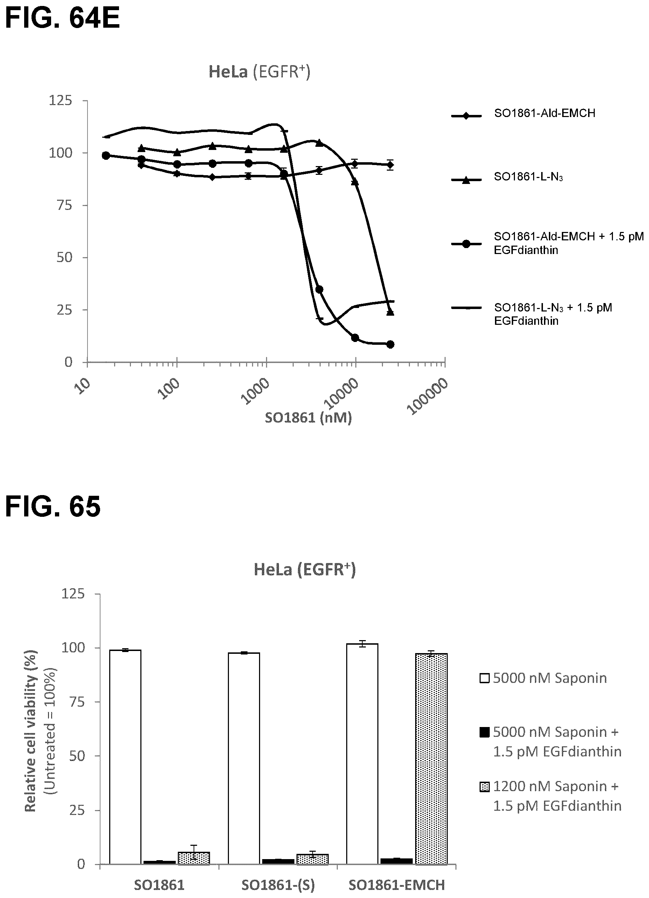

**Figure 64****.** *unconjugated* SO1861 *versus SO1861-Ald-EMCH activity. EGFR targeted antisense BNA oligo delivery and gene silencing in cancer cells, according to the invention.* A, B, C) Cell viability analyses of A431 (EGFR⁺⁺), HeLa (EGFR⁺) or A2058 (EGFR) cells treated with SO1861 or SO1861-Ald-EMCH with or without 1.5 pM EGFdianthin. D, E) Cell viability analyses of A431 (EGFR⁺⁺) or HeLa (EGFR⁺) cells treated with SO1861 or SO1861-L-N₃ (also referred to as SO1861-N3 or SO1861-N3/azide) with or without 1.5 pM EGFdianthin.
**Figure 65****.** *unconjugated SO1861 versus SO1861-Ald-EMCH (labile hydrazone bond) versus SO1861-HATU (also referred to as SO1861-(S) (stable) and SO1861-Glu-HATU).* Cell viability analyses of HeLa cells (EGFR⁺) treated with SO1861, SO1861-Glu-HATU (also referred to as SO1861-(S) (S=HATU)) and SO1861-Ald-EMCH (the hydrazone bond between the SO1861 aglycone core and the EMCH linker is also referred to as a 'labile linker'), with or without EGFdiantin.

### DETAILED DESCRIPTION

The present invention will be described with respect to particular embodiments but the invention is not limited thereto but only by the claims.

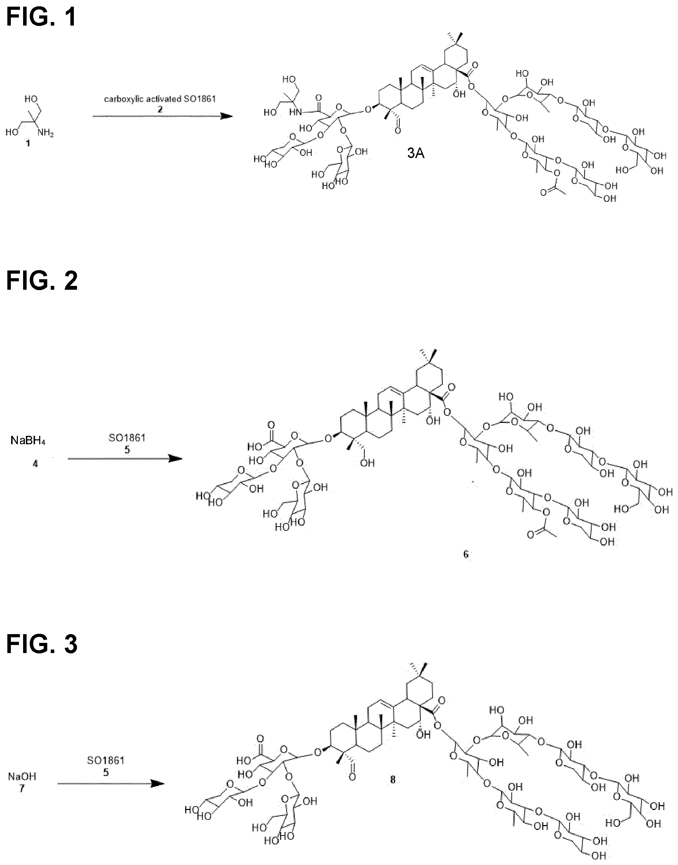

Surprisingly, the inventors have found that modified saponins, i.e. saponin derivatives, having the groups:
- a branched tri-saccharide moiety bound at C-3 of the aglycone of the saponin and containing a modified glucuronic acid; and/or
- a modified aldehyde at C-4 of the aglycone of the saponin; and/or
- a polysaccharide moiety bound at C-28 position of the aglycone of the saponin, and containing a modified acetyl group in said polysaccharide moiety;
have a reduced toxicity when cell viability is considered of cells contacted with the saponin derivatives; have activity when potentiation of e.g. toxin cytotoxicity or BNA mediated gene silencing is considered (without wishing to be bound by any theory: relating to similar or improved endosomal escape enhancing activity of the modified saponin), if one or two of said aforementioned groups in the modified saponin are derivatised (i.e. one or two of: the aldehyde group in the aglycone, a carboxyl group of a glucuronic acid in the polysaccharide chain bound at C-3 of the aglycone, and an acetyl group in the polysaccharide chain bound at C-28 of the aglycone); and/or have reduced hemolytic activity, when compared with the toxicity, activity and haemolytic activity of unmodified saponin. Therewith, the inventors provide saponin derivatives according to claim 1 with an improved therapeutic window, since for the saponin derivatives, the cytotoxicity is lower than cytotoxicity determined for their naturally occurring counterparts, the haemolytic activity is lower than haemolytic activity determined for the naturally occurring counterparts of the saponin derivatives, and for the single derivatised saponins and for the double-derivatised saponins, the ratio between IC50 values for cell toxicity and e.g. toxin potentiation or gene silencing is similar or increased, and/or since the ratio between IC50 values for saponin haemolytic activity and e.g. toxin potentiation or gene silencing is similar or increased. Reference is made to Tables A2 for an overview of exemplified saponin derivatives, in combination with Figures 1-14 and 36-40, and to Table A5 and Table A6 for an overview of the cytotoxicity, haemolytic activity and endosomal escape enhancing activity ('activity'), as well as an overview of the ratio between IC50 for cytotoxicity and IC50 for activity, and the ratio between IC50 for haemolytic activity and IC50 for activity, as determined on various cells.

A first aspect of the invention relates to a saponin derivative according to claim 1, which is based on a SO1861 saponin comprising a triterpene aglycone core structure (also referred to as 'aglycone') and a first saccharide chain and a second saccharide chain linked to the aglycone core structure, wherein:
i. the saponin derivative comprises said aglycone core structure comprising an aldehyde group at position C23 of the quillaic acid which has been derivatised; or
ii. the saponin derivative comprises the first saccharide chain A1 wherein the first saccharide chain comprises a carboxyl group of a glucuronic acid moiety, which has been derivatised; or
iii. the saponin derivative comprises the second saccharide chain A2 wherein the second saccharide chain comprises an acetoxy (Me(CO)O-) group which has been derivatised; or
iv. the saponin derivative comprises any combination of derivatisations i., ii. and iii., preferably any combinations of two derivatisations i., ii. and iii..

The saponin derivative according to the invention is a bidesmosidic triterpene glycoside.

Surprisingly, modification (derivatisation) of any one, two or three of the aldehyde group at C-23 of the aglycone of the saponin, the carboxyl group in the saccharide moiety at C-3 of the aglycone, i.e. in a glucuronic acid moiety, and the acetyl group in a saccharide unit in the (oligo-)saccharide moiety bound at C-28 of the aglycone of the saponin, results in a decrease in cytotoxicity when such saponin derivatives are contacted with cells, i.e. various types of cells. The decrease in cytotoxicity has been established by the inventors for the series of varying saponin derivatives listed in Table A2, Table A3 and Figures 1-14 and 36-40. It is thus part of the invention that the series of SO1861 saponin derivatives with decreased cytotoxicity are provided, wherein the decrease in cytotoxicity is relative to the cytotoxicity as determined for the unmodified naturally occurring saponin counterparts. The saponin derivatives can be formed from such naturally occurring saponins. Typically, saponin derivatives of the invention comprise one, two or three derivatisations when compared to the naturally occurring counterpart, present in nature, of SO1861. When the decrease in cytotoxicity is considered, saponin derivatives comprising one, two or three modifications (derivatisations) at the sites in the saponin molecule as outlined here above, are equally suitable, when saponins with decreased cytotoxicity are to be provided. Furthermore, the inventors surprisingly established that a large variety of different modifications are suitable for lowering cytotoxicity, lowering haemolytic activity, and for preserving and remaining sufficiently extent of endosomal escape enhancing activity. When haemolytic activity is considered, similar to decreased cytotoxicity, haemolytic activity is decreased when one, two or three of the indicated chemical groups in the saponin are derivatised. These derivatisations can be of various nature, such as those derivatisations outlined in Table A2, and the Figures 1-14 and 36-37. Both derivatisations as small as the derivatisation of the aldehyde group by reduction into a hydroxyl group decrease cytotoxicity and haemolytic activity, and as large as the derivatisation of the aldehyde group with EMCH, combined with the derivatisation of the carboxyl group of the glucuronic acid with AEM. It is apparent that for providing a saponin derivative with improved cytotoxicity in terms of a decreased cytotoxicity, and with improved haemolytic activity in terms of a decreased haemolytic activity, both when compared with the naturally occurring saponin counterpart, any one or more, such as one, two or three of the three chemical groups in the saponin can be derivatised by a wide array of different chemical groups with varying size and/or with a varying chemical properties.

Without wishing to be bound by any theory, it is assumed that the aldehyde group at the C-3 atom of the aglycone of the saponin relates and/or contributes to the endosomal escape enhancing activity of bidesmosidic triterpene glycoside type of saponins, i.e. for example the increased toxicity of (protein) toxins when contacted with cells in the presence of such saponins, compared to the toxicity of such toxins when the same dose is contacted to the same cells in the absence of such saponins, both in vitro and in vivo. Indeed, the inventors established that saponin derivatives with a derivatised carboxyl group in the glucuronic acid unit, and/or a derivatised acetyl group in the polysaccharide chain, and comprising the free aldehyde group in the aglycone, have endosomal escape enhancing activity. These derivatives have decreased haemolytic activity and decreased cytotoxicity. For example, the saponin derivatives as molecules 3A, 8, 11, 18, 19 and 28 (Table A2, Table A3, Table A5, Table A6, Figures 1, 3, 6, 11, 12, 39) have a free unmodified aldehyde group in the aglycone core, and indeed display activity when the enhancement of the cytotoxicity of antibody-drug conjugates which are contacted with various (tumor) cells expressing the receptor to which the antibody binds, is considered. These saponin derivatives, when based on SO1861 saponin, are thus explicitly envisaged embodiments of the invention.

Surprisingly, also saponin derivatives with a derivatised aldehyde group in the aglycone, such that the saponin derivative does not comprise the free aldehyde group, still display the characteristic endosomal escape enhancing activity when the cytotoxicity of an effector molecule provided to (tumor) cells in the form of a ligand-toxin conjugate, e.g. an ADC, and with the prerequisite that none or only one of the acetyl group in the polysaccharide chain at C-28 and the carboxyl group in the polysaccharide chain at C-23 is derivatised. For example, the saponin derivatives with a modified aldehyde group and with none or a single further derivatisation, indicated as molecules 6, 9, 10, 14, 15, 20, 27 and 29 in Table A2, Table A3 and Figures 2, 4, 5, 8, 9, 13, 38 and 40, have the capacity to enhance the cytotoxic effect of effector molecules that are contacted with tumor cells in the presence of such saponin derivatives with derivatised aldehyde group in the aglycone. All these saponin derivatives display decreased cytotoxicity and display decreased haemolytic activity and, when based on SO1861 saponin, are hence explicitly envisaged embodiments of the invention.

The inventors have also found that certain modifications lead to an increased critical micelle concentration (CMC) when compared with the corresponding unmodified saponin. For example, the saponin derivatives indicated as molecules 2, 6, 8, 10, 15, 27 and 28, preferably the saponin derivatives indicated as molecules 2, 6, 8, 10, and 15 have an increased CMC when compared to their corresponding underivatised saponin and are hence explicitly envisaged embodiments of the invention. Without wishing to be bound by any theory, it is believed that an increased CMC is advantageous for several reasons. For example, an increased CMC may facilitate the use of the modified saponins in subsequent conjugation reactions since free molecules are generally more susceptible to conjugation reactions than molecules ordered in a micellar structure. Furthermore, in case the saponin derivatives need to exert a biological function (e.g. in an *in vivo* treatment or *ex vivo* method or *in vitro* method), for example in case the saponin derivatives are used as such or even in case they are released *in-situ* after cleavage from a carrier or another entity, an increased CMC when compared to unmodified saponin is advantageous since the free saponin molecules will be more readily available to interact with their biological target than in case these saponin derivatives are ordered in a micellar structure. An increased CMC may also be useful to facilitate the large scale production and concentration of the saponin derivatives since at concentrations beyond (above) the critical micellar concentration, saponins form micelles which hinder isolation (e.g. using preparative HPLC). Surprisingly, for the saponin derivatives according to the invention the observed increased CMC was not associated with increased cytotoxicity or hemolytic activity. The relationship between CMC and cytotoxicity is not predictable and complex, as can for example be seen from the data in Table 2 of de Groot et al. ("Saponin interactions with model membrane systems-Langmuir monolayer studies, hemolysis and formation of ISCOMs", Planta medica 82.18 (2016): 1496-1512.), which shows that, taking α-Hederin as the reference point, an increase in CMC may be associated with an increase in general cytotoxicity (as is the case for Digitonin) but may just as well be associated with a decrease in cytotoxicity (as is the case for Glycyrrhizin and Hederacoside C). Furthermore, for the saponin derivatives indicated as molecules 2, 6, 10, and 15 the increased CMC is also associated with an increased Ratio: IC50 hemolysis / IC50 activity, compared to the corresponding free saponin, such that these saponin derivatives are particularly preferred embodiments of the invention.

The inventors thus provide saponin derivatives with an improved therapeutic window when cytotoxicity is considered and/or when haemolytic activity is considered, and when the potentiation of e.g. toxins is considered and/or an increased CMC compared to the corresponding underivatised saponin. Such saponin derivatives of the invention are in particular suitable for application in a therapeutic regimen involving e.g. an ADC or an AOC for the prophylaxis or treatment of e.g. a cancer. The safety of such saponin derivatives is improved when cytotoxicity and/or haemolytic activity is considered, especially when such saponin derivatives are administered to a patient in need of e.g. treatment with an ADC or with and AOC.

An embodiment is the saponin derivative according to the invention, wherein the saponin derivative comprises the first saccharide chain wherein the first saccharide chain comprises a carboxyl group of a glucuronic acid moiety, which has been derivatised, and/or wherein the saponin derivative comprises the second saccharide chain wherein the second saccharide chain comprises an acetoxy (Me(CO)O-) group which has been derivatised (here also referred to as derivatised acetate group), preferably the saponin derivative comprises both of said first saccharide chain which has been derivatised and said second saccharide chain which has been derivatised, more preferably, the saponin derivative comprises both of said first saccharide chain which has been derivatised and said second saccharide chain which has been derivatised and the saponin derivative comprises an aglycone core structure comprising an aldehyde group or an aldehyde group which has been derivatised, most preferably, the saponin derivative comprises both of said first saccharide chain which has been derivatised and said second saccharide chain which has been derivatised and the saponin derivative comprises an aglycone core structure comprising an aldehyde group. Equally preferred are all other possible combinations of two of such derivatisations, leaving one of these three chemical groups in the saponin unaltered when the naturally occurring saponin is considered. Furthermore, the one, two or three, preferably one or two, of the chemical groups in the saponin are derivatised according to any one or more of the listed derivatisations in Table A2.

According to the invention the saponin derivative comprises an aglycone core structure being quillaic acid. In examples not claimed, the saponin derivative comprises an aglycone core structure selected from the group consisting of:
2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en;
gypsogenic acid,
   and
derivatives thereof.

Since the inventors now found that improved SO1861 based saponin derivatives can be provided with regard to decreased cytotoxicity and lower haemolysis of cells contacted with such derivatives, based on saponins of the triterpene glycoside type, basically any saponin with such endosomal escape enhancing activity as tested by the inventors, which is SO1861 based saponins having the aglycone of the afore embodiment and listed in Table A1, can be improved accordingly. Lowering toxicity and lowering haemolytic activity while preserving activity to a sufficiently high extent when potentiation of toxins and for example BNAs is considered, is an important achievement by the inventors, when the widening of the therapeutic window of the saponin derivatives alone or in combination with e.g. an ADC or an AOC is considered. A sufficiently high dose of derivatised saponin can be applied in e.g. tumor therapy for a cancer patient in need thereof, while the (risk for) cytotoxic side-effects and the (risk for) undesired haemolytic activity exerted or induced by the saponin derivative is decreased when compared with the application of the natural saponin counterpart. Improvements of the therapeutic window of the saponin derivatives of the invention are for example apparent for the exemplified saponin derivatives, based on SO1861, shown in Table A5 and Table A6: the ratio between the IC50 for either cytotoxicity, or haemolytic activity and the IC50 for endosomal escape enhancing activity are listed, as well as the haemolytic activity, cytotoxicity and the activity.

The saponin derivative according to the invention, wherein the saponin derivative comprises an aglycone core structure being quillaic acid, wherein the first saccharide chain, is linked to the C₃ atom (also denoted as 'C-3' atom) of the aglycone core structure, and wherein the second saccharide chain, is linked to the C₂₈ atom of the aglycone core structure.

The saponin derivative according to the invention, comprises the first saccharide chain Gal-(1→2)-[Xyl-(1→3)]-GlcA-, and the second saccharide chain Glc-(1→3)-Xyl-(1→4)-Rha-(1→2)-[Xyl-(1→3)-4-OAc-Qui-(1→4)]-Fuc-.

In examples which are not claimed, the first saccharide chain, if present, is selected from (list S1):
GlcA-,
Glc-,
Gal-,
Rha-(1→2)-Ara-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof,
and/or wherein the second saccharide chain, if present, is selected from (list S2):
Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1→4)-Rha-(1→2)-[R1-(→4)]-Fuc- wherein R1 is 4E-Methoxycinnamic acid,
Xyl-(1→4)-Rha-(1→2)-[R2-(→4)]-Fuc- wherein R2 is 4Z-Methoxycinnamic acid,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- wherein R3 is 4E-Methoxycinnamic acid,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- or Xyl-)(1→3)-(Ara- or Xyl-)(1→4)-(Rha- or Fuc-)(1→2)-[4-OAc-(Rha- or Fuc-)(1→4)]-(Rha- or
Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Xyl-(1→3)-Xyl-(1→4)-Rha-(1→2)-[3,4-di-OAc-Qui-(1→4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
Glc-(1→3)-[Glc-(1→6)]-Gal-, and
derivatives thereof.

Typically, saponins that enhance cytotoxicity of toxins, when cells are contacted with the saponin and the toxin, have one or two of such mono- or polysaccharide chains bound to the aglycone. Preferred are those saponins selected for derivatisation that comprise two saccharide chains. An overview of particularly preferred saponins for subjecting such saponins to single, double or triple derivatisation, preferably single or double derivatisation when endosomal escape enhancing activity should be preserved to sufficiently high extent, is provided in Table A1, wherein SO1861 is claimed and wherein other saponin examples are not claimed. Of course, structural variants of such saponins are equally suitable for derivatisation according to the invention, if such saponins display endosomal escape enhancing activity towards e.g. a toxin, a BNA, etc.

According to the invention, the saponin derivative comprises the first saccharide chain and comprises the second saccharide chain, wherein the aglycone core structure is quillaic acid, wherein one, two or three, preferably one or two, of:
i. an aldehyde group in the aglycone core structure has been derivatised,
ii. the first saccharide chain comprises a carboxyl group of a glucuronic acid moiety which has been derivatised, and
iii. the second saccharide chain comprises an acetoxy (Me(CO)O-) group which has been derivatised.

The following Summary illustrates suitable derivatisations:

| **Functional Group** | **Derivatisation** |
|---|---|
| **Aldehyde** (chemoselective, reversible) | - hydrazone formation with hydrazides |
| | - imine formation with amines |
| | - chemoselective reversible oxime formation with hydroxylamines |
| **Acetoxy** (chemoselective) | - deacetylation resulting in alcohol |
| **Carboxylic acid** (chemoselective) | - amide formation with amines or ester formation with alcohols (after activation) |

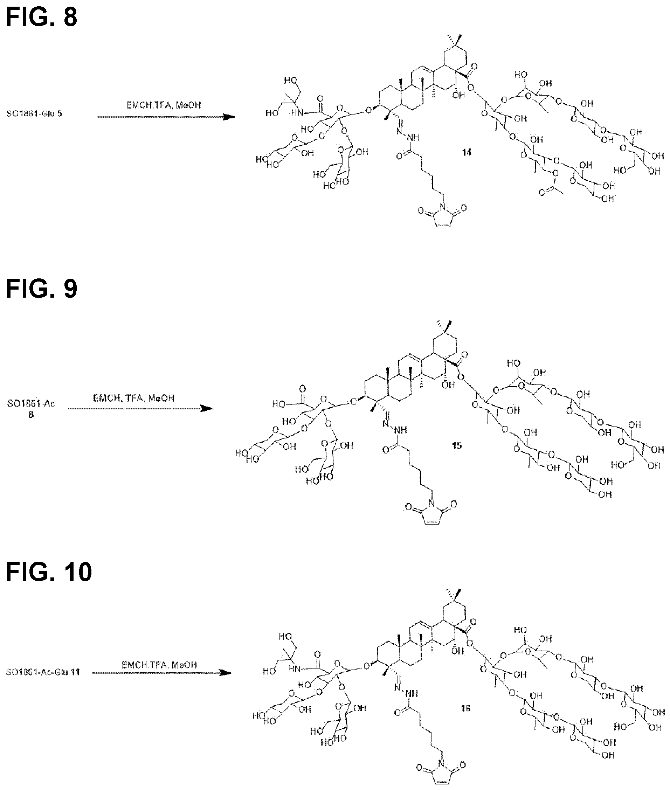

According to the invention, a saponin can comprise three derivatisations and still display sufficiently high endosomal escape enhancing activity. In particular when the decrease in cytotoxicity and/or haemolytic activity is larger than the (potential or apparent) decrease of the ability to potentiate the effect and activity of an effector molecule inside a cell, such as a toxin or a BNA in a tumor cell contacted with the effector molecule and the derivatised saponin. Thus, the invention provides derivatised saponin comprising a single, two or three derivatisations, when the aldehyde group of the aglycone is considered, when the carboxyl group in the glucuronic acid unit in the polysaccharide at C-3 is considered, if present, and when the acetyl group in the polysaccharide chain at C-28 is considered, if present. Preferred is a saponin derivative having one or two modifications. Suitable for improving endosomal escape of an effector molecule such as a toxin or a BNA are for example saponin derivatives with a free aldehyde group and with one or two derivatisations in saccharide chains. As said before, also saponin derivatives with a derivatised aldehyde group are equally suitable. Such saponin derivatives that do not have the free aldehyde group in the aglycone upon the derivatisation, still display sufficient and efficient endosomal escape enhancing activity. Without wishing to be bound by any theory, as a result of the acidic conditions in the endosome and in the lysosome of (mammalian) cells such as human cells, an aldehyde group may again be formed inside the cell upon pH driven cleavage of the moiety initially bound to the aldehyde group of the saponin for providing the saponin derivative with derivatised aglycone at position C-23. An example of a saponin derivative with a modified aldehyde group which may be formed again in the endosome or lysosome, is a saponin derivative comprising a hydrazone bond which is formed between the carbonyl group of the aldehyde and for example a hydrazide moiety in a chemical group bound to the aglycone, such as N-ε-maleimidocaproic acid hydrazide (EMCH), or EMCH with mercaptoethanol bound to the maleimide group, forming a thio-ether bond. Examples of such saponin derivatives are provided in Figure 8 and Figure 9, and are displayed as Molecule 2 and Molecule 3, here below.

In examples not claimed, the saponin derivative is a derivative of a saponin selected from the group of saponins consisting of: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, GE1741, SO1542, SO1584, SO1658, SO1674, SO1832, SO1904, SO1862, QS-7, QS1861, QS-7 api, QS1862, QS-17, QS-18, QS-21 A-apio, QS-21 A-xylo, QS-21 B-apio, QS-21 B-xylo, beta-Aescin, Aescin la, Teaseed saponin I, Teaseedsaponin J, Assamsaponin F, Digitonin, Primula acid 1 and AS64R, stereoisomers thereof and combinations thereof, preferably the saponin derivative is selected from the group consisting of a QS-21 derivative, a SA1641 derivative and a GE1741 derivative, more preferably the saponin derivative is QS-21 derivative. According to the invention, the saponin derivative is SO1861 derivative. These saponins are essentially saponins displaying endosomal escape enhancing activity as established by the inventors, or that are structurally highly similar to saponins for which the endosomal escape enhancing activity has been established. Structural outline of these saponins is summarized in Table A1.

The saponin derivative according to the invention is a derivative of the quillaic acid saponin which is represented by Molecule 1: 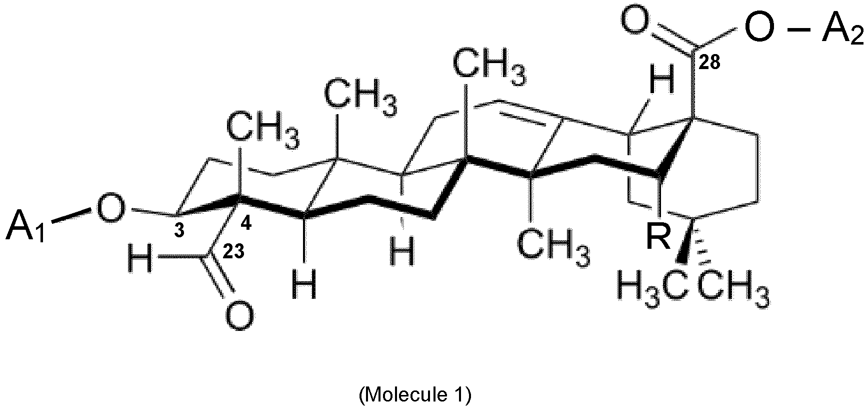 wherein
the first saccharide chain A₁ is Gal-(1→2)-[Xyl-(1→3)]-GlcA-;
and the second saccharide chain A₂ is Glc-(1→3)-Xyl-(1→4)-Rha-(1→2)-[Xyl-(1→3)-4-OAc-Qui-(1→4)]-Fuc-;
and R is hydroxyl;
wherein the saponin derivative corresponds to the saponin represented by Molecule 1 wherein at least one of the following derivatisations is present:
i. the aldehyde group at position C₂₃ of the quillaic acid has been derivatised;
ii. the carboxyl group of a glucuronic acid moiety of A₁ has been derivatised; and
iii. the acetoxy group(s) of A₂ has/have been derivatised.

An embodiment is the saponin derivative according to the invention, wherein A₁ is Gal-(1→2)-[Xyl-(1→3)]-GlcA- and wherein the carboxyl group of a glucuronic acid moiety of A₁ has been derivatised and/or wherein A₂ is Glc-(1→3)-Xyl-(1→4)-Rha-(1→2)-[Xyl-(1→3)-4-OAc-Qui-(1→4)]-Fuc- and wherein said acetoxy group of A₂ has been derivatised.

According to the invention, the saponin represented by Molecule 1 is a bidesmosidic triterpene saponin.

An embodiment is the saponin derivative according to the invention, wherein the saponin derivative corresponds to the saponin represented by Molecule 1 wherein at least one of the following derivatisations is present, preferably one or two of the following derivatisations is present, more preferably one:
i. the aldehyde group at position C₂₃ of the quillaic acid has been derivatised by;
   - reduction to an alcohol;
   - transformation into a hydrazone bond, preferably through reaction with a hydrazide;
ii. the carboxyl group of a glucuronic acid moiety of A₁has been derivatised by transformation into an amide bond, preferably through reaction with an amine; and
iii. the acetoxy group(s) of A₂has/have been derivatised by transformation into a hydroxyl group (HO-) by deacetylation.

An embodiment is the saponin derivative according to the invention, wherein the saponin derivative corresponds to the saponin represented by Molecule 1 wherein at least one of the following derivatisations is present, preferably one or two of the following derivatisations is present, more preferably one:
i. the aldehyde group at position C₂₃ of the quillaic acid has been derivatised by;
   - reduction to an alcohol;
   - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
   - transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;
ii. the carboxyl group of a glucuronic acid moiety of A₁has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a SO1861-Glu-AMPD or a saponin-Glu-AEM such as a a SO1861-Glu-AEM; and
iii. the acetoxy group(s) of A₂has/have been derivatised by transformation into a hydroxyl group (HO-) by deacetylation.

The saponin derivative according to the invention, wherein the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAc-beta-D-quinovopyranosyl-(1→4)]-beta-D-fucopyranoside, SO1861.

In examples not claimed, the saponin derivative is selected from the group consisting of derivatives of: SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1862, SO1904, stereoisomers thereof and combinations thereof. The saponin derivative according to the invention is a SO1861 derivative.

An embodiment is the saponin derivative according to the invention, wherein the saponin derivative is a SO1861 derivative comprising a single derivatisation, wherein the single derivatisation is transformation of a carboxyl group of a glucuronic acid moiety of SO1861, such as by binding 1-[Bis(dimethylamino)methylene]-1H-1,2,3-triazolo[4,5-b]pyridinium 3-oxid hexafluorophosphate (HATU) to the carboxyl group of the glucuronic acid moiety of SO1861 (See Figure 59) or by binding (benzotriazol-1-yloxy)tris(dimethylamino)phosphonium hexafluorophosphate (BOP) to the carboxyl group of the glucuronic moiety of SO1861, or wherein the saponin derivative is a SO1861 derivative represented by Molecule 2, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH): 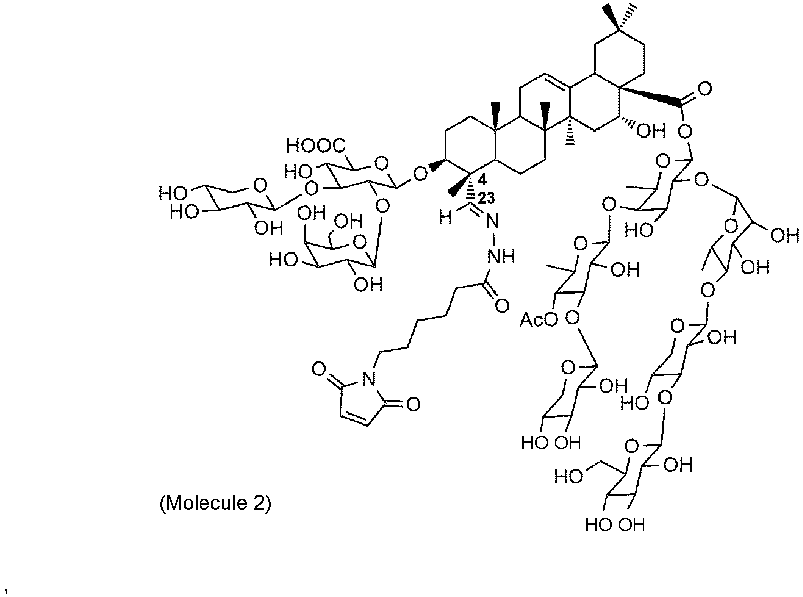 or wherein the saponin derivative is a SO1861 derivative represented by Molecule 3, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH) wherein the maleimide group of the EMCH is derivatised with mercaptoethanol therewith forming a thio-ether bond:

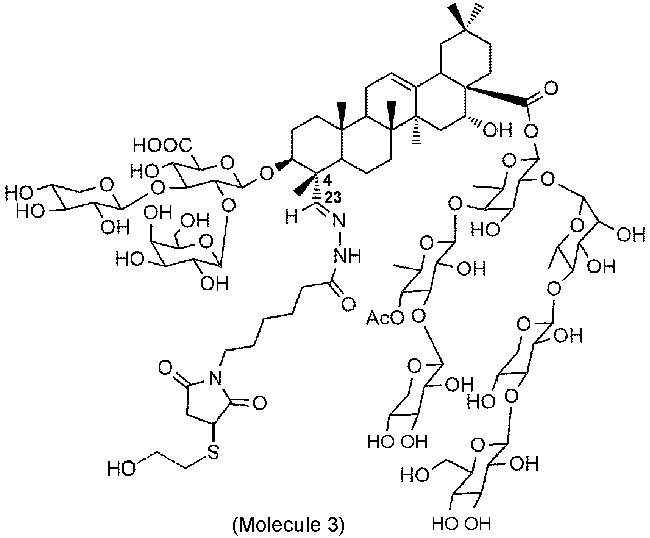

The saponin represented by Molecule 2 is suitable for application as a precursor for a conjugation reaction with a further molecule comprising a free sulfhydryl group. The maleimide group of the saponin derivative displayed as Molecule 2 can form a thio-ether bond with such a free sulfhydryl group. For example, the saponin derivative of Molecule 2 can be covalently coupled to a peptide or a protein which comprises a free sulfhydryl group such as a cysteine with a free sulfhydryl group. Such a protein is for example an antibody or a binding fragment or binding domain thereof, such as Fab, scFv, single domain antibody, such as V_{HH}, for example camelid V_{H}. Application of the saponin derivative of Molecule 2 in a coupling reaction with e.g. an antibody that comprises a free sulfhydryl group, provides a conjugate for targeted delivery of the saponin to and inside a cell, when the antibody (or the binding domain or fragment thereof) is an antibody for specific binding to a target cell surface molecule such as a receptor, e.g. as present on a tumor cell. Preferably, the saponin derivative is coupled to an antibody or V_{HH} capable of binding to a tumor-cell specific surface molecule such as a receptor, e.g. HER2, EGFR, CD71.

An embodiment is the saponin derivative according to the invention, with the proviso that the saponin derivative is not a SO1861 derivative comprising a single derivatisation, wherein the single derivatisation is transformation of the carboxyl group of a glucuronic acid moiety of SO1861 by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, or wherein the saponin derivative is a SO1861 derivative represented by Molecule 2, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH):

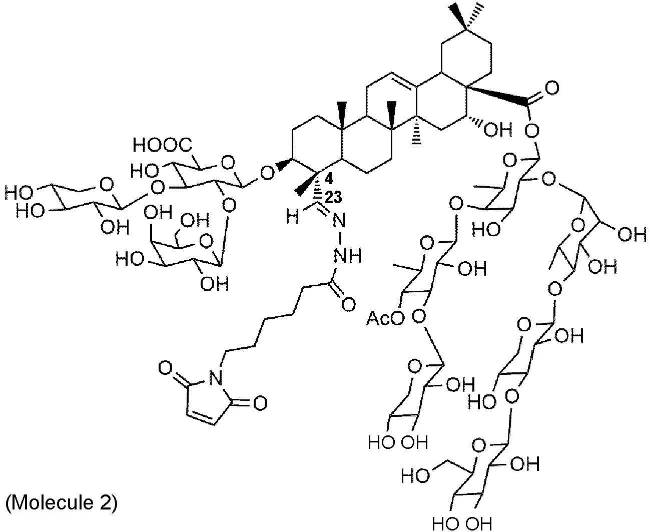

. An embodiment is the saponin derivative according to the invention, wherein
i. the saponin derivative comprises an aglycone core structure wherein the aglycone core structure comprises an aldehyde group which has been derivatised by:
   - reduction to an alcohol;
   - transformation into a hydrazone bond through reaction with N-ε-maleimidocaproic acid hydrazide (EMCH) wherein the maleimide group of the EMCH is optionally derivatised by formation of a thio-ether bond with mercaptoethanol;
   - transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;
ii. the first saccharide chain comprises a carboxyl group of a glucuronic acid moiety, which has been derivatised by transformation into an amide bond through reaction with 2-amino-2-methyl-1,3-propanediol (AMPD) or *N*-(2-aminoethyl)maleimide (AEM);
iii. the second saccharide chain comprises an acetoxy group (Me(CO)O-) which has been derivatised by transformation into a hydroxyl group (HO-) by deacetylation; or
iv. the saponin derivative comprises any combination of two or three derivatisations i., ii. and iii., preferably any combination of two derivatisations i., ii. and iii.;
preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thio-ether bond with mercaptoethanol.

An embodiment is the saponin derivative according to the invention, wherein the saponin derivative comprises an aglycone core structure wherein the aglycone core structure comprises an aldehyde group and wherein the first saccharide chain comprises a carboxyl group of a glucuronic acid moiety, which has been derivatised by transformation into an amide bond through reaction with N-(2-aminoethyl)maleimide (AEM).

An embodiment is the saponin derivative according to the invention, with the proviso that when the aldehyde group in the aglycone core structure is derivatised by transformation into a hydrazone bond through reaction with N-ε-maleimidocaproic acid hydrazide (EMCH) and the saponin is SO1861, at least one of the glucuronic acid and the acetoxy group (Me(CO)O-) is also derivatised, and with the proviso that when the saponin is SO1861 and the carboxyl group of the glucuronic acid moiety of SO1861 is derivatised by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, at least one of the aldehyde group and the acetoxy group (Me(CO)O-) is also modified.

An embodiment is the saponin derivative according to the invention, with the proviso that when the aldehyde group in the aglycone core structure of the saponin derivative is derivatised through reaction with EMCH and the saponin is SO1861, at least one of the glucuronic acid and the acetoxy group (Me(CO)O-) is also derivatised, and with the proviso that when the saponin is SO1861 and the carboxyl group of the glucuronic acid moiety of SO1861 is derivatised by bound HATU, at least one of the aldehyde group and the acetoxy group (Me(CO)O-) is also derivatised.

An embodiment, referred to herein as embodiment D1, is the saponin derivative according to the invention, wherein the aglycone core structure comprises an aldehyde group which has been derivatised by transformation, such as via reductive amination, into an amine by reaction with a compound of formula (A1):

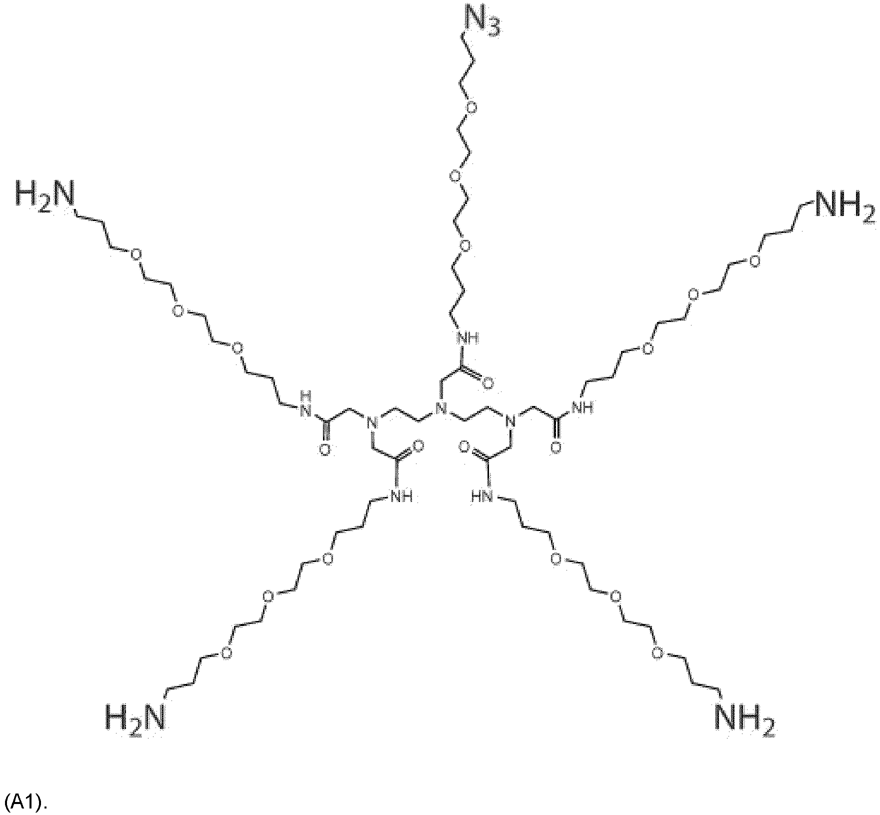

That is to say, the embodiment, referred to herein as embodiment D1, is the saponin derivative according to the invention, characterized in that it is not a result of a reaction between SA1641 and a compound of formula (A1). Thus, the embodiment D1 is the saponin derivative according to the invention, characterized in that it is not a result of the coupling via reductive amination of at least one SA1641 molecule to a compound of formula (A1).

An embodiment, referred to herein as embodiment D2, is the saponin derivative according to the invention, characterized in that the saponin derivative is not a SO1861, wherein the aglycone core structure comprises an aldehyde group which has been derivatised by transformation into a hydrazone bond through reaction with N-ε-maleimidocaproic acid hydrazide (EMCH) wherein the maleimide group of the EMCH is optionally derivatised by formation of a thio-ether bond with a thiol, and wherein no other derivatisations are present on the saponin, preferably characterized in that the saponin derivative is not a saponin, in particular SO1861, wherein the aglycone core structure comprises an aldehyde group which has been derivatised by transformation into a hydrazone bond through reaction with N-ε-maleimidocaproic acid hydrazide (EMCH) wherein the maleimide group of the EMCH is optionally derivatised by formation of a thio-ether bond with a thiol.

An embodiment, referred to herein as embodiment D3, is the saponin derivative according to the invention, characterized in that the saponin derivative is not a SO1861, wherein the aglycone core structure comprises an aldehyde group which has been derivatised by transformation into a hydrazone bond through reaction with N-ε-maleimidocaproic acid hydrazide (EMCH) wherein the maleimide group of the EMCH is derivatised by formation of a thio-ether bond with a thiol selected from one, preferably all of:
- mercaptoethanol,
- a poly(amidoamine) dendrimer having an ethylenediamine core which has been derivatised with at least 2-iminothiolane,
- a conjugate of cyanin-3 and a poly(amidoamine) dendrimer having an ethylenediamine core which has further been derivatised with at least 2-iminothiolane,
- a G4 dendron which has been derivatised with at least 2-iminothiolane,
- a conjugate of cyanin-5 and a G4 dendron which has further been derivatised with at least 2-iminothiolane,
- bovine serum albumin (BSA), and
- a peptide with the sequence SESDDAMFCDAMDESDSK [SEQ ID NO: 1]
and wherein no other derivatisations are present on the saponin. As will be understood by the person skilled in the art, the expression "G4 dendron" should be interpreted to mean a compound of formula (A2):

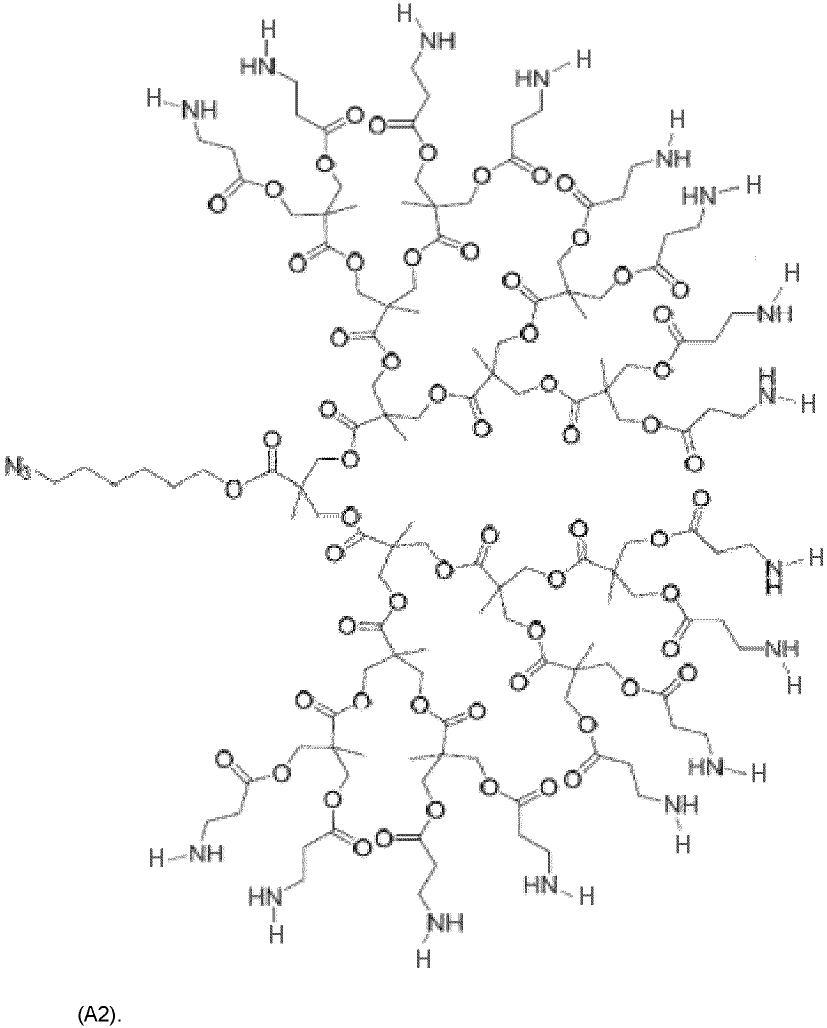

An embodiment, referred to herein as embodiment D4, is the saponin derivative according to the invention, characterized in that the saponin derivative is not a SO1861, wherein a carboxyl group has been derivatised by transformation into an amide by reaction with an optionally further derivatised conjugate of cyanin-3 and a poly(amidoamine) dendrimer having an ethylenediamine core, and wherein no other derivatisations are present on the saponin, preferably characterized in that the saponin derivative is not a saponin, in particular SO1861, wherein a carboxyl group has been derivatised by transformation into an amide by reaction with an optionally further derivatised conjugate of cyanin-3 and a poly(amidoamine) dendrimer having an ethylenediamine core.

An embodiment, referred to herein as embodiment D5, is the saponin derivative according to the invention, characterized in that the saponin derivative is not a SO1861, wherein the aglycone core structure comprises an aldehyde group which has been derivatised by transformation, such as via reductive amination, into an amine by reaction with a conjugate of cyanin-3 and a poly(amidoamine) dendrimer having an ethylenediamine core, and wherein no other derivatisations are present on the saponin, preferably characterized in that the saponin derivative is not a saponin, in particular SO1861, wherein the aglycone core structure comprises an aldehyde group which has been derivatised by transformation, such as via reductive amination, into an amine by reaction with a conjugate of cyanin-3 and a poly(amidoamine) dendrimer having an ethylenediamine core.

An embodiment, referred to herein as embodiment D6, is the saponin derivative according to the invention, characterized in that the saponin derivative does not comprise a toxin, a micro RNA, or a polynucleotide encoding a protein, preferably in that the saponin derivative does not comprise a pharmaceutically active substance, such as a toxin, a drug, a polypeptide and/or a polynucleotide, more preferably characterized in that the saponin derivative does not comprise an effector molecule.

An embodiment, referred to herein as embodiment D7, is the saponin derivative according to the invention, characterized in that the saponin derivative does not comprise a polymeric or oligomeric structure, selected from the group consisting of
- poly- or oligo(amines), such as polyethylenimine and poly(amidoamine),
- polyethylene glycol,
- poly- or oligo(esters), such as poly(lactids),
- poly(lactams),
- polylactide-co-glycolide copolymers,
- poly- or oligosaccharides, such as cyclodextrin and polydextrose,
- poly- or oligo(amino acids), such as proteins and peptides, and
- nucleic acids and analogues thereof, such as DNA, RNA, LNA (locked nucleic acid) and PNA (peptide nucleic acid);
preferably characterized in that the saponin derivative does not comprise a polymeric or oligomeric structure which is a structurally ordered formation such as a polymer, oligomer, dendrimer, dendronized polymer, or dendronized oligomer or it is an assembled polymeric structure such as a hydrogel, microgel, nanogel, stabilized polymeric micelle or liposome, more preferably characterized in that the saponin derivative does not comprise a polymeric or oligomeric structure.

An embodiment, referred to herein as embodiment D8, is the saponin derivative according to the invention, characterized in that the saponin derivative does not comprise a molecular structure built up chiefly or completely from at least 2 equal or similar units bonded together.

An embodiment, referred to herein as embodiment D9, is the saponin derivative according to the invention, characterized in that the saponin derivative is not the compound of formula (A3), which is a reaction product of SO1861 and N-[(Dimethylamino)-1H-1,2,3-triazolo-[4,5-b]pyridin-1-ylmethylene]-N-methylmethanaminium hexafluorophosphate N-oxide (HATU): 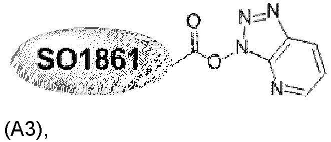 preferably characterized in that the saponin derivative is not an activated ester. See Figure 59.

An embodiment, referred to herein as embodiment D10, is the saponin derivative according to the invention, characterized in that the saponin derivative is not a SO1861 wherein a carboxyl group has been derivatised by transformation into an amide bond or an ester bond, and wherein no other derivatisations are present on the saponin, preferably characterized in that the saponin derivative is not a SO1861 wherein a carboxyl group has been derivatised by transformation into an amide bond or an ester bond.

An embodiment, referred to herein as embodiment D11, is the saponin derivative according to the invention, characterized in that the saponin derivative does not comprise a dianthin moiety.

A preferred embodiment, referred to herein as embodiment D12, is the saponin derivative according to the invention, characterized in that the saponin derivative comprises a single saponin moiety.

A preferred embodiment, referred to herein as embodiment D13, is the saponin derivative according to the invention, characterized in that the saponin derivative has a molecular weight of less than 2500 g/mol, preferably less than 2300 g/mol, more preferably less than 2150 g/mol.

A preferred embodiment, referred to herein as embodiment D14, is the saponin derivative according to the invention, characterized in that the saponin derivatisation has a molecular weight of less than 400 g/mol, preferably less than 300 g/mol, more preferably less than 270 g/mol. The molecular weight of the saponin derivatisation corresponds to the molecular weight of the saponin derivative exclusive of the aglycone core and the two (for bidesmosidic saponins) glycon (sugar) chains. The skilled person will understand that in case the saponin derivative has a lower molecular weight than its corresponding underivatised saponin (e.g. as is the case for SO1861-Ac-OH which corresponds to SO1861 derivatised by deacetylation), the saponin derivatisation does not bring any increase in molecular weight and thus complies with the requirement that that the saponin derivatisation has a molecular weight of less than 400 g/mol, preferably less than 300 g/mol, more preferably less than 270 g/mol of embodiment D14.

As will be understood by the skilled person, embodiments D1-D14 may be combined amongst each other, as well as with the other embodiments described in the present application. For example, in embodiments of the invention the following combinations of embodiments D1-D14 are provided:
- D12 and one or more of D1-D11, D13;
- D13 and one or more of D1-D12;
- D12, D13 and one or more of D1-D11;
- D1, D2, D10 and D12;
- D1, D3, D7, D9 and preferably D13; or
- D3, D9, D12 and D13.

It will be understood by the skilled person that these combinations of embodiments D1-D14 may again be combined with other embodiments according to the invention for example, and preferably, with embodiment D14.

A particularly preferred embodiment corresponds to a combination of embodiments D3, D9, D12 and one or both of D13 and D14. In other words, a particularly preferred embodiment is the saponin derivative according to the invention wherein the saponin derivative comprises a single saponin moiety, wherein the saponin derivative has a molecular weight of less than 2500 g/mol, preferably less than 2300 g/mol, more preferably less than 2150 g/mol, and wherein the saponin derivative
- is not a SO1861, wherein the aglycone core structure comprises an aldehyde group which has been derivatised by transformation into a hydrazone bond through reaction with N-ε-maleimidocaproic acid hydrazide (EMCH) wherein the maleimide group of the EMCH is optionally derivatised by formation of a thio-ether bond with mercaptoethanol, and wherein preferably no other derivatisations are present on the saponin; and
- is not an activated ester which is the reaction product of SO1861 and N-[(Dimethylamino)-1H-1,2,3-triazolo-[4,5-b]pyridin-1-ylmethylene]-N-methylmethanaminium hexafluorophosphate N-oxide (HATU).

A second aspect of the invention relates to a first pharmaceutical composition comprising the saponin derivative according to the invention and optionally a pharmaceutically acceptable excipient and/or diluent.

An embodiment is the first pharmaceutical composition according to the invention comprising a saponin derivative according to the invention, preferably a pharmaceutically acceptable diluent, and further comprising:
- a pharmaceutically acceptable salt, preferably a pharmaceutically acceptable inorganic salt, such as an ammonium, calcium, copper, iron, magnesium, manganese, potassium, sodium, strontium or zinc salt, preferably NaCl; and/or
- a pharmaceutically acceptable buffer system, such as a phosphate, a borate, a citrate, a carbonate, a histidine, a lactate, a tromethamine, a gluconate, an aspartate, a glutamate, a tartarate, a succinate, a malate, a fumarate, an acetate and/or a ketoglutarate containing buffer system.

An embodiment is the first pharmaceutical composition according to the invention comprising a saponin derivative according to the invention and a pharmaceutically acceptable diluent, preferably water, wherein the composition is liquid at a temperature of 25°C and has a pH within the range of 2-11, preferably within the range of 4-9, more preferably within the range of 6-8.

An embodiment is the first pharmaceutical composition according to the invention comprising a saponin derivative according to the invention and a pharmaceutically acceptable diluent, preferably water, wherein the composition is liquid at a temperature of 25°C and wherein the concentration of the saponin derivative is within the range of 10⁻¹² to 1 mol/l, preferably within the range of 10⁻⁹ to 0.1 mol/l, more preferably within the range of 10⁻⁶ to 0.1 mol/l.

Typically, such a first pharmaceutical composition is suitable for use in combination with e.g. an ADC or an AOC. For example, the first pharmaceutical composition is administered to a patient in need of administration of the ADC or AOC, before the ADC or AOC is administered, together with the ADC or AOC, or (shortly) after administration of the ADC or the AOC to the patient in need of such ADC or AOC therapy. For example, the first pharmaceutical composition is mixed with a pharmaceutical composition comprising the ADC or the AOC, and a suitable dose of the mixture obtained is administered to a patient in need of ADC or AOC therapy. According to the invention, the saponin derivative comprised by the first pharmaceutical composition enhances the efficacy and potency of the effector molecule comprised by such an ADC or AOC, when the saponin derivative and the ADC or AOC co-localize inside a target cell such as a tumor cell. Under influence of the saponin derivative, the effector molecule is released into the cytosol of the target cell to a higher extent, compared to contacting the same cells with the same dose of ADC or AOC in the absence of the saponin derivative. Thus, similar efficacy can be obtained at lower ADC or AOC dose when the effector molecule co-localizes inside a target cell together with the saponin derivative of the first pharmaceutical composition, compared to the dose required to achieve the same efficacy in the absence of the saponin derivative inside the cell where the ADC or the AOC comprising the effector molecule is delivered.

An embodiment is the first pharmaceutical composition of the invention, wherein the saponin derivative is the saponin derivative represented by Molecule 2: 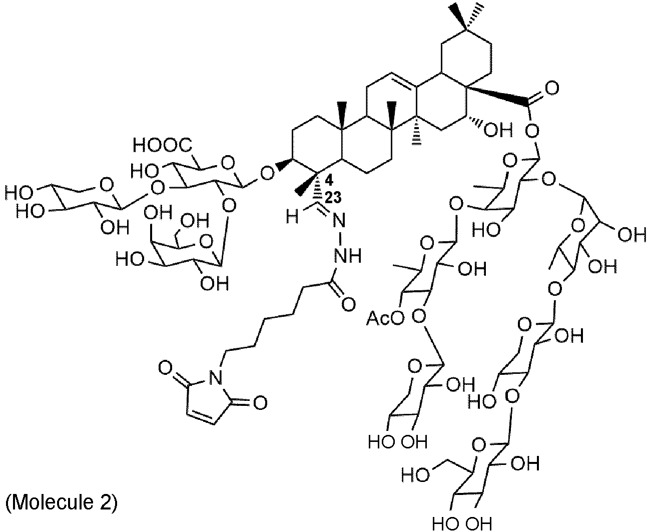 or SO1861 derivative comprising a single derivatisation, wherein the single derivatisation is transformation of the carboxyl group of the glucuronic acid moiety of SO1861 by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, or the saponin derivative is the saponin derivative represented by Molecule 3:

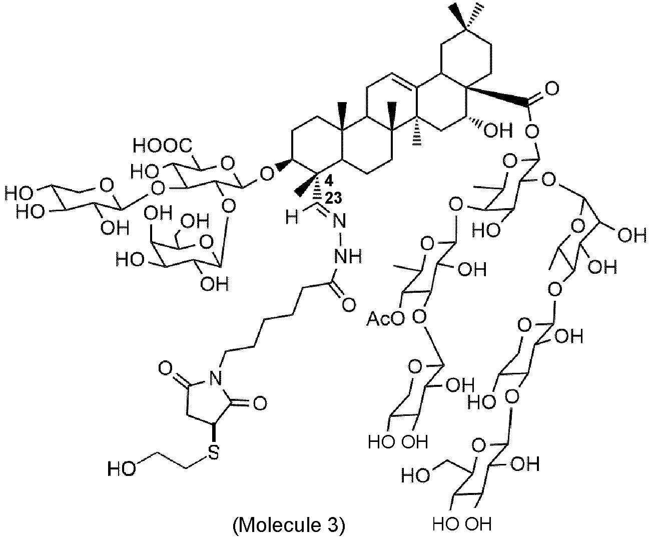

. A third aspect of the invention relates to a pharmaceutical combination comprising:
∘ the first pharmaceutical composition of the invention; and
∘ a second pharmaceutical composition comprising any one or more of an antibody-toxin conjugate, a receptor-ligand -toxin conjugate, an antibody-drug conjugate, a receptor-ligand - drug conjugate, an antibody-oligonucleotide conjugate or a receptor-ligand - oligonucleotide conjugate, and optionally comprising a pharmaceutically acceptable excipient and/or diluent.

A fourth aspect of the invention relates to a third pharmaceutical composition comprising the saponin derivative of the invention and further comprising any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate, and optionally comprising a pharmaceutically acceptable excipient and/or diluent.

An embodiment is the pharmaceutical combination of the invention or the third pharmaceutical composition of the invention, wherein the second pharmaceutical composition or the third pharmaceutical composition comprises any one or more of an antibody-drug conjugate, a receptor-ligand - drug conjugate, an antibody-oligonucleotide conjugate or a receptor-ligand - oligonucleotide conjugate, wherein the drug is for example a toxin such as saporin and dianthin, and wherein the oligonucleotide is for example an siRNA or a BNA, for example for gene silencing of apolipoprotein B or HSP27.

In examples not claimed of the pharmaceutical combination or the third pharmaceutical composition, wherein the saponin derivative is a saponin derivative selected from the group consisting of derivatives of: SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1862, SO1904, stereoisomers thereof and combinations thereof, preferably the saponin derivative is selected from the group consisting of a GE1741 derivative, a SA1641 derivative, a QS-21 derivative, and a combination thereof, more preferably the saponin derivative is a QS21 derivative. According to the invention, the saponin derivative is a SO1861 derivative, even more preferably the saponin derivative represented by Molecule 2 or Molecule 3.

An embodiment is the third pharmaceutical composition according to the invention comprising a saponin derivative according to the invention, preferably a pharmaceutically acceptable diluent, and further comprising:
- a pharmaceutically acceptable salt, preferably a pharmaceutically acceptable inorganic salt, such as an ammonium, calcium, copper, iron, magnesium, manganese, potassium, sodium, strontium or zinc salt, preferably NaCl; and/or
- a pharmaceutically acceptable buffer system, such as a phosphate, a borate, a citrate, a carbonate, a histidine, a lactate, a tromethamine, a gluconate, an aspartate, a glutamate, a tartarate, a succinate, a malate, a fumarate, an acetate and/or a ketoglutarate containing buffer system.

An embodiment is the third pharmaceutical composition according to the invention comprising a saponin derivative according to the invention and a pharmaceutically acceptable diluent, preferably water, wherein the composition is liquid at a temperature of 25°C and has a pH within the range of 2-11, preferably within the range of 4-9, more preferably within the range of 6-8.

An embodiment is the third pharmaceutical composition according to the invention comprising a saponin derivative according to the invention and a pharmaceutically acceptable diluent, preferably water, wherein the composition is liquid at a temperature of 25°C and wherein the concentration of the saponin derivative is within the range of 10⁻¹² to 1 mol/l, preferably within the range of 10⁻⁹ to 0.1 mol/l, more preferably within the range of 10⁻⁶ to 0.1 mol/l.

A fifth aspect of the invention relates to the first pharmaceutical composition of the invention, the pharmaceutical combination of the invention, or the third pharmaceutical composition of the invention, for use as a medicament. In preferred embodiments there is provided the first pharmaceutical composition of the invention wherein the saponin derivative comprises, preferably consists of SO1861-Ald-EMCH, SO1861-Ald-EMCH-mercaptoethanol, SO1861-L-N₃ or SO1861-Glu-HATU, the pharmaceutical combination of the invention wherein the saponin derivative comprises, preferably consists of SO1861-Ald-EMCH, SO1861-Ald-EMCH-mercaptoethanol, SO1861-L-N₃ or SO1861-Glu-HATU, or the third pharmaceutical composition of the invention wherein the saponin derivative comprises, preferably consists of SO1861-Ald-EMCH, SO1861-Ald-EMCH-mercaptoethanol, SO1861-L-N₃ or SO1861-Glu-HATU, for use as a medicament.

In another aspect of the invention there is provided the saponin derivative as described herein, preferably SO1861-Ald-EMCH, SO1861-Ald-EMCH-mercaptoethanol, SO1861-L-N₃ or SO1861-Glu-HATU for use as a medicament.

A sixth aspect of the invention relates to the first pharmaceutical composition of the invention, the pharmaceutical combination of the invention, or the third pharmaceutical composition of the invention, for use in the treatment or prophylaxis of a cancer, an infectious disease, viral infection, hypercholesterolemia, primary hyperoxaluria, haemophilia A, haemophilia B, alpha-1 antitrypsin related liver disease, acute hepatic porphyria, transthyretin-mediated amyloidosis, or an auto-immune disease. In preferred embodiments there is provided the first pharmaceutical composition of the invention wherein the saponin derivative comprises, preferably consists of SO1861-Ald-EMCH, SO1861-Ald-EMCH-mercaptoethanol, SO1861-L-N₃ or SO1861-Glu-HATU, the pharmaceutical combination of the invention wherein the saponin derivative comprises, preferably consists of SO1861-Ald-EMCH, SO1861-Ald-EMCH-mercaptoethanol, SO1861-L-N₃ or SO1861-Glu-HATU, or the third pharmaceutical composition of the invention wherein the saponin derivative comprises, preferably consists of SO1861-Ald-EMCH, SO1861-Ald-EMCH-mercaptoethanol, SO1861-L-N₃ or SO1861-Glu-HATU, for use in the treatment or prophylaxis of a cancer, an infectious disease, viral infection, hypercholesterolemia, primary hyperoxaluria, haemophilia A, haemophilia B, alpha-1 antitrypsin related liver disease, acute hepatic porphyria, transthyretin-mediated amyloidosis, or an auto-immune disease.

A seventh aspect of the invention relates to an *in vitro* or ex *vivo* method for transferring a molecule from outside a cell to inside said cell, preferably into the cytosol of said cell, comprising the steps of:
a) providing a cell;
b) providing the molecule for transferring from outside the cell into the cell provided in step a);
c) providing a saponin derivative according to the invention;
d) contacting the cell of step a) *in vitro* or *ex vivo* with the molecule of step b) and the saponin derivative of step c), therewith establishing the transfer of the molecule from outside the cell into said cell.

An embodiment is the method of the invention, wherein the cell is a human cell such as a T-cell, an NK-cell, a tumor cell, and/or wherein the molecule of step b) is any one of: an antibody-drug conjugate, a receptor-ligand - drug conjugate, an antibody-oligonucleotide conjugate or a receptor-ligand - oligonucleotide conjugate, wherein the drug is for example a toxin and wherein the oligonucleotide is for example an siRNA or a BNA. In examples not claimed, the saponin derivative is selected from the group consisting of derivatives of: SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1862, SO1904, stereoisomers thereof and combinations thereof. According to the invention, the saponin derivative is a SO1861 derivative; In embodiments, wherein the saponin derivative is a SO1861 derivative comprising a single derivatisation, wherein the single derivatisation is transformation of a carboxyl group of a glucuronic acid moiety of SO1861 into an amide bound through reaction with an amine, such as by binding 1-[Bis(dimethylamino)methylene]-1H-1,2,3-triazolo[4,5-b]pyridinium 3-oxid hexafluorophosphate (HATU) to the carboxyl group of the glucuronic acid moiety of SO1861 or by binding (benzotriazol-1-yloxy)tris(dimethylamino)phosphonium hexafluorophosphate (BOP) to the carboxyl group of the glucuronic moiety of SO1861, or wherein the saponin derivative is a SO1861 derivative represented by Molecule 2, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH): 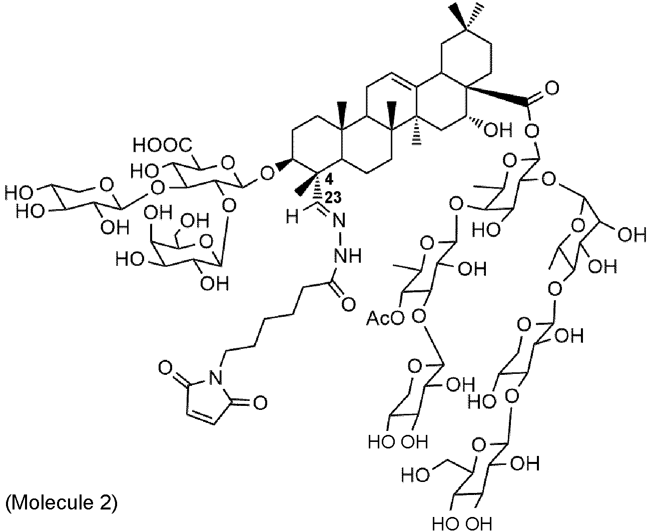 ,
or wherein the saponin derivative is a SO1861 derivative represented by Molecule 3, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH) wherein the maleimide group of the EMCH is derivatised with mercaptoethanol therewith forming a thio-ether bond: 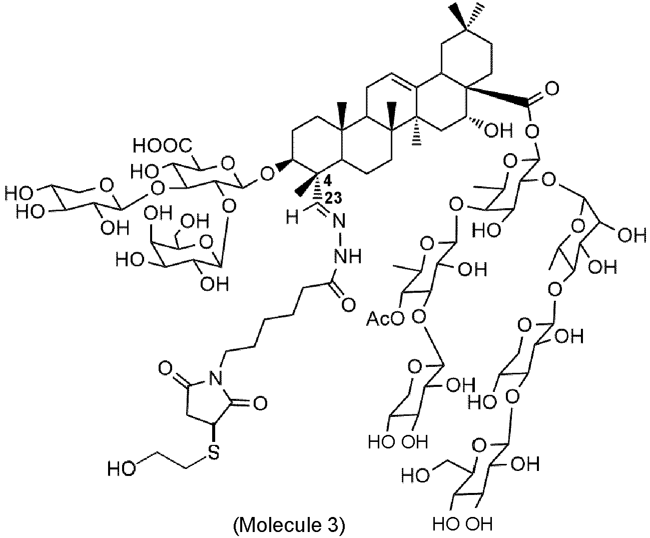
; or the saponin derivative is a derivative with the proviso that the saponin derivative is not a SO1861 derivative comprising a single derivatisation, wherein the single derivatisation is transformation of the carboxyl group of a glucuronic acid moiety of SO1861 by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, or wherein the saponin derivative is a SO1861 derivative represented by Molecule 2, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH): 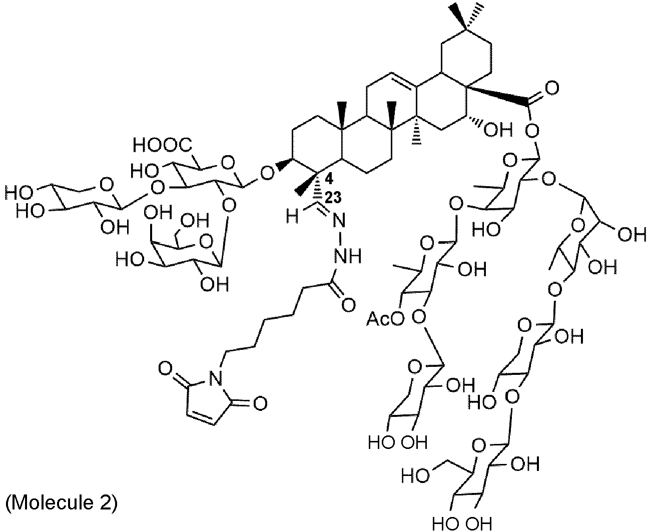
; or wherein the saponin derivative is a derivative wherein
   i. the saponin derivative comprises said aglycone core structure wherein the aglycone core structure comprises an aldehyde group which has been derivatised by:
      - reduction to an alcohol;
      - transformation into a hydrazone bond through reaction with N-ε-maleimidocaproic acid hydrazide (EMCH) wherein the maleimide group of the EMCH is optionally derivatised by formation of a thio-ether bond with mercaptoethanol;
      - transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
      - transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;
   ii. the first saccharide chain comprises said carboxyl group, preferably a carboxyl group of a glucuronic acid moiety, which has been derivatised by transformation into an amide bond through reaction with 2-amino-2-methyl-1,3-propanediol (AMPD) or N-(2-aminoethyl)maleimide (AEM);
   iii. the second saccharide chain comprises said acetoxy group (Me(CO)O-) which has been derivatised by transformation into a hydroxyl group (HO-) by deacetylation; or
   iv. the saponin derivative comprises any combination of two or three derivatisations i., ii. and iii., preferably any combination of two derivatisations i., ii. and iii.;
preferably, the saponin derivative comprises said aglycone core structure wherein the aglycone core structure comprises an aldehyde group which has been derivatised by transformation into a hydrazone bond through reaction with EMCH wherein the maleimide group of the EMCH is optionally derivatised by formation of a thio-ether bond with mercaptoethanol; or wherein the saponin derivative comprises an aglycone core structure wherein the aglycone core structure comprises an aldehyde group and wherein the first saccharide chain comprises said carboxyl group, preferably a carboxyl group of a glucuronic acid moiety, which has been derivatised by transformation into an amide bond through reaction with N-(2-aminoethyl)maleimide (AEM); or wherein the saponin derivative is a derivative with the proviso that when the aldehyde group in the aglycone core structure is derivatised by transformation into a hydrazone bond through reaction with N-ε-maleimidocaproic acid hydrazide (EMCH) and the saponin is SO1861, at least one of the glucuronic acid and the acetoxy group (Me(CO)O-) is also derivatised, and with the proviso that when the saponin is SO1861 and the carboxyl group of the glucuronic acid moiety of SO1861 is derivatised by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, at least one of the aldehyde group and the acetoxy group (Me(CO)O-) is also modified; or wherein the saponin is a derivative with the proviso that when the aldehyde group in the aglycone core structure of the saponin derivative is derivatised through reaction with EMCH and the saponin is SO1861, at least one of the glucuronic acid and the acetoxy group (Me(CO)O-) is also derivatised, and with the proviso that when the saponin is SO1861 and the carboxyl group of the glucuronic acid moiety of SO1861 is derivatised by bound HATU, at least one of the aldehyde group and the acetoxy group (Me(CO)O-) is also derivatised.

In particular embodiments the *in vitro* or ex *vivo* method for transferring a molecule from outside a cell to inside said cell, preferably into the cytosol of said cell as described herein is provided wherein the saponin derivative comprises, preferably consists of SO1861-Ald-EMCH, SO1861-Ald-EMCH-mercaptoethanol, SO1861-L-N₃ or SO1861-Glu-HATU.

While the invention has been described in terms of several embodiments, it is contemplated that alternatives, modifications, permutations and equivalents thereof will become apparent to one having ordinary skill in the art upon reading the specification and upon study of the drawings. The invention is not limited in any way to the illustrated embodiments. Changes can be made without departing from the scope which is defined by the appended claims.

The present invention has been described above with reference to a number of exemplary embodiments. Modifications are possible, and are included in the scope of protection as defined in the appended claims. The invention is further illustrated by the following examples, which shows SO1861 according to the invention and examples which are not claimed, and which should not be interpreted as limiting the present invention in any way.

| TABLE A1. Saponins displaying (late) endosomal/lysosomal escape enhancing activity, and saponins comprising a structure reminiscent to such saponins displaying (late) endosomal/lysosomal escape enhancing activity | | | |
|---|---|---|---|
| **Saponin Name** | **Aglycone core** | **Carbohydrate substituent at the C-3beta-OH group** | **Carbohydrate substituent at the C-28-OH group** |
| NP-005236 | 2alpha-Hydroxyoleanolic acid | GlcA- | Glc/Gal- |
| AMA-1 | 16alpha-Hydroxyoleanolic acid | Glc- | Rha-(1→2)-[Xyl-(1→4)]-Rha- |
| AMR | 16alpha-Hydroxyoleanolic acid | Glc- | Rha-(1→2)-[Ara-(1→3)-Xyl-(1→4)]-Rha- |
| alpha-Hederin | Hederagenin (23-Hydroxyoleanolic acid) | Rha-(1→2)-Ara- | - |
| NP-012672 | 16alpha,23-Dihydroxyoleanolic acid | Ara/Xyl-(1→4)-Rha/Fuc-(1→2)-Glc/Gal-(1→2)-Rha/Fuc-(1→2)-GlcA- | Ara/Xyl- |
| NP-017777 | Gypsogenin | Gal-(1→2)-[Xyl-(1→3)]-GlcA- | Xyl-(1→4)-Rha-(1→2)-[R-(→4)]-Fuc- (R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc-Fuc- |
| NP-018110^{c}, NP-017772^{d} | Gypsogenin | Gal-(1→2)-[Xyl-(1→3)]-GlcA- | Xyl-(1→4)-[Glc-(1→3)]-Rha-(1→2)-3,4-di-OAc-Fuc- |
| NP-01 8109 | Gypsogenin | Gal-(1→2)-[Xyl-(1→3)]-GlcA- | Xyl-(1→4)-[Glc-(1→3)]-Rha-(1→2)-[R-(→4)]-3-OAc-Fuc- (R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-01 8108 | Gypsogenin | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1→4)]-Rha/Fuc- |
| SA1641^{a}, AE X55^{b} | Gypsogenin | Gal-(1→2)-[Xyl-(1→3)]-GlcA- | Xyl-(1→3)-Xyl-(1→4)-Rha-(1→2)-[Qui-(1→4)]-Fuc- |
| NP-017674 | Quillaic acid | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc- |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017706 | Quillaic acid | Gal-(1→2)-[Xyl-(1→3)]-GlcA- | Api-(1→3)-Xyl-(1→4)-Rha-(1→2)-[Rha-(1→3)]-4-OAc-Fuc- |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc-Rha-(1→3)]-Fuc- |
| SA1657 | Quillaic acid | Gal-(1→2)-[Xyl-(1→3)]-GlcA- | Xyl-(1→3)-Xyl-(1→4)-Rha-(1→2)-[Qui-(1→4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1→4)]-Fuc- |
| SO1861 | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | Gal-(1→2)-[Xyl-(1→3)]-GlcA- | Glc-(1→3)-[Xyl-(1→4)]-Rha-(1→2)-Fuc- |
| SO1584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1832 | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| beta-Aescin (described: Aescin la) | Protoaescigenin-21 (2-methylbut-2-enoate)-22-acetat | Glc-(1→2)-[Glc-(1→4)]-GlcA- | - |
| Teaseed saponin I | 23-Oxo-barringtogenol C - 21,22-bis(2-methylbut-2-enoate) | Glc-(1→2)-Ara-(1→3)-[Gal-(1→2)]-GlcA- | - |
| Teaseedsaponin J | 23-Oxo-barringtogenol C - 21,22-bis(2-methylbut-2-enoate) | Xyl-(1→2)-Ara-(1→3)-[Gal-(1→2)]-GlcA- | - |
| Assamsaponin F | 23-Oxo-barringtogenol C - 21 (2-methylbut-2-enoate)-16,22-diacetat | Glc-(1→2)-Ara-(1→3)-[Gal-(1→2)]-GlcA- | - |
| Digitonin | Digitogenin | Glc-(1→3)-Gal-(1→2)-[Xyl-(1→3)]-Glc-(1→4)-Gal- | - |
| Primula acid 1 | 3,16,28-Trihydroxyoleanan-12-en | Rha-(1→2)-Gal-(1→3)-[Glc-(1→2)]-GlcA- | - |
| AS64R | Gypsogenic acid | - | Glc-(1→3)-[Glc-(1→6)]-Gal- |
| | | **Carbohydrate substituent at the C-23-OH group** | |
| AS6.2 | Gypsogenic acid | Gal- | Glc-(1→3)-[Glc-(1→6)]-Gal- |
| a, b: Different names refer to different isolates of the same structure | | | |
| c, d: Different names refer to different isolates of the same structure | | | |

### EXAMPLES AND EXEMPLARY EMBODIMENTS

### Materials:

SO1861, SO1832, SO1862 (isomer) and SO1904 were isolated and purified by Analyticon Discovery GmbH from raw plant extract obtained from Saponaria officinalis L. QS21(pure), QS18 (fraction), QS17 (fraction), QS7 ( fraction) QS21 (fraction) were purchased from Desert King International, San Diego. Trastuzumab (Tras, Herceptin^{®}, Roche), Cetuximab (Cet, Erbitux^{®}, Merck KGaA) were purchased from pharmacy. EGFdianthin was produced from *E.coli* according to standard procedures. Cetuximab-saporin conjugates were produced and purchased from Advanced Targeting Systems (San Diego, CA). Tris(2-carboxyethyl)phosphine hydrochloride (TCEP, 98%, Sigma-Aldrich), 5,5-Dithiobis(2-nitrobenzoic acid) (DTNB, Ellman's reagent, 99%, Sigma-Aldrich), Zeba^{™} Spin Desalting Columns (2 mL, Thermo-Fisher), NuPAGE^{™} 4-12% Bis-Tris Protein Gels (Thermo-Fisher), NuPAGE^{™} MES SDS Running Buffer (Thermo-Fisher), Novex^{™} Sharp Pre-stained Protein Standard (Thermo-Fisher), PageBlue^{™} Protein Staining Solution (Thermo-Fischer), Pierce^{™} BCA Protein Assay Kit (Thermo-Fisher), N-Ethylmaleimide (NEM, 98%, Sigma-Aldrich), 1,4-Dithiothreitol (DTT, 98%, Sigma-Aldrich), Sephadex G25 (GE Healthcare), Sephadex G50 M (GE Healthcare), Superdex 200P (GE Healthcare), Isopropyl alcohol (IPA, 99.6%, VWR), Tris(hydroxymethyl)aminomethane (Tris, 99%, Sigma-Aldrich), Tris(hydroxymethyl)aminomethane hydrochloride (Tris.HCL, Sigma-Aldrich), L-Histidine (99%, Sigma-Aldrich), D-(+)-Trehalose dehydrate (99%, Sigma-Aldrich), Polyethylene glycol sorbitan monolaurate (TWEEN 20, Sigma-Aldrich), Dulbecco's Phosphate-Buffered Saline (DPBS, Thermo-Fisher), Guanidine hydrochloride (99%, Sigma-Aldrich), Ethylenediaminetetraacetic acid disodium salt dihydrate (EDTA-Na2, 99%, Sigma-Aldrich), sterile filters 0.2 µm and 0.45 µm (Sartorius), Succinimidyl 4-(N-maleimidomethyl)cyclohexane-1-carboxylate (SMCC, Thermo-Fisher), Vivaspin T4 and T15 concentrator (Sartorius), Superdex 200PG (GE Healthcare), Tetra(ethylene glycol) succinimidyl 3-(2-pyridyldithio)propionate (PEG4-SPDP, Thermo-Fisher), [O-(7-Azabenzotriazol-1-yl)-N,N,N,N-tetramethyluronium-hexafluorphosphat] (HATU, 97%, Sigma-Aldrich), Dimethyl sulfoxide (DMSO, 99%, Sigma-Aldrich), N-(2-Aminoethyl)maleimide trifluoroacetate salt (AEM, 98%, Sigma-Aldrich), L-Cysteine (98.5 %, Sigma-Aldrich), deionized water (DI) was freshly taken from Ultrapure Lab Water Systems (MilliQ, Merck), Nickel-nitrilotriacetic acid agarose (Ni-NTA agarose, Protino), Glycine (99.5%, VWR), 5,5-Dithiobis(2-nitrobenzoic acid (Ellman's reagent, DTNB, 98%, Sigma-Aldrich), S-Acetylmercaptosuccinic anhydride Fluorescein (SAMSA reagent, Invitrogen) Sodium bicarbonate (99.7%, Sigma-Aldrich), Sodium carbonate (99.9%, Sigma-Aldrich), PD MiniTrap desalting columns with Sephadex G-25 resin (GE Healthcare), PD10 G25 desalting column (GE Healthcare), Zeba Spin Desalting Columns in 0.5, 2, 5, and 10 mL (Thermo-Fisher), Vivaspin Centrifugal Filters T4 10 kDa MWCO, T4 100 kDa MWCO, and T15 (Sartorius), Biosep s3000 aSEC column (Phenomenex), Vivacell Ultrafiltration Units 10 and 30 kDa MWCO (Sartorius), Nalgene Rapid-Flow filter (Thermo-Fisher).

### Abbreviations

AEM: *N*-(2-Aminoethyl)maleimide trifluoroacetate salt
AMPD: 2-Amino-2-methyl-1,3-propanediol
BOP: (Benzotriazol-1-yloxy)tris(dimethylamino)phosphonium hexafluorophosphate
DIPEA: N,N-diisopropylethylamine
DMF: N,N-dimethylformamide
EMCH.TFA: N-(ε-maleimidocaproic acid) hydrazide, trifluoroacetic acid salt
HATU: 1-[Bis(dimethylamino)methylene]-1H-1,2,3-triazolo[4,5-b]pyridinium 3-oxid hexafluorophosphate
Min: minutes
NMM: 4-Methylmorpholine
r.t.: retention time
TCEP: tris(2-carboxyethyl)phosphine hydrochloride
Temp: temperature
TFA: trifluoroacetic acid

### Analytical methods

### LC-MS method 1

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product neg or neg/pos within in a range of 1500-2400 or 2000-3000; ELSD: gas pressure 40 psi, drift tube temp: 50°C; column: Acquity C18, 50×2.1 mm, 1.7 µm Temp: 60°C, Flow: 0.6 mL/min,

Gradient depending on the polarity of the product:
At0 = 2% A, t5.0min = 50% A, t6.0min = 98% A
Bt0 = 2% A, t5.0min = 98% A, t6.0min = 98% A
Post time: 1.0 min, Eluent A: acetonitrile, Eluent B: 10 mM ammonium bicarbonate in water (pH=9.5). LC-MS method 2, ²
Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50 °C; column: Waters XSelectTM CSH C18, 50×2.1mm, 2.5 µm, Temp: 25°C, Flow: 0.5 mL/min, Gradient: t0min = 5% A, t2.0min = 98% A, t2.7min = 98% A, Posttime: 0.3 min, Eluent A: acetonitrile, Eluent B: 10 mM ammonium bicarbonate in water (pH = 9.5).

### LC-MS method 3

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product pos/neg 105-800, 500-1200 or 1500-2500; ELSD: gas pressure 40 psi, drift tube temp: 50°C; column: Waters XSelectTM CSH C18, 50×2.1 mm, 2.5 µm, Temp: 40°C, Flow: 0.5 mL/min, Gradient: t0min = 5% A, t2.0min = 98% A, t2.7min = 98% A, Posttime: 0.3 min, Eluent A: 0.1% formic acid in acetonitrile, Eluent B: 0.1% formic acid in water.

### LC-MS method 4

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50°C column: Waters Acquity Shield RP18, 50×2.1 mm, 1.7 µm, Temp: 25°C, Flow: 0.5 mL/min, Gradient: t0min = 5% A, t2.0min = 98% A, t2.7min = 98% A, Posttime: 0.3 min, Eluent A: acetonitrile, Eluent B: 10 mM ammonium bicarbonate in water (pH = 9.5).

### Preparative methods

### Preparative MP-LC method 1,

Instrument type: Reveleris^{™} prep MPLC; column: Waters XSelectTM CSH C18 (145×25 mm, 10 µm); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient:
^{A}t0min = 5% B, t1min = 5% B, t2min = 10% B, t17min = 50% B, t18min = 100% B, t23min = 100% B
^{B}t0min = 5% B, t1min = 5% B, t2min = 20% B, t17min = 60% B, t18min = 100% B, t23min = 100% B
; Detection UV: 210, 235, 254 nm and ELSD.

### Preparative MP-LC method 2

Instrument type: Reveleris^{™} prep MPLC; Column: Phenomenex LUNA C18(3) (150×25 mm, 10 µm); Flow: 40 mL/min; Column temp: room temperature; Eluent A: 0.1% (v/v) Formic acid in water, Eluent B: 0.1% (v/v) Formic acid in acetonitrile; Gradient:
^{A}t0min = 5% B, t1min = 5% B, t2min = 20% B, t17min = 60% B, t18min = 100% B, t23min = 100% B
^{B}t0min = 2% B, t1min = 2% B, t2min = 2% B, t17min = 30% B, t18min = 100% B, t23min = 100% B
^{C}t0min = 5% B, t1min = 5% B, t2min = 10% B, t17min = 50% B, t18min = 100% B, t23min = 100% B
^{B}t0min = 5% B, t1min = 5% B, t2min = 5% B, t17min = 40% B, t18min = 100% B, t23min = 100% B
; Detection UV : 210, 235, 254 nm and ELSD.

### Preparative LC-MS method 3

MS instrument type: Agilent Technologies G6130B Quadrupole; HPLC instrument type: Agilent Technologies 1290 preparative LC; Column: Waters XSelectTM CSH (C18, 150×19 mm, 10 µm); Flow: 25 ml/min; Column temp: room temperature; Eluent A: 100% acetonitrile; Eluent B: 10 mM ammonium bicarbonate in water pH=9.0; Gradient:
^{A}t0 = 20% A, t2.5min = 20% A, t11min = 60% A, t13min = 100% A, t17min = 100% A
^{B}t0 = 5% A, t2.5min = 5% A, t11min = 40% A, t13min = 100% A, t17min = 100% A
; Detection: DAD (210 nm); Detection: MSD (ESI pos/neg) mass range: 100 - 800; Fraction collection based on DAD.

### Preparative LC-MS method 4

MS instrument type: Agilent Technologies G6130B Quadrupole; HPLC instrument type: Agilent Technologies 1290 preparative LC; Column: Waters XBridge Protein (C4, 150×19 mm, 10 µm); Flow: 25 ml/min; Column temp: room temperature; Eluent A: 100% acetonitrile; Eluent B: 10 mM ammonium bicarbonate in water pH=9.0; Gradient:
^{A}t0 = 2% A, t2.5min = 2% A, t11min = 30% A, t13min = 100% A, t17min = 100% A
^{B}t0 = 10% A, t2.5min = 10% A, t11min = 50% A, t13min = 100% A, t17min = 100% A
^{C}t0 = 5% A, t2.5min = 5% A, t11min = 40% A, t13min = 100% A, t17min = 100% A
; Detection: DAD (210 nm); Detection: MSD (ESI pos/neg) mass range: 100 - 800; Fraction collection based on DAD

### Flash chromatography

Grace Reveleris X2^{®} C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50 bar (725 psi); Detection: UV 200-400 nm, combination of up to 4 UV signals and scan of entire UV range, ELSD; Column sizes: 4-330 g on instrument, luer type, 750 g up to 3000 g with optional holder.

### Example 1: synthesis of saponin derivatives

The following modified SO1861 saponins, i.e. saponin derivatives, were synthesized based on the naturally occurring SO1861, as summarized in Table A2:

**Table A2: overview of the synthesized modified SO1861 (SO1861 derivatives):**

| **Saponin derivative** | **Molecule No.** | **Derivatised group** | **Number of derivatised groups** | **Type of derivatisation** |
|---|---|---|---|---|
| SO1861-Ald-EMCH | **2** | aldehyde | 1 | Transformation of the aglycone core aldehyde group into a hydrazone bond through reaction with N-ε-maleimidocaproic acid hydrazide (EMCH) (Molecule 2). See Figure 60. |
| SO1861-Ald-EMCH-mercaptoethanol or SO1861-Ald-EMCH-blocked | **3** | aldehyde | 1 | Transformation of the aglycone core aldehyde group into a hydrazone bond through reaction with EMCH which has been derivatised by formation of a thio-ether bond with mercaptoethanol (Molecule 3). |
| SO1861-Glu-AMPD | **3A** | Glucuronic acid | 1 | Transformation of the carboxyl group of the glucuronic acid unit in the first saccharide chain bound to C₃ of the aglycone core structure of the saponin into an amide bond through reaction with 2-amino-2-methyl-1,3-propanediol (AMPD). See Figure 1. |
| SO1861-Ald-OH | **6** | Aldehyde | 1 | Reduction of the aglycone core aldehyde group into an alcohol (treatment with NaBH₄ (**4**)). See Figure 2. |
| SO1861-Ac-OH | **8** | Acetoxy | 1 | Transformation of the acetoxy (Me(CO)O-) group in the second saccharide chain bound to C₂₈ of the aglycone core structure of the saponin into a hydroxyl group (HO-) by deacetylation. See Figure 3. |
| SO1861-(Ald-OH)-(Glu-AMPD) | **9** | Aldehyde Glucuronic acid | 2 | Reduction of the aglycone core aldehyde group into an alcohol and transformation of the carboxyl group of the glucuronic acid unit in the first saccharide chain bound to C₃ of the aglycone core structure of the saponin into an amide bond through reaction with AMPD. See Figure 4. |
| SO1861-(Ald-OH)-(Ac-OH) | **10** | Aldehyde Acetoxy | 2 | Reduction of the aglycone core aldehyde group into an alcohol and transformation of the acetoxy (Me(CO)O-) group in the second saccharide chain bound to C₂₈ of the aglycone core structure of the saponin into a hydroxyl group (HO-) by deacetylation. See Figure 5. |
| SO1861-(Ac-OH)-(Glu-AMPD) | **11** | Acetoxy Glucuronic acid | 2 | Transformation of the acetoxy (Me(CO)O-) group in the second saccharide chain bound to C₂₈ of the aglycone core structure of the saponin into a hydroxyl group (HO-) by deacetylation and transformation of the carboxyl group of the glucuronic acid unit in the first saccharide chain bound to C₃ of the aglycone core structure of the saponin into an amide bond through reaction with AMPD. See Figure 6. |
| SO1861-(Ald-OH)-(Ac-OH)-(Glu-AMPD) | **12** | Aldehyde Acetoxy Glucuronic acid | 3 | Reduction of the aglycone core aldehyde group into an alcohol, transformation of the acetoxy (Me(CO)O-) group in the second saccharide chain bound to C₂₈ of the aglycone core structure of the saponin into a hydroxyl group (HO-) by deacetylation and transformation of the carboxyl group of the glucuronic acid unit in the first saccharide chain bound to C₃ of the aglycone core structure of the saponin into an amide bond through reaction with AMPD. See Figure 7. |
| SO1861-(Ald-EMCH)-(Glu-AMPD) | **14** | Aldehyde Glucuronic acid | 2 | Transformation of the aglycone core aldehyde group into a hydrazone bond through reaction with EMCH and transformation of the carboxyl group of the glucuronic acid unit in the first saccharide chain bound to C₃ of the aglycone core structure of the saponin into an amide bond through reaction with AMPD. See Figure 8. |
| SO1861-(Ald-EMCH)-(Ac-OH) | **15** | Acetoxy Aldehyde | 2 | Transformation of the aglycone core aldehyde group into a hydrazone bond through reaction with EMCH and transformation of the acetoxy (Me(CO)O-) group in the second saccharide chain bound to C₂₈ of the aglycone core structure of the saponin into a hydroxyl group (HO-) by deacetylation. See Figure 9. |
| SO1861-(Ald-EMCH)-(Ac-OH)-(Glu-AMPD) | **16** | Acetoxy Aldehyde Glucuronic acid | 3 | Transformation of the aglycone core aldehyde group into a hydrazone bond through reaction with EMCH, transformation of the acetoxy (Me(CO)O-) group in the second saccharide chain bound to C₂₈ of the aglycone core structure of the saponin into a hydroxyl group (HO-) by deacetylation and transformation of the carboxyl group of the glucuronic acid unit in the first saccharide chain bound to C₃ of the aglycone core structure of the saponin into an amide bond through reaction with AMPD. See Figure 10. |
| SO1861-Glu-AEM or SPT-S-Mal | **18** | Glucuronic acid | 1 | Transformation of the carboxyl group of the glucuronic acid unit in the first saccharide chain bound to C₃ of the aglycone core structure of the saponin into an amide bond through reaction with *N*-(2-aminoethyl)maleimide (AEM). See Figure 11. |
| SO1861-(Glu-AEM)-(Ac-OH) | **19** | Acetoxy Glucuronic acid | 2 | Transformation of the acetoxy (Me(CO)O-) group in the second saccharide chain bound to C₂₈ of the aglycone core structure of the saponin into a hydroxyl group (HO-) by deacetylation and transformation of the carboxyl group of the glucuronic acid unit in the first saccharide chain bound to C₃ of the aglycone core structure of the saponin into an amide bond through reaction with AEM. See Figure 12. |
| SO1861-(Glu-AEM)-(Ald-OH) | **20** | Aldehyde Glucuronic acid | 2 | Reduction of the aglycone core aldehyde group into an alcohol and transformation of the carboxyl group of the glucuronic acid unit in the first saccharide chain bound to C₃ of the aglycone core structure of the saponin into an amide bond through reaction with AEM. See Figure 13. |
| SO1861-(Glu-AEM)-(Ald-OH)-(Ac-OH) | **21** | Aldehyde Acetoxy Glucuronic acid | 3 | See Figure 14. |
| SO1861-L-N₃ | **23** | Aldehyde | 1 | Transformation of the aglycone core aldehyde group into a hydrazone bond through reaction with molecule **22** (see Figure 36) |
| SO1861-L-NHS | **25** | Aldehyde | 1 | Transformation of the aglycone core aldehyde group into a hydrazone bond through reaction with first the molecule **22** (see Figure 36), providing molecule **23,** which has been derivatised by reaction with DBCO-NHS (molecule **24,** see Figure 37) |
| SO1861-Glu-HATU | **26** | Glucuronic acid | 1 | Transformation of the carboxyl group of the glucuronic acid unit in the first saccharide chain bound to C₃ of the aglycone core structure of the saponin through reaction with 1-[Bis(dimethylamino)methylene]-1H-1,2,3-triazolo[4,5-b]pyridinium 3-oxid hexafluorophosphate (See Figure 59) |

Referring to the following description of the syntheses of SO1861 derivatives, reference is made to Table A2 and to the drawings.

### Synthesis of SO1861-Ald-EMCH (molecule 2); see Figure 60, Figure 61A

SO1861 from Saponaria officinalis L (59 mg, 31.7 µmol) and EMCH (301 mg, 888 µmol) were placed in a round flask with stirrer and dissolved in 13 mL methanol. TFA (400 µL, cat.) was added to the solution and the reaction mixture was stirred for 3 h at 800 rpm and room temperature on a RCT B magnetic stirrer (IKA Labortechnik). After stirring for 3 h, the mix was diluted either with MilliQ water or PBS and dialyzed extensively for 24 h against either with MilliQ water or PBS using regenerated cellulose membrane tubes (Spectra/Por 7) with a MWCO of 1 kDa. After dialysis, the solution was lyophilized to obtain a white powder. Yield 62.4 mg (95%). Dried aliquots were further used for characterization via ¹H NMR and MALDI-TOF-MS.

¹H NMR (400 MHz, methanol-*D*₄) (SO1861): *δ* = 0.50-5.50 (m, saponin triterpenoid and sugar backbone protons), 9.43 (1H, s, aldehyde proton of saponin, H^{a}).

¹H NMR (400 MHz, methanol-*D*₄) (SO1861-Ald-EMCH, PBS workup): *δ* = 0.50-5.50 (m, saponin triterpenoid and sugar backbone protons), 6.79 (2 H, s, maleimide protons, H^{c}), 7.62-7.68 (1 H, m, hydrazone proton, H^{b}).

MALDI-TOF-MS (RP mode): *m*/*z* 2124 Da ([M+K]⁺, saponin-EMCH), *m*/*z* 2109 Da ([M+K]⁺, SO1861-ALD-EMCH), *m*/*z* 2094 Da ([M+Na]⁺, SO1861-ALD-EMCH). See Figure 61A.

MALDI-TOF-MS (RN mode): m/z 2275 Da ([M-H]⁻, saponin-EMCH conjugate), 2244 Da ([M-H]⁻ , saponin-EMCH conjugate), 2222 Da ([M-H]⁻, saponin-EMCH conjugate), 2178 Da ([M-H]⁻, saponin-EMCH conjugate), 2144 Da ([M-H]⁻, saponin-EMCH conjugate), 2122 Da ([M-H]⁻, saponin-EMCH conjugate), 2092 Da ([M-H]⁻, saponin-EMCH conjugate), 2070 Da ([M-H]⁻, SO1861-ALD-EMCH), 2038 Da ([M-H]⁻, SO1832-EMCH), 1936 Da ([M-H]⁻, SO1730-EMCH), 1861 Da ([M-H]⁻, SO1861). The SO1861-ALD-EMCH is represented by Molecule 2 (Chemical Formula: C₉₃H₁₄₃N₃O₄₈, Exact Mass: 2069.88):

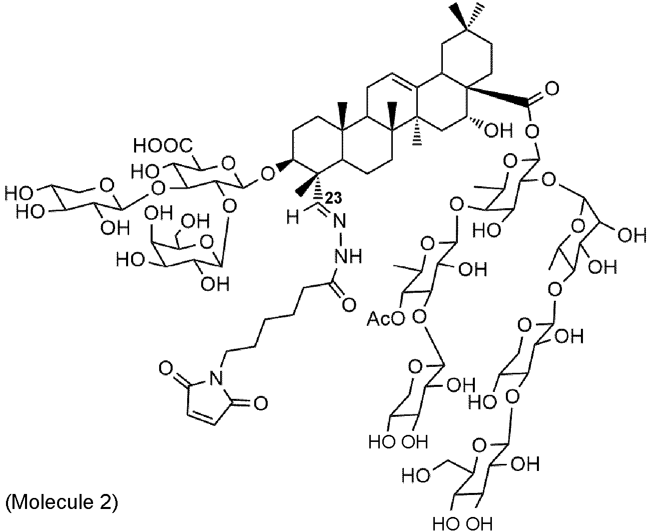

For testing the pH dependent hydrolysis of the hydrazone bond, SO1861-Ald-EMCH was dissolved in an HCl solution at pH 3 and MALDI-TOF-MS spectra were recorded at two different points in time (Figure 62). As shown on Figure 62 A and 62 B, a clear decreasing tendency of the peak at *m*/*z* 2070 Da that corresponds to SO1861-Ald-EMCH is visible in Figure 61 B. Since SO1861 is generated during hydrolysis, an increase of the peak at *m*/*z* 1861 Da was recorded that accompanied the decreasing tendency at *m*/*z* 2070 Da. These results show that the hydrazone bond is responsive towards hydrolysis and gets cleaved even if it is attached on SO1861.

### Synthesis of SO1861-Ald-EMCH-mercaptoethanol (molecule 3; SO1861-Ald-EMCH-blocked); see Figure 60 and Figure 61B

The maleimide group of SO1861-Ald-EMCH performs a rapid and specific Michael addition reaction with thiols when carried out in a pH range of 6.5-7.5.

To SO1861-Ald-EMCH (0.1 mg, 48 nmol) 200 µL mercaptoethanol (18 mg, 230 µmol) was added and the solution was shaken for 1 h at 800 rpm and room temperature on a ThermoMixer C (Eppendorf). After shaking for 1 h, the solution was diluted with methanol and dialyzed extensively for 4 h against methanol using regenerated cellulose membrane tubes (Spectra/Por 7) with a MWCO of 1 kDa. After dialysis the SO1861-Ald-EMCH-mercaptoethanol was provided (Molecule 3), an aliquot was taken out and analyzed via MALDI-TOF-MS.

MALDI-TOF-MS (RP mode): *m*/*z* 2193 Da ([M+K]⁺, SO1861-Ald-EMCH-mercaptoethanol), *m*/*z* 2185 Da ([M+K]⁺, SO1861-Ald-EMCH-mercaptoethanol), *m*/*z* 2170 Da ([M+Na]⁺, SO1861-Ald-EMCH-mercaptoethanol). See Figure 61B. The SO1861-Ald-EMCH-mercaptoethanol is represented by Molecule 3 (Chemical Formula: C₉₅H₁₄₉N₃O₄₉S, Exact Mass: 2147.90):

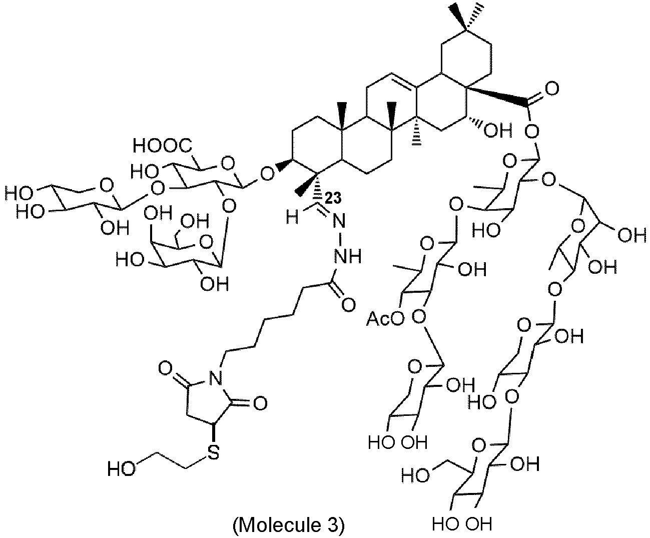

### Synthesis of SO1861-Glu-AMPD (molecule 3A); see Figure 1

SO1861 (28.8 mg, 0.015 mmol), AMPD (8.11 mg, 0.077 mmol) and HATU (17.6 mg, 0.046 mmol) were dissolved in a mixture of DMF (1.00 mL) and NMM (8.48 µL, 0.077 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight. Next, the product was repurified by using preparative LC-MS.³ Fractions corresponding to the product were immediately pooled together, frozen and lyophilized overnight to give the title compound (20.2 mg, 67%) as a white fluffy solid. Purity based on LC-MS = 93% (Chemical Formula: C₈₇H₁₃₉NO₄₇, Exact Mass: 1949,85.
LRMS (m/z): 1949 [M-1]¹⁻
LC-MS r.t. (min): 2.45^{1B}

### Synthesis of SO1861-Ald-OH (molecule 6); see Figure 2

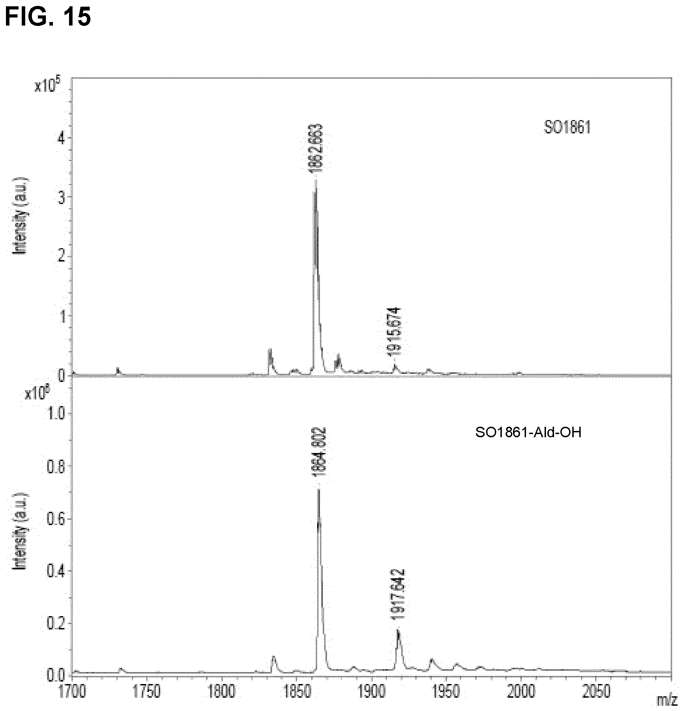

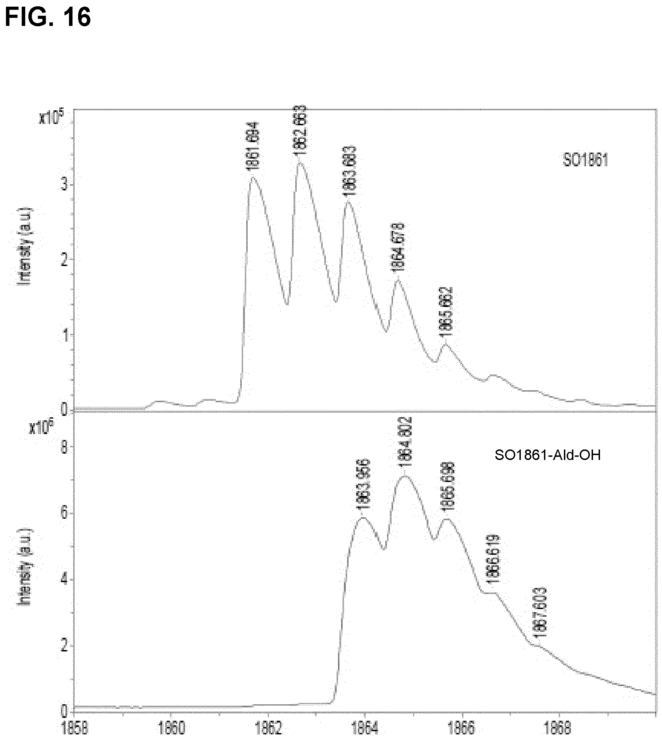

SO1861 (20.0 mg, 10.7 µmol) was dissolved in methanol (1.00 mL). Next, sodium borohydride (4.06 mg, 0.107 mmol; NaBH₄) was added. The reaction mixture was shaken for 1 min and left standing at room temperature. After 30 min the reaction mixture was diluted with water (0.50 mL) and submitted to preparative MP-LC.² Fractions corresponding to the product were immediately pooled together, frozen and lyophilized overnight to give the title compound (15.9 mg, 79%) as a white fluffy solid. Purity based on LC-MS 97% (Chemical Formula: C₈₃H₁₃₂O₄₆, Exact Mass: 1864,80).
LRMS (m/z): 1865 [M-1]¹⁻ (see Figure 15 and 16)
LC-MS r.t. (min): 1.95^{1B}

### Synthesis of SO1861-Ac-OH (molecule 8); see Figure 3

To SO1861 (9.30 mg, 4.99 µmol) was added a solution of sodium hydroxide (2.00 mg, 0.050 mmol) in water (0.25 mL) and methanol (0.25 mL). The reaction mixture was shaken for 1 min and left standing at room temperature. After 2 hours the reaction mixture was subjected to preparative MP-LC.² Fractions corresponding to the product were immediately pooled together, frozen and lyophilized overnight to give the title compound (8.86 mg, 97%) as a white fluffy solid. Purity based on LC-MS = 97% (Chemical Formula: C₈₁H₁₂₈O₄₅, Exact Mass: 1820,77).
LRMS (m/z): 1820 [M-1]¹⁻
LC-MS r.t. (min): 1.83^{1B}

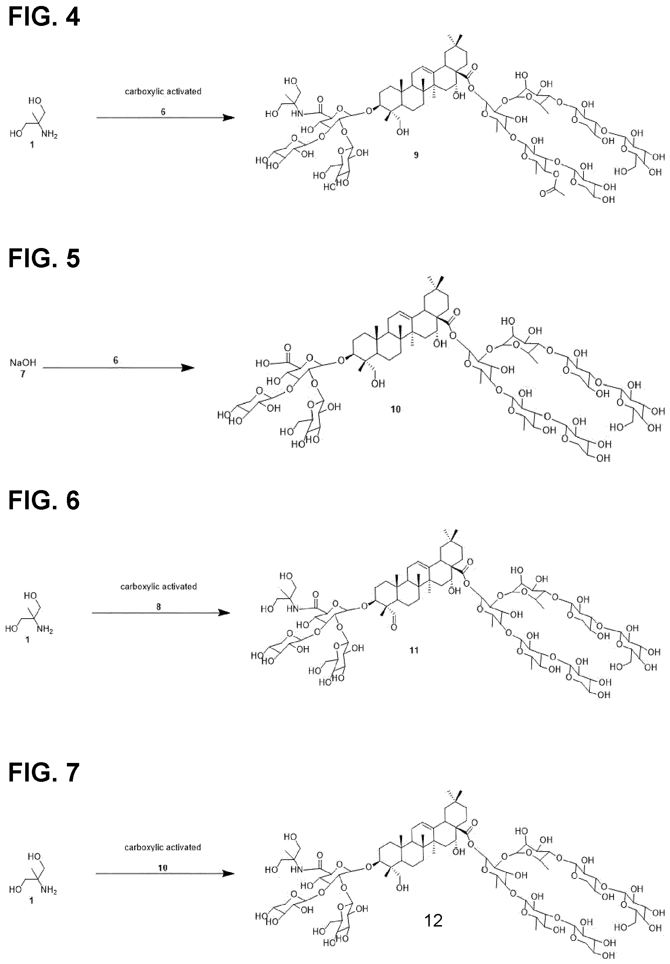

### Synthesis of SO1861-(Ald-OH)-(Glu-AMPD) (molecule 9); see Figure 4

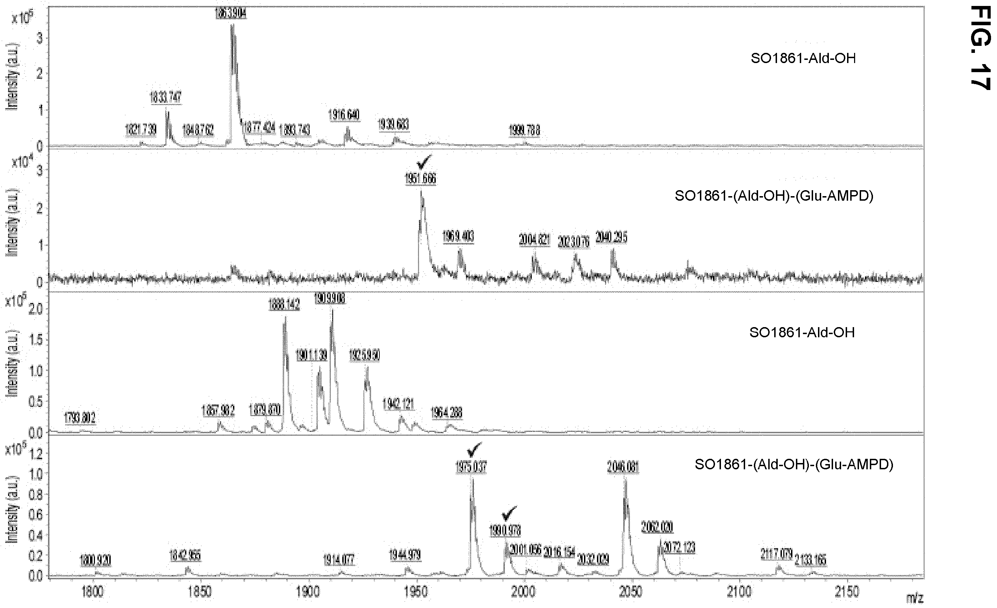

SO1861-Ald-OH (9.37 mg, 5.02 µmol), AMPD (2.64 mg, 0.025 mmol) and BOP (6.66 mg, 0.015 mmol) were dissolved in a mixture of DMF (0.50 mL) and NMM (5.52 µL, 0.050 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight to give the title compound (6.32 mg, 64%) as a white fluffy solid. Purity based on LC-MS = 95% (Chemical Formula: C₈₇H₁₄₁NO₄₇, Exact Mass: 1951,87.
LRMS (m/z): 1952 [M-1]¹⁻ (see Figure 17)
LC-MS r.t. (min): 2.45^{1B}

### Synthesis of SO1861-(Ald-OH)-(Ac-OH) (molecule 10); see Figure 5

To SO1861-Ald-OH (26.8 mg, 0.014 mmol) was added a solution of sodium hydroxide (5.74 mg, 0.144 mmol) in water (0.50 mL) and methanol (0.50 mL). The reaction mixture was shaken for 1 min and left standing at room temperature. After 2 hours the reaction mixture was subjected to preparative MP-LC.² Fractions corresponding to the product were immediately pooled together, frozen and lyophilized overnight to give the title compound (24.2 mg, 92%) as a white fluffy solid. Purity based on LC-MS = 98%. (Chemical Formula: C₈₁H₁₃₀O₄₅, Exact Mass: 1822,79)
LRMS (m/z): 1822 [M-1]¹⁻
LC-MS r.t. (min): 1.81^{1B}

### Synthesis of SO1861-(Ac-OH)-(Glu-AMPD) (molecule 11); see Figure 6

SO1861-Ac-OH (14.3 mg, 7.84 µmol), AMPD (4.12 mg, 0.039 mmol) and BOP (10.4 mg, 0.024 mmol) were dissolved in a mixture of DMF (0.50 mL) and NMM (8.62 µL, 0.078 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight. Next, the product was repurified by using first preparative MP-LC², followed by preparative LC-MS.³ Fractions corresponding to the product were immediately pooled together, frozen and lyophilized overnight to give the title compound (9.47 mg, 63%) as a white fluffy solid. Purity based on LC-MS = 98% (Chemical Formula: C₈₅H₁₃₇NO₄₆, Exact Mass: 1907,84).
LRMS (m/z): 1908 [M-1]¹⁻
LC-MS r.t. (min): 2.31^{1B}

### Synthesis of SO1861-(Ald-OH)-(Ac-OH)-(Glu-AMPD) (molecule 12); see Figure 7

SO1861-(Ald-OH)-(Ac-OH) (8.57 mg, 4.70 µmol), AMPD (42.58 mg, 0.025 mmol) and BOP (6.57 mg, 0.015 mmol) were dissolved in a mixture of DMF (0.50 mL) and NMM (5.17 µL, 0.047 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight. Next, the product was repurified by using again preparative MP-LC². Fractions corresponding to the product were immediately pooled together, frozen and lyophilized overnight to give the title compound (7.21 mg, 80%) as a white fluffy solid. Purity based on LC-MS = 97.8% Chemical Formula: C₈₅H₁₃₉NO₄₆, Exact Mass: 1909,86)
LRMS (m/z): 1910 [M-1]¹⁻
LC-MS r.t. (min): 2.21^{1B}

### Synthesis of SO1861-(Ald-EMCH)-(Glu-AMPD) (molecule 14); see Figure 8

SO1861-Glu-AMPD (10.6 mg, 5.43 µmol) and EMCH.TFA (9.22 mg, 0.027 mmol) were dissolved in methanol (extra dry, 0.50 mL). Next, TFA (1.66 µL, 0.022 mmol) was added. The reaction mixture was shaken for 1 min and left standing at room temperature. After 2 hours the reaction mixture was subjected to preparative MP-LC.¹ Fractions corresponding to the product were immediately pooled together, frozen and lyophilized overnight. Next, the product was repurified by using by preparative LC-MS.³ Fractions corresponding to the product were pooled together. The resulting solution was neutralized using formic acid, frozen and lyophilized overnight to give the title compound (2.61 mg, 22%) as a white fluffy solid. Purity based on LC-MS = 95% (Chemical Formula: C₉₇H₁₅₂N₄O₄₉, Exact Mass: 2156,95).
LRMS (m/z): 2156 [M-1]¹⁻
LC-MS r.t. (min): 2.64^{1B}

### Synthesis of SO1861-(Ald-EMCH)-(Ac-OH) (molecule 15); see Figure 9

SO1861-Ac-OH (9.05 mg, 4.97 µmol) and EMCH.TFA (8.43 mg, 0.025 mmol) were dissolved in methanol (extra dry, 0.50 mL). Next, TFA (1.52 µL, 0.022 mmol) was added. The reaction mixture was shaken for 1 min and left standing at room temperature. After 2 hours the reaction mixture was subjected to to preparative MP-LC.¹ Fractions corresponding to the product were immediately pooled together, frozen and lyophilized overnight to give the title compound (6.58 mg, 65%) as a white fluffy solid. Purity based on LC-MS = 97% (Chemical Formula: C₉₁H₁₄₁N₃O₄₇, Exact Mass: 2027,87).
LRMS (m/z): 2028 [M-1]¹⁻
LC-MS r.t. (min): 1.96^{1B}

### Synthesis of SO1861-(Ald-EMCH)-(Ac-OH)-(Glu-AMPD) (molecule 16); see Figure 10

SO1861-(Ac-OH)-(Glu-AMPD) (6.00 mg, 3.14 µmol) and EMCH.TFA (5.33 mg, 0.016 mmol) were dissolved in methanol (extra dry, 0.50 mL). Next, TFA (0.96 µL, 0.013 mmol) was added. The reaction mixture was shaken for 1 min and left standing at room temperature. After 2 hours the reaction mixture was subjected to to preparative MP-LC.¹ Fractions corresponding to the product were immediately pooled together, frozen and lyophilized overnight. Next, the product was repurified by using by preparative LC-MS.³ Fractions corresponding to the product were pooled together. The resulting solution was neutralized using formic acid, frozen and lyophilized overnight to give the title compound (1.04 mg, 16%) as a white fluffy solid. Purity based on LC-MS = 94% (Chemical Formula: C₉₅H₁₅₀N₄O₄₈, Exact Mass: 2114,94).
LRMS (m/z): 2115 [M-1]¹⁻
LC-MS r.t. (min): 2.55^{1B}

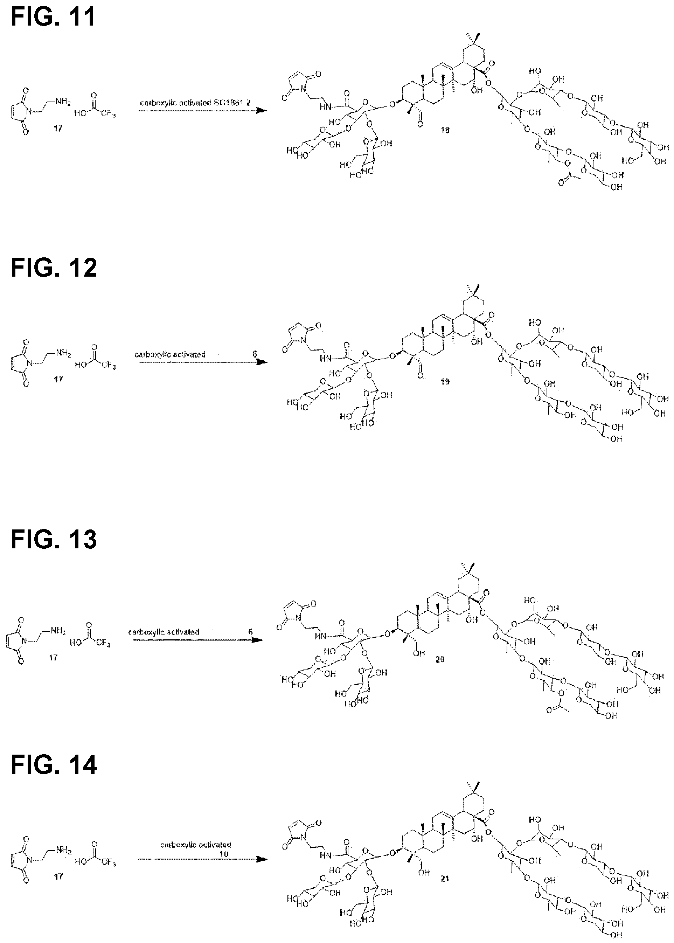

### Synthesis of SO1861-Glu-AEM (molecule 18); see Figure 11

SO1861 (10.4 mg, 5.58 µmol), AEM (7.10 mg, 0.028 mmol) and HATU (6.36 mg, 0.017 mmol) were dissolved in a mixture of DMF (1.00 mL) and NMM (6.13 µL, 0.056 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight to give the title compound (7.82 mg, 71%) as a white fluffy solid. Purity based on LC-MS = 95% (Chemical Formula: C₈₉H₁₃₆N₂O₄₇, Exact Mass: 1984,83).
LRMS (m/z): 1985 [M-1]¹⁻
LC-MS r.t. (min): 2.62^{1B}

### Synthesis of SO1861-(Glu-AEM)-(Ac-OH) (molecule 19); see Figure 12

SO1861-Ac-OH (9.02 mg, 4.95 µmol), AEM (7.10 mg, 0.028 mmol) and HATU (5.65 mg, 0.015 mmol) were dissolved in a mixture of DMF (0.50 mL) and NMM (5.44 µL, 0.050 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight to give the title compound (7.16 mg, 74%) as a white fluffy solid. Purity based on LC-MS = 96% (Chemical Formula: C₈₇H₁₃₄N₂O₄₆, Exact Mass: 1942,82).
LRMS (m/z): 1944 [M-1]¹⁻
LC-MS r.t. (min): 2.47^{1B}

### Synthesis of SO1861-(Glu-AEM)-(Ald-OH) (molecule 20); see Figure 13

SO1861-Ald-OH (9.38 mg, 5.03 µmol), AEM (6.39 mg, 0.025 mmol) and HATU (5.73 mg, 0.015 mmol) were dissolved in a mixture of DMF (0.50 mL) and NMM (5.53 µL, 0.050 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight to give the title compound (8.63 mg, 86%) as a white fluffy solid. Purity based on LC-MS = 95% (Chemical Formula: C₈₉H₁₃₈N₂O47, Exact Mass: 1986,85).
LRMS (m/z): 1987 [M-1]¹⁻
LC-MS r.t. (min): 2.62^{1B}

### Synthesis of SO1861-(Glu-AEM)-(Ald-OH)-(Ac-OH) (molecule 21); see Figure 14

SO1861-(Ald-OH)-(Ac-OH) (8.92 mg, 4.89 µmol), AEM (6.54 mg, 0.026 mmol) and HATU (5.65 mg, 0.015 mmol) were dissolved in a mixture of DMF (0.50 mL) and NMM (5.38 µL, 0.049 mmol). The reaction mixture was shaken for 1 min and left standing at room temperature. After 1 hour the reaction mixture was subjected to preparative MP-LC.² Fractions corresponding to the product were immediately pooled together, frozen and lyophilized overnight to give the title compound (8.92 mg, 94%) as a white fluffy solid. Purity based on LC-MS = 97% (Chemical Formula: C₈₇H₁₃₆N₂O₄₆, Exact Mass: 1944,84).
LRMS (m/z): 1944 [M-1]¹⁻
LC-MS r.t. (min): 2.46^{1B}

### Synthesis of SO1861-L-N₃ (molecule 23); see Figure 36

Chemical Formula: C₉₄H₁₅₁N₅O₅₀, Exact Mass: 2149,94

### Synthesis of SO1861-L-NHS (molecule 25); see Figure 37

SO1861-L-N₃ (7.71 mg, 3.58 µmol) and DBCO-NHS (2.88 mg, 7.17 µmol) were dissolved in dry DMF (0.50 mL). The reaction mixture was shaken for 1 min and left standing at room temperature. After 30 min the reaction mixture was added dropwise to diethyl ether (40 mL). After centrifugation (7800 RPM, 5 min) the supernatant was decanted and the pellet was resuspended in diethyl ether (20 mL) and centrifuged again. After decanting the supernatant the residue was dissolved in water/acetonitrile (3:1, v/v, 3 mL) and the resulting solution was directly frozen and lyophilized overnight to give the title compound (8.81 mg, 96%) as a white fluffy solid. Purity based on LC-MS 84%. Contains 14% of the hydrolysed NHS ester (Chemical Formula: C₁₁₇H₁₆₉N₇O₅₅, Exact Mass: 2552,06).
LRMS (m/z): 2551 [M-1]¹⁻
LC-MS r.t. (min): 2.76/2.78² (double peaks due to isomers)

### Synthesis of SO1861-Glu-HATU (molecule 26); see Figure 59

For producing SO1861-Glu-HATU the carboxylic group of SO1861 is activated via a reagent used in peptide coupling chemistry to generate an active ester, namely 1-[Bis(dimethylamino)methylene]-1H-1,2,3-triazolo[4,5-b]pyridinium 3-oxide hexafluorophosphate (HATU). The resulting active ester of SO1861 is shown in Figure 59.

The following modified QS-21 saponins, i.e. saponin derivatives, were synthesized based on the naturally occurring QS-21:

**Table A3: overview of the synthesized modified QS-21:**

| **Saponin derivative** | **Molecule No.** | **Derivatised group** | **Number of derivatised groups** | **Type of derivatisation** |
|---|---|---|---|---|
| QS21-Ald-OH | **27** | Aldehyde | 1 | Reduction of the aglycone core aldehyde group into an alcohol. See Figure 38. |
| QS21-Glu-AEM | **28** | Glucuronic acid | 1 | Transformation of the carboxyl group of the glucuronic acid unit in the first saccharide chain bound to C₃ of the aglycone core structure of the saponin into an amide bond through reaction with N-(2-aminoethyl)maleimide (AEM). See Figure 39. |
| QS21-(Ald-OH)-(Glu-AEM) | **29** | Aldehyde Glucuronic acid | 2 | Reduction of the aglycone core aldehyde group into an alcohol and transformation of the carboxyl group of the glucuronic acid unit in the first saccharide chain bound to C₃ of the aglycone core structure of the saponin into an amide bond through reaction with AEM. See Figure 40. |
| QS21-Ald-EMCH | **30** | aldehyde | 1 | Transformation of the aglycone core aldehyde group into a hydrazone bond through reaction with EMCH. See Figure 40B. |
| QS21-Glu-AMPD | **31** | Glucuronic acid | 1 | Transformation of the carboxyl group of the glucuronic acid unit in the first saccharide chain bound to C₃ of the aglycone core structure of the saponin into an amide bond through reaction with AMPD. See Figure 40C. |
| QS21-(Ald-EMCH)-(Glu-AMPD) | **32** | Aldehyde, glucuronic acid | 2 | Transformation of the aglycone core aldehyde group into a hydrazone bond through reaction with EMCH and transformation of the carboxyl group of the glucuronic acid unit in the first saccharide chain bound to C₃ of the aglycone core structure of the saponin into an amide bond through reaction with AMPD. See Figure 40D. |
| QS21-(Ald-OH)-(Glu-AMPD) | **33** | Aldehyde, glucuronic acid | 2 | Reduction of the aglycone core aldehyde group into an alcohol and transformation of the carboxyl group of the glucuronic acid unit in the first saccharide chain bound to C₃ of the aglycone core structure of the saponin into an amide bond through reaction with AMPD. See Figure 40E. |

### Synthesis of QS21-Ald-OH (molecule 27); see Figure 38

QS21 (9.41 mg, 4.73 µmol) was dissolved in methanol (0.50 mL). Next, sodium borohydride (1.79 mg, 0.047 mmol) was added. The reaction mixture was shaken for 1 min and left standing at room temperature. After 30 min the reaction mixture was diluted with water (0.50 mL) and submitted to preparative MP-LC.2A Fractions corresponding to the product were immediately pooled together, frozen and lyophilized overnight to give the title compound (4.68 mg, 50%) as a white fluffy solid. Purity based on LC-MS 99% (Exact mass: 1990, 4 Isomers: Api/Xyl (2:1)).
LRMS (m/z): 1990 [M-1]¹⁻
LC-MS r.t. (min): 1.25/2.31^{1B} (double peaks, 17/83 UV-area %, due to QS21 being a mixture)

### Synthesis of QS21-Glu-AEM (molecule 28); see Figure 39

QS-21 (2.42 mg, 1.22 µmol; Figure 41), AEM (1.68 mg, 6.61 µmol) and HATU (1.48 mg, 3.89 µmol) were dissolved in a mixture of DMF (0.50 mL) and NMM (1.34 µL, 0.012 mmol). The reaction mixture was shaken for 1 min and left standing at room temperature. After 1 hour the reaction mixture was subjected to preparative MP-LC.2A Fractions corresponding to the product were immediately pooled together, frozen and lyophilized overnight to give the title compound (1.80 mg, 70%) as a white fluffy solid. Purity based on LC-MS 92% (Exact mass: 2110, 4 Isomers: Api/Xyl (2:1)).
LRMS (m/z): 2110 [M-1]¹⁻
LC-MS r.t. (min): 2.84/2.93^{1B} (double peaks, 10/90 UV-area %, due to QS21 being a mixture)

### Synthesis of QS21-(Ald-OH)-(Glu-AEM) (molecule 29); see Figure 40A

QS-21-Ald-OH (1.92 mg, 0.964 µmol), AEM (1.29 mg, 5.08 µmol) and HATU (1.10 mg, 2.89 µmol) were dissolved in a mixture of DMF (0.50 mL) and NMM (1.06 µL, 9.64 µmol). The reaction mixture was shaken for 1 min and left standing at room temperature. After 1 hour the reaction mixture was subjected to preparative MP-LC.2A Fractions corresponding to the product were immediately pooled together, frozen and lyophilized overnight to give the title compound (1.46 mg, 72%) as a white fluffy solid. Purity based on LC-MS 92% (Exact mass: 2112, 4 Isomers: Api/Xyl (2:1)).
LRMS (m/z): 2112 [M-1]¹⁻
LC-MS r.t. (min): 2.83/2.92^{1B} (double peaks, 7/93 UV-area %, due to QS21 being a mixture)

### Synthesis of QS21-Ald-EMCH (Figure 40B; molecule 30)

QS21 (4.82 mg, 2.42 µmol) and EMCH.TFA (4.11 mg, 0.012 mmol) were dissolved in methanol (extra dry, 0.25 mL). The reaction mixture was shaken for 1 min and left standing at room temperature. After 2 hours the reaction mixture was subjected to to preparative MP-LC.^{2A} Fractions corresponding to the product were immediately pooled together, frozen and lyophilized overnight. Next, the product was repurified by using preparative MP-LC.^{2A} Fractions corresponding to the product were immediately pooled together, frozen and lyophilized overnight to give the title compound (2.78 mg, 52%) as a white fluffy solid. Purity based on LC-MS 96%.
LRMS (m/z): 2196 [M-1]¹⁻
LC-MS r.t. (min): 2.44^{1B} (multiple peaks due to QS21 being a mixture)

### Synthesis of QS21-Glu-AMPD (Figure 40C; molecule 31)

QS21 (4.89 mg, 2.46 µmol), AMPD (1.29 mg, 0.012 mmol) and BOP (3.26 mg, 7.37 µmol) were dissolved in a mixture of DMF (0.50 mL) and NMM (2.70 µL, 0.025 mmol). The reaction mixture was shaken for 1 min and left standing at room temperature. After 1 hour the reaction mixture was subjected to preparative MP-LC.^{2A} Fractions corresponding to the product were immediately pooled together, frozen and lyophilized overnight to give the title compound (3.76 mg, 74%) as a white fluffy solid. Purity based on LC-MS 94%.
LRMS (m/z): 2076 [M-1]¹⁻
LC-MS r.t. (min): 2.78^{1B} (multiple peaks due to QS21 being a mixture)

### Synthesis of QS21-(Ald-EMCH)-(Glu-AMPD) (Figure 40D; molecule 32)

QS21-Glu (2.47 mg, 1.19 µmol) and EMCH.TFA (2.02 mg, 5.95 µmol) were dissolved in methanol (extra dry, 100 µL). Next, TFA (0.36 µL, 4.76 µmol) was added. The reaction mixture was shaken for 1 min and left standing at room temperature. After 2 hours the reaction mixture was subjected to to preparative MP-LC.^{2A} Fractions corresponding to the product were immediately pooled together, frozen and lyophilized overnight to give the title compound (2.25 mg, 83%) as a white fluffy solid. Purity based on LC-MS 95%.
LRMS (m/z): 2283 [M-1]¹⁻
LC-MS r.t. (min): 2.88^{1B} (multiple peaks due to QS21 being a mixture)

### Synthesis of QS21-(Ald-OH)-(Glu-AMPD) (Figure 40E; molecule 33)

QS21-(Ald-OH) (4.90 mg, 2.46 µmol), AMPD (1.29 mg, 0.012 mmol) and BOP (3.26 mg, 7.37 µmol) were dissolved in a mixture of DMF (0.50 mL) and NMM (2.70 µL, 0.025 mmol). The reaction mixture was shaken for 1 min and left standing at room temperature. After 1 hour the reaction mixture was subjected to preparative MP-LC.^{2A} Fractions corresponding to the product were immediately pooled together, frozen and lyophilized overnight to give the title compound (2.16 mg, 42%) as a white fluffy solid. Purity based on LC-MS 96%.
LRMS (m/z): 2077 [M-1]¹⁻
LC-MS r.t. (min): 2.77^{1B} (multiple peaks due to QS21 being a mixture)

### Example 2: Activity of saponin derivatives - pilot study

It was found that the saponin modifications described herein not interfere substantially with the ability of the saponin to enhance endosomal escape (modified saponin or saponin freed from the conjugate inside the endosome). Results of experiments are summarized in Table Ex2, here below.

Chemically modified saponin SO1861 did show reactivity in a cell-based bioassay, with relative cell viability as the read out. HeLa cells were incubated for 72 h with the following constructs and cell viability before and after the 72 h-incubation was assessed. In the experiments, cells were exposed to 1,5 pM dianthin-EGF conjugate. A negative control were cells incubated with buffer vehicle and 10 microgram/ml saponin, without dianthin-EGF. Cell viability was set to 100% for the control in which both saponin and EGF-dianthin were omitted. Positive controls were 10 microgram/ml of non-modified saponin SO1861 + dianthin-EGF. Cell viability after 72 h was essentially 0%. For the chemically modified saponin variants, 10 microgram/ml saponin was tested in combination with 1,5 pM dianthin-EGF. SO1861-Ald-EMCH reduced cell viability at 10 microgram/ml.

These data demonstrate that the saponin can be modified at the free aldehyde group or at the free carbonyl group without losing the endosomal escape enhancing activity.

**Table Ex2. Cell killing activity (+ or -) of SO1861 and SO1861 derivatives when co-administrated with a targeted toxin (EGFdianthin). Co-administration results in enhanced cell killing compared to untreated control of EGFR expressing cells (e.g. A431, HeLa)**

| | **Buffer only** | **SO1861** | **SO1861-Ald-EMCH (also referred to as 'SO1861-EMCH')** | **SO1861-L-N₃ (also referred to as 'SO1861 N3/Azide')** | **SO1861-Glu-HATU (also referred to as 'SO1861-HATU' and 'SO1861-(S)')** |
|---|---|---|---|---|---|
| **10 µg/ml SO1861 or SO1861-derivative** | **-** | - | - | - | - |
| **10 µg/ml SO1861 or SO1861-derivative + 1.5 pM EGFdianthin** | - | + | + | + | + |

### Example 3: Activity of saponin derivatives - detailed study

Various saponins (e.g. SO1861, QS-21) were co-administrated as 'free' unconjugated molecules to cells in combination with a ligand toxin fusion (e.g. EGF-dianthin) or an antibody-protein toxin conjugate, resulting in enhanced cell killing activity of target-expressing cells.

The current inventors chemically modified SO1861 (isolated and purified from a root extract of Saponaria officinalis) and QS21 (isolated and purified from Quillaja Saponaria; Desert King) at various positions within the molecule (single, double or triple modifications), therewith providing a series of saponin derivatives as outlined in Tables A2 and A3. Saponin derivatives were tested for 1) endosomal escape enhancing activity of a ligand toxin (modified SO1861/QS21 titration + 5 pM EGFdianthin) on EGFR expressing cells (HeLa and A431); for 2) intrinsic cellular toxicity (modified SO1861/QS21 titration) on HeLa and A431; and for 3) Human red blood cell hemolysis activity (modified SO1861/QS21 titration on human red blood cells).

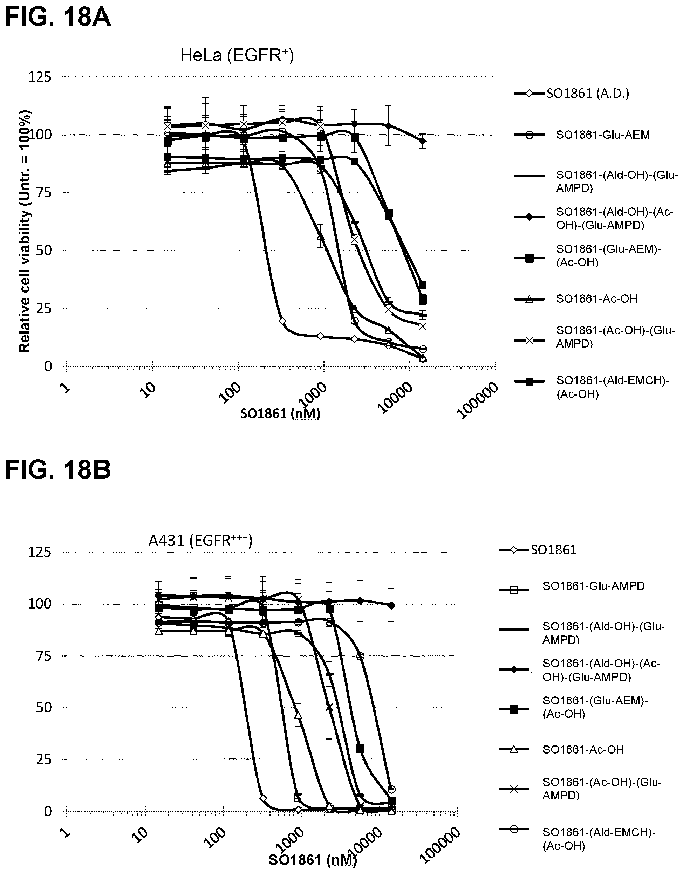

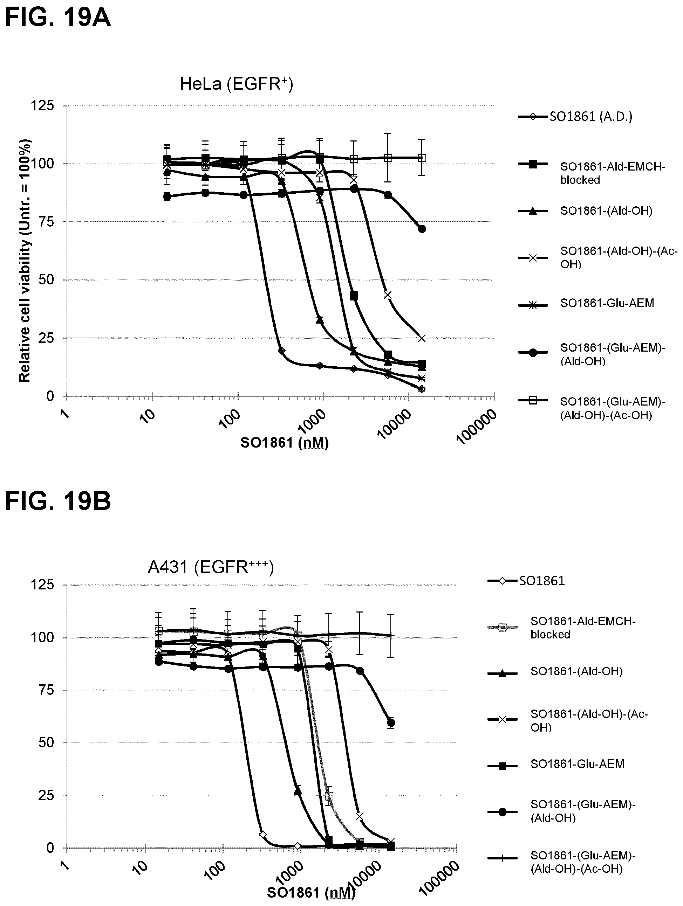

For determining the endosomal escape enhancing activity, modified SO1861 were titrated in the presence of a non-effective fixed concentration of 5 pM EGF-dianthin on EGFR expressing cells (HeLa and A431) (see Figures 18A-B and 19A-B). Furthermore, the endosomal escape enhancing activity was also determined for saponin derivatives titrated in the presence of a non-effective fixed concentration of 5 pm EGF-dianthin (see Figures 23A-B for the comparison of SO1861 with SO1861-Ald-EMCH and SO1861-Ald-EMCH-blocked- and Figures 24 A-B for SO1861 with SO1861-(Ald-EMCH)-(Ac-OH), SO1861-(Ald-EMCH)-(Glu-AMPD) and SO1861-(Ald-EMCH)-(Ac-OH)-(Glu-AMPD)). This revealed that modified saponin with single modifications compared to SO1861 showed activity at the following concentrations: SO1861-Ald-OH on HeLa: IC50 = 600 nM and A431: IC50 = 600 nM, SO1861-Glu-AMPD on HeLa: IC50 = 600 nM and A431: IC50 = 600 nM, SO1861-Ac-OH on HeLa: IC50 = 1000 nM and A431: IC50 = 800 nM, SO1861-Glu-AEM on HeLa: IC50 = 1500 nM and A431: IC50 = 2000 nM and SO1861-Ald-EMCH on HeLa: IC50 = 2000 nM and A431: IC50 = 2000 nM and the double modifications showed activity with the following IC50 values: SO1861-(Ac-OH)-(Glu-AMPD) on HeLa: IC50 = 3000 nM and A431: IC50 = 3000 nM SO1861-(Ald-OH)-(Glu-AMPD) on HeLa: IC50 = 4000 nM and A431: IC50 = 4000 nM, SO1861-(Ald-OH)-(Ac-OH) on HeLa: IC50 = 4000 nM and A431: IC50 = 5000 nM, SO1861-(Glu-AEM)-(Ac-OH) on HeLa: IC50 = 8000 nM and A431: IC50 = 4000 nM, SO1861-(Ald-EMCH)-(Ac-OH) on HeLa: IC50 = 8000 nM and A431: IC50 = 10.000 nM, SO1861-(Glu-AEM)-(Ald-OH) on HeLa: IC50 = 40.000 nM and A431: IC50 = 20.000 nM. Tested triple modifications showed no activity at the current concentrations. With unmodified SO1861 control the following IC50 values were obtained: at HeLa: IC50 = 100 nM, and at A431: IC50 = 200 nM. The endosomal escape enhancing of modified QS21 was determined by titrating the saponin derivative in the presence of a non-effective fixed concentration of 5 pM EGF-dianthin on EGFR expressing cells (see Figures 30A and 30B). This revealed that the following modified QS21 compared with unmodified QS21 showed activity at the following concentrations: QS21 on HeLa: IC50 = 200 nM and A431: IC50 = 200 nM, QS21-Ald-OH on HeLa: IC50 = 600 nM and A431: IC50 = 600 nM, QS21-Glu-AEM on HeLa: IC50 = 600 nM and A431: IC50 = 700 nM, QS21-(Ald-OH)-(Glu-AEM) on HeLa: IC50 = 1500 nM and A431: IC50 = 3000 nM. In conclusion, unmodified SO1861 and QS21 were both effective at IC50 = 200 nM in HeLa and A431 cells. Single SO1861/QS21 modifications (SO1861-Ald-EMCH, SO1861-Ald-EMCH-blocked, SO1861-Glu-AMPD, SO1861-Ald-OH, SO1861-Ac-OH, SO1861-Glu-AEM, QS21-Ald-OH, QS21-Glu-AEM) showed activity at IC50 = 600 nM - 2000 nM in HeLa or A431 (Table A5 and Table A6) whereas double SO1861/QS21 modification showed activity at IC50 = 1500-40.000 nM in Hela or A431 cells (Table A5 and Table A6). For the triple modification of SO1861 no activity could be observed up to 20.000 nM.

As said, for determining the endosomal escape enhancing activity, SO1861 derivatives, QS21 derivatives and their non-derivatised counterparts were titrated in the presence of a non-effective fixed concentration of 5 pM EGFdianthin on EGFR expressing cells (HeLa and A431). This revealed that non-derivatised SO1861 and non-derivatised QS21 and the QS21 derivative QS21-Glu-AMPD were effective at IC50 = 200 nM in HeLa and A431 cells. Single SO1861/QS21 modifications (SO1861-Ald-EMCH, SO1861-Ald-EMCH (blocked) (SO1861-Ald-EMCH(mercaptoethanol), SO1861-Glu-AMPD, SO1861-(Ald-OH), SO1861-Ac-OH, SO1861-Glu-AEM, QS21-Ald-EMCH, QS21-(Ald-OH), QS21-Glu-AEM) showed activity at IC50 = 600 nM - 2000 nM in HeLa or A431 (Table A5 and A6) whereas double SO1861 modification and double QS21 modification showed activity at IC50 = 1500-40.000 nM in Hela or A431 cells (Table A5 and A6). For the triple modification of SO1861 no activity could be observed up to 20.000 nM.

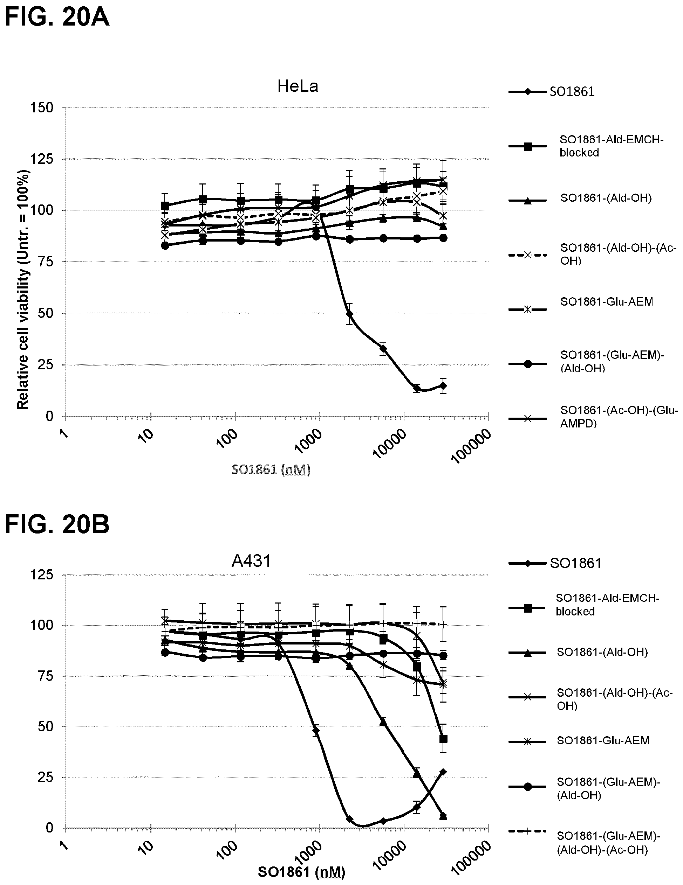

For the toxicity determination modified SO1861 was titrated on HeLa cells (see Figures 20A and 21A) and A431 cells (see Figures 20B and 21B). Figures 25A and 25B depict a detail of the toxicity test for SO1861, SO1861-Ald-EMCH, SO1861-Ald-EMCH-blocked, Figures 26A and 26B display a detail for SO1861, SO1861-(Ald-EMCH)-(Ac-OH), SO1861-(Ald-EMCH)-(Glu-AMPD) and SO1861-(Ald-EMCH)-(Ac-OH)-(Glu-AMPD). This revealed that unmodified SO1861 on HeLa cells shows strongest intrinsic toxicity: IC50 = 2000 nM whereas the single modifications SO1861-Ac on HeLa cells show toxicity at IC50 = 10.000 nM. For all other SO1861-derivatives intrinsic toxicity on HeLa cells (IC50) was higher than 20.000 nM. In A431 cells toxicity of unmodified SO1861 is observed at IC50: 1000 nM whereas the single modifications SO1861-Ac-OH, SO1861-Ald-OH, SO1861-Glu-AMPD, SO1861-Ald-EMCH show toxicity at receptively IC50 = 2000 nM, IC50 = 7000 nM, IC50 = 20.000 nM and IC50 = 30.000 nM. For the toxicity determination of modified QS21, the saponin derivative was titrated on HeLa cells (see Figure 31A) and A431 cells (Figures 31B). This revealed that QS21 shows toxicity on HeLa cells: IC50 = 6000 nM and A431 cells: IC50 = 3000 nM, QS21-Ald-OH on HeLa cells: IC50 = 20.000 nM and A431 cells: IC50 = 20.000 nM, QS21-Glu-AEM on HeLa cells: IC50 = >100.000 nM and A431 cells: IC50 = > 100.000 nM, QS21-(Ald-OH)-(Glu-AEM) on HeLa cells: IC50 = >100.000 nM and A431 cells: IC50 = > 100.000 nM, For the SO1861or QS21 double modifications and SO1861 triple modifications no toxicity could be observed up to 100.000 nM. As said, unmodified or modified SO1861 or QS21 were titrated on HeLa and A431 cells. This revealed that unmodified SO1861 showed toxicity at IC50 = 1000 nM (HeLa) and IC50 = 2000 nM (A431), whereas QS21 showed toxicity at IC50 = 6000 nM (HeLa) (IC50 = 3000 nM (A431) and QS21-Glu-AMPD showed toxicity at IC50 = 9000 nM (HeLa) and IC50 = 5000 nM (A431) (Table A5 and A6). For the single SO1861 modifications and for the single QS21 modifications on HeLa cells, SO1861-Ald-EMCH, SO1861-Ald-EMCH (blocked), SO1861-(Ald-OH), SO1861-Glu-AEM, QS21-Ald-EMCH, QS21-Glu-AEM showed no toxicity up to 100.000 nM, whereas SO1861-Glu-AMPD, SO1861-Ac-OH, QS21-(Ald-OH) showed toxicity at respectively IC50 = 20.000 nM, IC50 = 10.000 nM, IC50= 20.000 nM (Table A5 and A6). In A431 cells, SO1861-Glu-AEM and QS21-Glu-AEM showed no toxicity up to 100.000 nM whereas toxicity could be observed for SO1861-Ald-EMCH (IC50 = 30.000 nM), SO1861-Ald-EMCH (blocked) (IC50 = 30.000 nM), SO1861-(Ald-OH) (IC50 = 7000 nM), SO1861-Ac-OH (IC50 = 2000 nM), SO1861-Glu-AMPD (IC50 = 20.000 nM), QS21-Ald-EMCH (IC50 = 30.000 nM), QS21-(Ald-OH) (IC50 = 20.000 nM) (Table A5 and A6). For the SO1861 double modifications or QS21 double modifications and SO1861 triple modifications no toxicity could be observed up to 100.000 nM (Table A5 and A6).

In addition, hemolysis activity of the unmodified and modified SO1861 was determined by a human red blood cell hemolysis assay (see Figure 22, Figure 27, Figure 28 and Figure 32). This revealed that unmodified SO1861 showed activity at IC50= 8000 nM and unmodified QS21 at IC50 = 3000 nM. The single modifications SO1861-Ald-EMCH showed no hemolytic activity up to 1.000.000 nM, whereas hemolytic activity of human red blood cells could be observed for SO1861-Ald-EMCH-blocked (IC50 = 300.000 nM), SO1861-Ald-OH (IC50 = 30.000 nM), SO1861-Ac-OH (IC50 = 20.000 nM), SO1861-Glu-AMPD (IC50 = 20.000 nM), SO1861-Glu-AEM (IC50 = 30.000 nM) QS21-Ald-OH (IC50 = 20.000 nM), and QS21-Glu-AEM (IC50 = 10.000 nM) (Table A5 and Table A6). For the SO1861 or QS21 double modifications no hemolytic activity (up to 1.000.000 nM) was observed for SO1861-(Ald-EMCH)-(Glu-AMPD), SO1861-(Ald-EMCH)-(Ac-OH), SO1861-(Glu-AEM)-(Ald-OH) and QS21-(Ald-OH)-(Glu-AEM), whereas hemolytic activity was observed for SO1861-(Ald-OH)-(Glu-AMPD) (IC50 = 100.000), SO1861-(Ald-OH)-(Ac-OH) (IC50 = 200.000), SO1861-(Ac-OH)-(Glu-AMPD) (IC50= 140.000), SO1861-(Glu-AEM)-(Ac-OH) (IC50= 100.000). For none of the SO1861 triple modifications hemolytic could be observed up to 100.000 nM. The hemolysis assay revealed hemolytic activity for unmodified SO1861 at IC50 = 8000 nM and unmodified QS21 at IC50 = 3000 nM and modified QS21-Glu-AMPD at IC50 = 3000 nM. The single modification SO1861-Ald-EMCH showed no hemolytic activity up to 1.000.000 nM, whereas hemolytic activity of human red blood cells could be observed for SO1861-Ald-EMCH (blocked) (IC50 = 300.000 nM), SO1861-(Ald-OH) (IC50 = 30.000 nM), SO1861-Ac-OH (IC50 = 20.000 nM), SO1861-Glu-AMPD (IC50 = 20.000 nM), SO1861-Glu-AEM (IC50 = 30.000 nM) QS21-Ald-EMCH (IC50 = 30.000 nM), QS21-(Ald-OH) (IC50 = 20.000 nM), and QS21-Glu-AEM (IC50 = 10.000 nM) (Table A5 and A6). For the SO1861 double modifications or QS21 double modifications no hemolytic activity (up to 1.000.000 nM) was observed for SO1861-Ald-EMCH-(Glu-AMPD), SO1861-(Ac-OH)-EMCH, SO1861-(Ald-OH)-(Glu-AEM), QS21-Ald-EMCH-(Glu-AMPD) and QS21-(Ald-OH)-(Glu-AEM), whereas hemolytic activity was observed for SO1861-(Ald-OH)-(Glu-AMPD) (IC50 = 100.000 nM), SO1861-(Ald-OH)-(Ac-OH) (IC50 = 200.000 nM), SO1861-(Ac-OH)-(Glu-AMPD) (IC50 = 140.000 nM), SO1861-(Ac-OH)-(Glu-AEM) (IC50 = 100.000 nM) and QS21-(Ald-OH)-(Glu-AMPD) (IC50 = 40.000 nM) (Table A5 and A6). For none of the SO1861 triple modifications hemolytic could be observed up to 100.000 nM (Table A5 and A6).

The endosomal escape enhancing activity (titration of saponin + 5 pM cetuximab-Saporin on HeLa and A431 cells, see Figures 33A-B), toxicity (titration of saponins on HeLa and A431 cell, see Figures 34A-B) and hemolytic activity (titration of saponins on human red blood cells, see Figure 32 and Figure 35) of various QS saponins fractions was tested. This revealed that QS21 (fraction), QS17 (fraction), QS18 (fraction) showed activity at 200 nM in HeLa cells and A431 cells, whereas QS7 (fraction) showed activity at IC50= 6000 nM (HeLa) and 10.000 nM (A431). When it was observed that during the determination of the toxicity that QS21 (fraction), QS17 (fraction), QS18 (fraction) showed toxicity at IC50 = 10.000 nM in HeLa cells and A431 cells, whereas QS7 (fraction) showed toxicity at IC50 = 20.000 nM (Figure 34). Next, hemolysis assay was performed and this revealed hemolytic activity of QS21 (fraction) at IC50 = 3000 nM QS17 (fraction), QS18 (fraction) at IC50 = 5000 nM, whereas for QS7 (fraction) no hemolytic activity could be detected up to 20.000 nM (Figure 35).

The hemolytic activity of various SO saponins (SO1862 (isomer), SO1832, SO1904) was tested as well as the antibody-SO1861 conjugates (cetuximab-SO1861 (DAR4), trastuzuzmab-SO1861 (DAR4)). This revealed that hemolytic activity of SO1862 (isomer, SO1832, SO1904 was comparable with SO1861 (IC50 = 10.000 nM) (Figure 29). The cetuximab-SO1861 (DAR4) conjugate showed no hemolytic activity up to 60.000 nM whereas the trastuzumab-SO1861 (DAR4) showed initial hemolytic activity from 60.000 nM onwards (IC50 = 200.000 nM) (Figure 29).

When comparing the cytotoxicity, the hemolytic activity and the endosomal escape enhancing activity of SO1861, SO1861-Ald-EMCH and SO1861-Ald-EMCH-mercaptoethanol (SO1861-Ald-EMCH-Blocked), the latter two were similarly or essentially equally cytotoxic, hemolytically active and active in the endosomal escape enhancing activity bio-assay, and the latter two were less cytotoxic and less hemolytically active than SO1861. See also Table A5 and Table A6.

### Cell viability assay

Cell viability was determined by an MTS-assay, performed according to the manufacturer's instruction (CellTiter 96^{®} AQueous One Solution Cell Proliferation Assay, Promega). The MTS solution was diluted 20x in DMEM without phenol red (PAN-Biotech GmbH) supplemented with 10% FBS (PAN-Biotech GmbH). The cells were washed once with 200 µL PBS per well, after which 100 µL diluted MTS solution was added per well. The plate was incubated for approximately 20-30 minutes at 37°C. Subsequently, the optical density at 492 nm was measured on a Thermo Scientific Multiskan FC plate reader (Thermo Scientific). For quantification the background signal of 'medium only' wells was subtracted from all other wells, before the ratio of untreated/treated cells was calculated, by dividing the background corrected signal of untreated wells over the background corrected signal of the treated wells.

### FACS analysis

Cells were seeded in DMEM (PAN-Biotech GmbH) supplemented with 10% fetal calf serum (PAN-Biotech GmbH) and 1% penicillin/streptomycin (PAN-Biotech GmbH), at 500,000 c/plate in 10 cm dishes and incubated for 48 hrs (5% CO₂, 37°C), until a confluency of 90% was reached. Next, the cells were trypsinized (TryplE Express, Gibco Thermo Scientific) to single cells. 0.75 x 10⁶ Cells were transferred to a 15 mL falcon tube and centrifuged (1,400 rpm, 3 min). The supernatant was discarded while leaving the cell pellet submerged. The pellet was dissociated by gentle tapping the falcon tube on a vortex shaker and the cells were washed with 4 mL cold PBS (Mg²⁺ and Ca²⁺ free, 2% FBS). After washing, the cells were resuspended in 3 mL cold PBS (Mg²⁺ and Ca²⁺ free, 2% FBS) and divided equally over 3 round bottom FACS tubes (1 mL/tube). The cells were centrifuged again and resuspended in 200 µL cold PBS (Mg²⁺ and Ca²⁺ free, 2% FBS) or 200 µL antibody solution; containing 5 µL antibody in 195 µL cold PBS (Mg²⁺ and Ca²⁺ free, 2% FBS). APC Mouse IgG1, κ Isotype Ctrl FC (#400122, Biolegend) was used as isotype control, and APC anti-human EGFR (#352906, Biolegend) was used. Samples were incubated for 30 min at 4 °C on a tube roller mixer. Afterwards, the cells were washed 3x with cold PBS (Mg²⁺ and Ca²⁺ free, 2% FBS) and fixated for 20 min at room temperature using a 2% PFA solution in PBS. Cells were washed 2x with cold PBS, and resuspended in 250-350 µL cold PBS for FACS analysis. Samples were analyzed with a BD FACSCanto II flow cytometry system (BD Biosciences) and FlowJo software. Results of the FACS analyses are summarized in Table A4.

**Table A4. Cell surface expression levels (Mean Fluorescent Intensity (MFI) of EGFR and HER2 in various cell lines.**

| **Cell line** | **EGFR expression level (MFI)** | **HER2 expression (MFI)** |
|---|---|---|
| **A431** | 1593 | 10 |
| **HeLa** | 91 | 7 |
| **SK-BR-3** | 28 | 1162 |
| **A2058** | 1 | 5 |

### Hemolysis assay

Red blood cells (RBCs) were isolated from a buffy coat using a Ficoll gradient. The obtained RBC pellet (~4-5 ml) was washed 2x with 50 ml DPBS (without Ca²⁺/Mg²⁺, PAN-Biotech GmbH). Cells were pelleted by centrifugation for 10 min, 800xg at RT. RBC were counted and resuspended at 500.000.000 c/ml in DPBS (without Ca²⁺/Mg²⁺), based on total cell count.

Saponin dilutions were prepared in DPBS (with Ca²⁺/Mg²⁺, PAN-Biotech GmbH), at 1.11x final strength. For positive lysis control a 0.02% Triton-X100 solution was prepared in DPBS^{+/+}. Of all compound solutions 135 µl was dispensed/well in a 96 well V-bottom plate. To this 15 µl RBC suspension was added and mixed shortly (10 sec - 600 rpm). The plate was incubated 30 min at RT, with gentle agitation. Afterwards the plate was spun for 10 min at 800xg to pellet the RBC and 100-120 µl supernatant was transferred to a standard 96 wp (96 well-plate). Subsequently, the OD at 405 nm was measured on a Thermo Scientific Multiskan FC plate reader (Thermo Scientific). For quantification the background signal of 'DPBS^{+/+} only' wells was subtracted from all other wells before the percentage of hemolysis was calculated in comparison to 0.02% Triton-X100, by dividing the background corrected signal of treated wells over the background corrected signal of the 0.02% Triton-X100 wells (x 100).

**TABLE A5. Treatment of red blood cells and of HeLa cells with SO1861, SO1861 derivatives, QS-21 derivatives and QS-21.**

| Conjugate Sample | IC50 nM (Activity) | IC50 nM (Toxicity) | IC50 nM (hemolysis) | Ratio: IC50 toxicity / IC50 activity | Ratio: IC50 hemolysis / IC50 activity |
|---|---|---|---|---|---|
| SO1861 | 200 | 2000 | 10.000 | 11 | 70 |
| QS21 | 200 | 6000 | 3000 | 30 | 15 |
| SO1861-Ald-EMCH | 1500 | > 100.000 | >1.000.000 | >50 | >500 |
| SO1861-Ald-EMCH-blocked^{≠} | 1500 | > 100.000 | 300.000 | >50 | 200 |
| SO1861-Glu-AMPD | 600 | 20.000 | 20.000 | 66 | 66 |
| SO1861-Ald-OH | 600 | > 100.000 | 30.000 | >166 | 88 |
| SO1861-Ac-OH | 1.000 | 10.000 | 20.000 | 10 | 30 |
| SO1861-(Ald-OH)-(Glu-AMPD) | 4.000 | > 100.000 | 100.000 | >25 | 25 |
| SO1861-(Ald-OH)-(Ac-OH) | 5.000 | > 100.000 | 200.000 | >20 | 40 |
| SO1861-(Ac-OH)-(Glu-AMPD) | 3.000 | > 100.000 | 140.000 | >33 | 46 |
| SO1861-(Ald-OH)-(Ac-OH)-(Glu-AMPD) | >20.000 | > 100.000 | >1.000.000 | Not active / no tox | Not active / no tox |
| SO1861-(Ald-EMCH)-(Glu-AMPD) | 5000 | > 100.000 | >1.000.000 | >20 | >200 |
| SO1861-(Ald-EMCH)-(Ac-OH) | 8.000 | > 100.000 | >1.000.000 | >12 | >125 |
| SO1861-(Ald-EMCH)-(Ac-OH)-(Glu-AMPD) | >20.000 | > 100.000 | >1.000.000 | Not active / no tox | Not active / no tox |
| SO1861-Glu-AEM | 1.500 | > 100.000 | 30.000 | >66 | 20 |
| SO1861-(Glu-AEM)-(Ac-OH) | 8.000 | > 100.000 | 100.000 | >12 | 12 |
| SO1861-(Glu-AEM)-(Ald-OH) | 40.000 | > 100.000 | >1.000.000 | >2 | >25 |
| SO1861-(Glu-AEM)-(Ald-OH)-(Ac-OH) | >20.000 | > 100.000 | >1.000.000 | Not active / no tox | Not active / no tox |
| QS21-Ald-OH | 600 | 20.000 | 20.000 | 33 | |
| QS21-Glu-AEM | 600 | >100.000 | 10.000 | >166 | 16 |
| QS21-(Ald-OH)-(Glu-AEM) | 1500 | >100.000 | >1.000.000 | >50 | >500 |

| | | | | | |
|---|---|---|---|---|---|
| ‡ See Molecule 3, also referred to as SO1861-Ald-EMCH-mercaptoethanol or SO1861-Ald-EMCH(mercaptoethanol) | | | | | |

**TABLE A6. Treatment of red blood cells and of A431 cells with SO1861, SO1861 derivatives, QS-21 derivatives and QS-21.**

| Conjugate Sample | IC50 nM (Activity) | IC50 nM (Toxicity) | IC50 nM (hemolysis) | Ratio: IC50 toxicity / IC50 activity | Ratio: IC50 hemolysis / IC50 activity |
|---|---|---|---|---|---|
| SO1861 | 200 | 1000 | 8000 | 5 | 40 |
| QS21 | 200 | 3000 | 3000 | 15 | 15 |
| SO1861-Ald-EMCH | 1500 | 30.000 | >1.000.000 | 15 | >500 |
| SO1861-Ald-EMCH-blocked | 1500 | 30.000 | 300.000 | 20 | 200 |
| SO1861-Glu-AMPD | 600 | 20.000 | 20.000 | 33 | 33 |
| SO1861-Ald-OH | 600 | 7000 | 30.000 | 11 | 50 |
| SO1861-Ac-OH | 800 | 2000 | 20.000 | 2,5 | 25 |
| SO1861-(Ald-OH)-(Glu-AMPD) | 4.000 | > 100.000 | 100.000 | >25 | 25 |
| SO1861-(Ald-OH)-(Ac-OH) | 4.000 | > 100.000 | 200.000 | >25 | 50 |
| SO1861-(Ac-OH)-(Glu-AMPD) | 3.000 | > 100.000 | 140.000 | >33 | 46 |
| SO1861-(Ald-OH)-(Ac-OH)-(Glu-AMPD) | >20.000 | > 100.000 | >1.000.000 | Not active / no tox | Not active / no tox |
| SO1861-(Ald-EMCH)-(Glu-AMPD) | 15.000 | > 100.000 | >1.000.000 | >7 | >66 |
| SO1861-(Ald-EMCH)-(Ac-OH) | 10.000 | > 100.000 | >1.000.000 | >10 | >100 |
| SO1861-(Ald-EMCH)-(Ac-OH)-(Glu-AMPD) | >20.000 | > 100.000 | >1.000.000 | Not active / no tox | Not active / no tox |
| SO1861-Glu-AEM | 2000 | > 100.000 | 30.000 | >50 | 15 |
| SO1861-(Glu-AEM)-(Ac-OH) | 4.000 | > 100.000 | 100.000 | >25 | 25 |
| SO1861-(Glu-AEM)-(Ald-OH) | 20.000 | > 100.000 | >1.000.000 | >50 | >50 |
| SO1861-(Glu-AEM)-(Ald-OH)-(Ac-OH) | >20.000 | > 100.000 | >1.000.000 | Not active / no tox | Not active / no tox |
| QS21-Ald-OH | 600 | 20.000 | 20.000 | 33 | 33 |
| QS21-Glu-AEM | 700 | > 100.000 | 10.000 | >142 | 14 |
| QS21-(Ald-OH)-(Glu-AEM) | 3000 | > 100.000 | >1.000.000 | >33 | >333 |

### Example 4: Critical micellar concentration (CMC) of saponin derivatives

### Materials and Methods

The critical micellar concentration (CMC) of saponins derived from Saponaria Officinalis (SO) (Table A7) and derived from Quillaja Saponaria (QS) (Table A8, and Table A9) was determined by the method of DeVendittis et al. (A fluorimetric method for the estimation of the critical micelle concentration of surfactants, Analytical Biochemistry, Volume 115, Issue 2, August 1981, Pages 278-286) as follows:
The emission spectrum of 8-Anilinonaphthalene-1-sulfonic acid (ANS) in either purified water (MQ) or PBS (Dulbecco's PBS +/+) was determined at dry weight concentrations of saponins ranging from 1 to 1400 µM to cover the range below and above the CMC. Above the CMC, the fluorescence yield of ANS increases and the wavelength of maximum emission decreases due to portioning of the fluorescent dye into micelles. Fluorescence yields were recorded on a Fluoroskan Ascent FL (Thermo Scientific) at an excitation wavelength of 355 nm, and an emission wavelength of 460 nm. 6 µg at a concentration of 75.86 µM of ANS were used per sample and measurement.

### Results

### SO1861 saponins

The chemical modification at the functional groups aldehyde (Ald), glucuronic acid (Glu), and the removal of the acetyl group (Ac) showed an impact on micellar properties of the respective saponins. As shown in Figure 42, the mono-modification of the respective functional groups on the SO1861 saponin significantly influenced the micelle formation ability represented in the slope of the obtained relative fluorescence values of ANS. The modifications on the glucuronic acid (SO1861-Glu-AMPD, SO1861-Glu-AEM) clearly resulted in steeper slopes (Figure 42) resulting in lower CMC values as obtained for the native SO1861 of 185 µM. The similar observation has been obtained for the SO1861-Ald-EMCH-blocked sample. The modifications on the aldehyde and acetyl group, however, (SO1861-Ald-OH, SO1861-Ald-EMCH, SO1861-Ac-OH) resulted in significantly flatter slopes (Figure 42), leading to higher CMC values with respect to the native SO1861. The SO1861-Ald-EMCH sample was particularly interesting as the obtained slope was nearly flat and no CMC could be determined even for concentrations up to 800 µM.

Similar observations with respect to the site of modification have been obtained for bi-modification (Figure 43) and tri-modification (Figure 44) of the SO1861 saponin. While modifications on the glucuronic acid (SO1861-(Ald-OH)-(Glu-AMPD), SO1861-(Ac-OH)-(Glu-AMPD), SO1861-(Glu-AEM)-(Ac-OH), SO1861-(Glu-AEM)-(Ald-OH), SO1861-(Ald-EMCH)-(Glu-AMPD)) resulted in steeper ANS fluorescence yield slopes and thus in lower CMC values, modifications on the aldehyde and the acetyl position (SO1861-(Ald-OH)-(Ac-OH), SO1861-(Ald-EMCH)-(Ac-OH)) lead to flat ANS fluorescence yield slopes and thus to increased CMC values with respect to the native SO1861 (Table A7).

When comparing the tri-modified saponins SO1861-(Glu-AEM)-(Ald-OH)-(Ac-OH) and SO1861-(Ald-OH)-(Ac-OH)-(Glu-AMPD), the modification on the glucuronic acid at SO1861-(Glu-AEM)-(Ald-OH)-(Ac-OH) resulted in a flatter slope of the respective ANS fluorescence yields while the modification on the glucuronic acid at SO1861-(Ald-OH)-(Ac-OH)-(Glu-AMPD) resulted in a steeper slope of the respective ANS fluorescence yields with respect to the native SO1861 (Figure 44). These results indicate the importance of modification at the aldehyde and/or the acetoxy position in case CMC is considered, since even in Glu-modified derivatives (which have a lower CMC than free saponin), said aldehyde and/or the acetoxy modifications can increase the CMC, at least partially mitigating the negative effect of Glu-modification, when CMC is considered .

**Table A7. CMC values of SO saponins determined in PBS**

| **Saponin** | **CMC (µM)** |
|---|---|
| SO1861 | 185 ± 19 |
| | |
| SO1861-Ald-EMCH | n.d. |
| SO1861-Ac-OH | 350 ± 35 |
| SO1861-Ald-OH | 135 ± 14 |
| SO1861-Glu-AMPD | 50 ± 5 |
| SO1861-Ald-EMCH-blocked | 50 ± 5 |
| SO1861-Glu-AEM | 45 ± 5 |
| | |
| SO1861-(Ald-EMCH)-(Ac-OH) | > 300 |
| SO1861-(Ald-OH)-(Ac-OH) | > 280 |
| SO1861-(Glu-AEM)-(Ald-OH) | 90 ± 10 |
| SO1861-(Glu-AEM)-(Ac-OH) | 90 ± 10 |
| SO1861-(Ac-OH)-(Glu-AMPD) | 90 ± 10 |
| SO1861-(Ald-EMCH)-(Glu-AMPD) | > 30 |
| SO1861-(Ald-OH)-(Glu-AMPD) | 50 ± 5 |
| | |
| SO1861-(Glu-AEM)-(Ald-OH)-(Ac-OH) | > 150 |
| SO1861-(Ald-OH)-(Ac-OH)-(Glu-AMPD) | 120 ± 12 |
| SO1861-(Ald-EMCH)-(Ac-OH)-(Glu-AMPD) | n.d. |

### QS saponins

For the saponins derived from Quillaja Saponaria (QS), QS7, QS17, QS18, QS21 Frac, and QS21 SP CMC values have been determined which are displayed in Table A8. As shown in Figure 45, the slopes of the ANS fluorescence yields of the respected QS saponins are in correspondence to the derived CMC values. The obtained CMC values show a decreasing tendency starting with QS21 SP at the highest CMC value of 49 µM, over QS-17, QS-18, and QS-21 Frac, that all show a similar CMC at around 70 µM. Finally, for QS-7 a CMC value of 230 µM was obtained.

When comparing the ANS fluorescence yields of QS21 SP measured in purified water (MQ) and PBS, the slope in purified water (MQ) is slightly steeper leading to expected slightly higher CMC values in purified water (Figure 46, Table A9).

For the mono-modified QS21 saponins QS21-Ald-EMCH (molecule 30; Figure 40B), QS21-Glu-AEM, QS21-(Ald-OH), and QS21-Glu-AMPD (Figure 47A, Figure 47B) only the AMPD modification on the glucuronic acid (QS21-Glu-AMPD, Figure 47B) led to a steeper slope of the ANS fluorescence yields with respected to native QS21 resulting in a lower CMC value of 40 µM (Table A9). All other QS21 mono-modifications both at the glucuronic acid (QS21-Glu-AEM) and aldehyde position (QS21-(Ald-OH), QS21-Ald-EMCH) resulted in flatter ANS fluorescence yield slopes than the native QS21 (Figure 47B).

Similar to the finding for bi-modifications on the saponaria officinalis saponin SO1861, also the AldGlu modification of the QS21 saponin (QS21-(Ald-OH)-(Glu-AMPD), Figure 40E, molecule 33, Figure 47C) resulted in a steeper slope of the ANS fluorescence yields with respected to native QS21 leading to a lower CMC value of 39 µM (Table A9). All other QS21 bi-modifications both at the glucuronic acid and aldehyde position (QS21-(Ald-OH)-(Glu-AEM), QS21-Ald-EMCH-(Glu-AEM), Figure 47C) resulted in flatter ANS fluorescence yield slopes than the native QS21.

**Table A8. CMC values of QS saponins determined in purified water (MQ)**

| **Saponin** | **CMC (µM)** |
|---|---|
| QS7 | 230 ± 25 |
| QS21 (Frac) | 75 ± 8 |
| QS17 | 70 ± 7 |
| QS18 | 68 ± 7 |
| QS21 (SP) | 49 ± 5 |

**Table A9. CMC values of modified QS21 saponins determined in PBS**

| **Saponin** | **CMC (µM)** |
|---|---|
| QS21 (SP) in MQ | 49 ± 5 |
| QS21 (SP) in PBS | 40 ± 5 |
| | |
| QS21-Ald-OH | > 60 |
| QS21-Ald-EMCH | n.d. |
| QS21-Glu-AMPD | 20 |
| QS21-Glu-AEM | n.d. |
| QS21-(Ald-OH)-(Glu-AEM) | n.d. |
| QS21-Ald-EMCH-(Glu-AMPD) | n.d. |
| QS21-(Ald-OH)-(Glu-AMPD) | 39±4 |

### Example 5: Endosomal escape enhancing activity of SO1861 and SO1861-Ald-EMCH

SO1861 and SO1861-Ald-EMCH (also referred to as SO1861-EMCH, for example in Figures 48-58) were tested for their ability to enhance endosomal escape of a targeted protein toxin. For this, SO1861 or SO1861-Ald-EMCH was titrated on a fixed concentration of 10 pM cetuximab-saporin (cetuximab conjugated to the protein toxin, saporin, with a DAR4) on EGFR expressing cells (A431). This revealed that SO1861 (IC50 = 800 nM) and SO1861-Ald-EMCH (IC50 = 2000 nM) induce efficient cell killing of A431 cells in combination with 10 pM cetuximab-saporin, whereas SO1861 or SO1861-Ald-EMCH alone showed no cell killing activity (Figure 48).

Next, cetuximab-dianthin or cetuximab-saporin were titrated on various fixed concentrations of SO1861 or SO1861-Ald-EMCH. This revealed efficient cell killing with low pM concentrations of cetuximab-dianthin (IC50 = 1 pM, Figure 49) or cetuximab-saporin (IC50 = 0,5 pM, Figure 50) in the presence of 4000 nM SO1861-Ald-EMCH, 4829 nM SO1861-Ald-EMCH or 1500 nM SO1861. This cell killing effect was not observed with 300 nM SO1861 or 300 nM SO1861-Ald-EMCH (Figure 49 and 50).

Next, SO1861 or SO1861-Ald-EMCH was titrated on a fixed concentration of 10 pM EGFdianthin (EGFR targeting fusion protein toxin) on EGFR expressing cells (A431). This revealed that SO1861 (IC50 = 800 nM) and SO1861-Ald-EMCH (IC50 = 2000 nM) induce efficient cell killing of A431 cells in combination with 10 pM EGFdianthin, whereas SO1861 or SO1861-Ald-EMCH alone showed no cell killing activity (Figure 51).

Next, EGFdianthin was titrated on various fixed concentration of SO1861 or SO1861-Ald-EMCH. This revealed efficient cell killing with low pM concentrations of EGFdianthin (IC50 = 0,1 pM, Figure 52) in the presence of 4829 nM SO1861-Ald-EMCH or 1500 nM SO1861. This cell killing effect was not observed with 10 nM SO1861 or 300 nM SO1861 (Figure 52).

Next, trastuzumab-dianthin or trastuzumab-saporin (trastuzumab conjugated to the protein toxin, saporin, with a DAR4) was titrated on a fixed concentration of 1500 nM SO1861 or 4000 nM SO1861-Ald-EMCH on HER2 expressing cells (SK-BR-3). This revealed efficient cell killing with low pM concentrations of trastuzumab-dianthin (IC50 = 0,1 pM) or trastuzumab-saporin (IC50 = 0,1 pM) in the presence of 1500 nM SO1861 or 4000 nM SO1861-Ald-EMCH (Figure 53).

All these results outlined in Figures 48-53 show that SO1861-Ald-EMCH efficiently enhances endosomal escape and cytoplasmic delivery of a targeted protein toxin, thereby significantly reducing the effective concentration of the targeted protein toxin from nM range to low pM range.

SO1861-Ald-EMCH was tested for its ability to enhance endosomal escape of an antisense oligo nucleotide (BNA, bridged nucleic acid) against HSP27 mRNA. For this, SO1861-Ald-EMCH was titrated on a fixed concentration of 100 nM HSP27BNA, 100 nM cetuximab-HSP27BNA (cetuximab conjugated to the HSP27BNA, with a DAR4) or 100 nM trastuzumab-HSP27BNA (trastuzumab conjugated to the HSP27BNA, with a DAR4) on EGFR/HER2 expressing cells (A431). This revealed that SO1861-Ald-EMCH (IC50 = 700 nM) induces efficient HSP27 gene silencing cells in combination with 100 nM HSP27BNA, 100 nM cetuximab-HSP27BNA (Figure 54) or 100 nM trastuzumab-HSP27BNA in A431 cells (not shown). SO1861-Ald-EMCH alone showed no HSP27 gene silencing activity (Figure 54).

Next, cetuximab-HSP27BNA (DAR1.5 or DAR4), trastuzumab-HSP27BNA (DAR4.4) was titrated on various fixed concentration of SO1861-Ald-EMCH in EGFR (A431) or HER2 (SK-BR-3) expressing cells. This revealed efficient HSP27 gene silencing in A431 cells with low nM concentrations of cetuximab-HSP27BNA (IC50 = 0,5 nM, Figure 55) in the presence of 4000 nM SO1861-Ald-EMCH, whereas cetuximab-HSP27BNA alone or cetuximab-HSP27BNA + 100 nM SO1861-Ald-EMCH showed no gene silencing activity or only slight activity at very high concentrations (IC50 > 100 nM; Figure 550). In SKBR-3 cells, trastuzumab-HSP27BNA (IC50 = 0,5 nM, Figure 56) in the presence of 4000 nM SO1861-Ald-EMCH showed efficient HSP27 gene silencing activity, whereas trastuzumab-HSP27BNA alone or trastuzumab-HSP27BNA + 100 nM SO1861-Ald-EMCH showed only slight gene silencing activity (IC50 > 100 nM; Figure 56).

Next, untargeted HSP27BNA was titrated on a fixed concentration of SO1861-Ald-EMCH in various cell lines. This revealed effective HSP27 gene silencing (Figure 57 and 58) in A431, A2058 and SK-BR-3 cells with low nM concentrations of HSP27BNA (IC50(SK-BR3) = 2 nM; IC50(A431) = 10 nM; IC50 (A2058) = 10 nM) in the presence of 4000 nM or 4829 nM SO1861-Ald-EMCH, whereas HSP27BNA alone induced gene silencing at much higher concentrations (IC50(SK-BR3) = 300 nM; IC50(A431) = 1000 nM; IC50 (A2058) > 1000nM) (Figure 57 and 58). When that activity (with and without SO1861-Ald-EMCH) of HSP27BNA was compared with HSP27LNA (LNA, Locked nucleic acid) activity, the inventors observed that the endosomal escape/gene silencing enhancement factor is comparable, but at higher HSP27LNA concentrations compared to HSP27BNA (Figure 58).

All this shows that SO1861-Ald-EMCH efficiently enhances endosomal escape and cytoplasmic delivery of a targeted antisense BNA oligo as well as untargeted BNA/LNA oligos, thereby significantly reducing the effective concentration of the targeted and untargeted antisense oligo from µM range to low nM range.

### Materials

Trastuzumab (Tras, Herceptin^{®}, Roche), Cetuximab (Cet, Erbitux^{®}, Merck KGaA). Dianthin-cys was produced and purchased from Proteogenix, France, EGFdianthin was produced from *E.coli.* according to standard procedures. Cetuximab-saporin and trastuzumab-saporin conjugates were produced and purchased from Advanced Targeting Systems (San Diego, CA).

### Methods

### Flash chromatography

Grace Reveleris X2^{®} C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50 bar (725 psi); Detection: UV 200-400 nm, combination of up to 4 UV signals and scan of entire UV range, ELSD; Column sizes: 4-330 g on instrument, luer type, 750 g up to 3000 g with optional holder.

### HSP27BNA oligo sequences

HSP27 BNA oligo (5'-GGCacagccagtgGCG-3') according to Zhang *et al.* (2011) [Y Zhang, Z Qu, SKim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333]) ([SEQ-ID NO: 2]) was ordered with or without 5'-Thiol C6 linker at Bio-Synthesis Inc. (Lewisville, Texas). HSP27 LNA oliogo (5'-ggcacagccagtggcg-3') ([SEQ-ID NO: 3]) was ordered at at Bio-Synthesis Inc. (Lewisville, Texas).

### RNA isolation and gene expression analysis

RNA from cells was isolated and analysed according to standard protocols (Biorad). qPCR primers that were used are indicated in Table A10.

**Table A10. Primers used in qPCR are shown below:**

| Gene | Primer | Sequence (5'-3') | SEQ ID NO: |
|---|---|---|---|
| HSP27 | Forward | GCAGTCCAACGAGATCACCA | 4 |
| | Reverse | TAAGGCTTTACTTGGCGGCA | 5 |

### Tratuzumab-saporin and Cetuximab-saporin synthesis

Custom mAb-saporin conjugate were produced and purchased from Advanced Targeting Systems (San Diego, CA).

### Trastuzumab-dianthin and Cetuximab-dianthin synthesis

Dianthin-Cys (17.0 ml, ~9.6 mg) was concentrated by ultrafiltration using a vivaspin T15 filter tube (3,000 g, 20 °C, 10 minutes). The resulting 3.25 ml aliquot was gel filtered using zeba 10ml spin columns eluting with TBS pH 7.5.

Trastuzumab (mAb) or Cetuximab (mAb) (0.30 ml, ~10 mg) was diluted to 10 mg/ml with DPBS pH 7.5, desalted via zeba 5ml spin column eluting with DPBS pH 7.5 and normalised to 2.50 mg/ml. To an aliquot of mAb was added an aliquot of freshly prepared SMCC solution (1.00 mg/ml, 4.20 mole equivalents, 13.9 × 10⁻⁵ mmol) in DMSO, the mixture vortexed briefly then incubated for 60 minutes at 20°C with roller-mixing. After, the reaction was quenched by the addition of an aliquot of a freshly prepared glycine solution (2.0 mg/ml, 5.0 mole equivalents, 69.5 × 10⁻⁵ mmol) in DPBS pH 7.5. mAb-SMCC (4.27 mg, 2.80 × 10⁻⁵ mmol, 1.514 mg/ml) was obtained after gel filtration using a zeba 10ml spin column eluting with TBS pH 7.5.

To Dianthin-Cys (7.54 mg, 25.3 × 10⁻⁵ mmol, 2.258 mg/ml) was added an aliquot of freshly prepared TCEP solution (1.00 mg/ml, 0.5 mole equivalents, 12.6 × 10⁻⁵ mmol) in TBS pH 7.5, the mixture briefly vortexed then incubated for 60 minutes at 20°C with roller-mixing. After, Dianthin-SH (6.0 mg, 20.2 × 10⁻⁵ mmol, 1.722 mg/ml, Dianthin:SH = 1.1) was obtained by gel filtration using a zeba 10ml spin column eluting with TBS pH 7.5.

To the bulk mAb-SMCC was added the aliquot of Dianthin-SH (7.20 mole equivalents), the mixture vortexed briefly then incubated overnight at 20°C. After ca. 16 hours, the reaction was quenched by the addition of an aliquot of freshly prepared NEM solution (2.50 mg/ml, 5.0 mole equivalents, 101 × 10⁻⁵ mmol) in TBS pH 7.5. The reaction mixture was filtered to 0.45 µm and then concentrated to <2 ml by ultrafiltration using a vivaspin T15 filter tube (3,000 g, 20°C, 15 minutes). The conjugate was purified by gel filtration using a 1.6 × 35 cm Superdex 200PG column eluting with DPBS pH 7.5.

### Antibody-(L-HSP27 BNA)ⁿ [over HSP27 BNA disulfide]

### Trastuzumab-(L-HSP27)⁴,, Cetuximab-(L-HSP27)⁴, synthesis via PEG₄-SPDP with a DAR4 and Cetuximab-(L-HSP27)² synthesis via PEG₄-SPDP with a DAR2

Trastuzumab, Cetuximab, are referred hereafter as "Ab". Ab was conjugated to HSP27 BNA disulfide via a tetra(ethylene glycol) succinimidyl 3-(2-pyridyldithio)propionate (PEG₄-SPDP) linker forming a labile (L) disulfide bond between Ab and HSP27 BNA. The procedure is exemplary described for Trastuzumab-(L-HSP27 BNA)₄:
HSP27 BNA disulfide oligo (2.7 mg, 470 nmol, 6.10 mg/ml) was reacted with TCEP (10 mole equivalents, 4.7 µmol, 1.34 mg, 50 mg/ml) for 30 minutes at 20°C with roller mixing. After, the oligo-SH was purified by PD10 G25 desalting column eluting into TBS pH 7.5 and used promptly. Oligo-SH was obtained (2.48 mg, 90%, 1.24 mg/ml, SH to oligo ratio = 0.8)
Trastuzumab (1.5 mg, 10.3 nmol, 2.50 mg/ml) was reacted with an aliquot of freshly prepared PEG₄-SPDP solution (6.81 mole equivalents, 70.1 nmol, 39 µg) in DMSO (1 mg/ml) for 60 minutes at 20°C with roller mixing. After, the reaction was quenched with glycine (15.1 µl of 2 mg/ml freshly prepared solution in TBS pH 7.5) and then desalted via zeba desalting column eluting with TBS pH 7.5. An aliquot of the resulting Tras-S-PEG₄-SPDP was taken out and tested by UV-Vis analysis. SPDP incorporation was determined using TCEP to liberate pyridiyl-2-thione (PDT) and by UV-vis analysis at 343 nm (SPDP to Ab ratio: 4). The remaining Tras-(S-PEG₄-SPDP)₄ was reacted with an aliquot of freshly prepared HSP27 oligonucleotide (oligo-SH) (8 mole equivalents, 82.4 nmol, 1.24 mg/ml) and incubated overnight at 20°C with roller mixing. After 17 hours, the conjugate was analysed by UV-vis analysis to ascertain incorporation of HSP27 by displacement of pyridiyl-2-thione (PDT) at 343 nm. The crude conjugate was purified using a 1.6 × 33 cm Sephadex G50 column eluting with DPBS pH 7.5. The resulting Trastuzumab-(L-HSP27)₄ was obtained as a single fraction. Yield: n.d.. Purity: 96% , HSP27 BNA to Ab ratio = 4.4

### EXAMPLE 6 - endosomal escape enhancing activity of saponins

Previously, the efficacy of various saponins (SO1861, SA1642) were co administrated as 'free' unconjugated molecules to cells in combination with a ligand toxin fusion (e.g. EGFdianthin) or an antibody-protein toxin conjugate, resulting in enhanced cell killing activity of target expressing cells. Here, three different saponin molecules (SO1861, SO1862 (isomer of SO1861), SO1832 and SO1904) isolated from a root extract of *Saponaria officinalis* were titrated in the presence and absence of a non-effective fixed concentration of 1.5 pM EGFdianthin on HeLa (EGFR⁺) cells. This revealed a strong enhancement of cell killing activity for all tested saponin variants (IC50 = 300 nM; Figure 63A) compared to the treatments without EGFdianthin. Next, EGFdianthin was titrated with a fixed concentration of saponin (~1000 nM) and this revealed strong targeted cell killing enhancement at low pM concentrations of EGFdianthin (IC50 = 0.4 pM; Figure 63B), observed for all used saponins SO1861, SO1862 (isomer of SO1861), SO1832 and SO1904. EGF-dianthin alone could only induce cell killing at very high concentrations (IC50 = 10.000 pM). This shows that these specific types of saponins, all have the intrinsic capacity to efficiently induce endosomal escape with only a very low amount of targeted toxin available.

To extend this test, saponins from other sources were analyzed. A saponin purified from a root extract of *Gypsophila elegans* M.Bieb. (GE1741) was titrated on HeLa cells in the presence and absence of 1.5 pM EGFdianthin and compared with purified SO1861. GE1741 also enhances the EGFdianthin induced HeLa cell killing, but shows slightly less efficacy compared to SO1861. (GE1741 IC50 = 800 nM; Figure 63C) and also displays a higher general toxicity (IC50 = 5.000 nM in absence of EGFdianthin; Figure 63C). A similar test in which different partially purified mixtures of *Quillaja saponaria* saponins (QSmix 1-3) were co-administrated with 1.5 pM EGFdianthin on HeLa cells, revealed for 2 out of 3 (QSmix 1 and QSmix 3) similar activity as for SO1861 (IC50 QSmix/QSmix3 = 300 nM; Figure 63D). QSmix (2) is less efficient in enhancing 1.5 pM EGFdianthin induced cell killing (IC50 = 2000 nM; Figure 63D), however, no general toxicity is observed. This shows that also in QS extracts, specific type of saponins are available that efficiently induce endosomal escape of the targeting ligand toxin EGFdianthin. Hence, the saponins described in this example, such as *Quillaja saponaria* saponins, GE1741, SO1861, SO1862, SO1832 and SO1904 are particularly attractive saponins to derivatise according to the present invention.

### EXAMPLE 7 - endosomal escape enhancing activity of saponins and saponin derivatives

Labile/acid sensitive derivatisations (Ald-EMCH or SO1861-L-N₃ (also referred to as SO1861-N3 and SO1861-azide or SO1861-N3/azide), were applied to SO1861 via the aldehyde group, producing SO1861-Ald-EMCH or SO1861-L-N₃. To verify the activity of SO1861-Ald-EMCH the molecule was titrated in the presence and absence of a fixed non-effective (1.5 pM) EGFdianthin concentration on EGFR expressing (A431, HeLa) and non-expressing cells (A2058). In all three cell lines SO1861 alone showed a strong cell viability reduction, whereas SO1861-Ald-EMCH as single compound showed no toxicity up to 25.000 nM (Figure 64A-C). When SO1861-Ald-EMCH was combined with 1.5 pM EGFdianthin a strong target specific cell viability reduction is observed in the EGFR⁺ A431 and HeLa cells (IC50 = 3.000 nM; Figure 64A,B), while the EGFR⁻ A2058 cells are not affected at all (Figure 64C). Similar results were obtained for SO1861-L-N₃. SO1861-L-N₃ co-administrated with 1.5 pM EGFdianthin also shows efficient cell killing on A431 and HeLa cells (IC50 = 3.000 nM), but without EGFdianthin a general toxicity is observed at above 10.000 nM (Figure 64D, 64E).

HATU was conjugated to SO1861 via the carboxylic acid group of SO1861 producing, SO1861-(S), also referred to as SO1861-HATU and SO1861-Glu-HATU. To determine the activity, different concentrations of SO1861-(S) were co-administrated with 1.5 pM EGFdianthin and tested for cell killing activity in EGFR expressing HeLa cells. SO1861-(S) showed a similar activity as SO1861, indicating that conjugation to the carboxylic acid group does not affect the endosomal escape enhancing potency of the molecule, similar to what is observed with SO1861-Ald-EMCH (Figure 65).

## Claims

1. Saponin derivative based on a SO1861 saponin comprising a triterpene aglycone core structure and a first saccharide chain and a second saccharide chain linked to the aglycone core structure, wherein the saponin derivative is a SO1861 derivative having a quillaic acid aglycone core structure, wherein the saponin derivative corresponds to the saponin represented by Molecule 1: 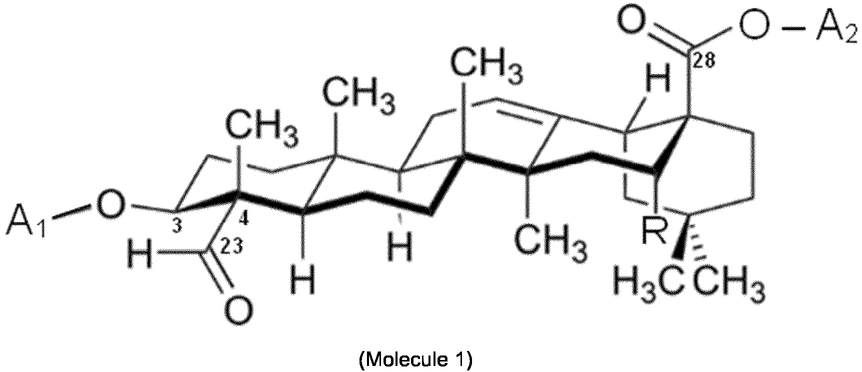 wherein the aglycone core structure of Molecule 1 is quillaic acid and R is defined as hydroxyl; wherein the first saccharide chain A1 is Gal-(1→2)-[Xyl-(1→3)]-GlcA- and the second saccharide chain A2 is Glc-(1→3)-Xyl-(1→4)-Rha-(1→2)-[Xyl-(1→3)-4-OAc-Qui-(1→4)]-Fuc-; and wherein:
i. the saponin derivative comprises said aglycone core structure comprising an aldehyde group at position C23 of the quillaic acid which has been derivatised; or
ii. the first saccharide chain A1 comprises a carboxyl group of a glucuronic acid moiety, which has been derivatised; or
iii. the second saccharide chain A2 comprises an acetoxy (Me(CO)O-) group which has been derivatised; or
iv. the saponin derivative comprises any combination of derivatisations i., ii. and iii., preferably any combinations of two derivatisations i., ii. and iii.

2. Saponin derivative according to claim 1, wherein the saponin derivative comprises both of (ii.) said first saccharide chain which has been derivatised and (iii.) said second saccharide chain which has been derivatised,
preferably, the saponin derivative comprises both of said first saccharide chain which has been derivatised and said second saccharide chain which has been derivatised and (i.) an aglycone core structure being quillaic acid comprising an aldehyde group which has been derivatised.

3. Saponin derivative according to claim 1 or 2, wherein at least one of the following derivatisations is present, preferably one or two of the following derivatisations is present, more preferably one:
i. the aldehyde group at position C23 of the quillaic acid has been derivatised by;
- reduction to an alcohol;
- transformation into a hydrazone bond, preferably through reaction with a hydrazide;
ii. the carboxyl group of the glucuronic acid moiety has been derivatised by transformation into an amide bond, preferably through reaction with an amine; and
iii. the acetoxy group, has been derivatised by transformation into a hydroxyl group (HO-) by deacetylation.

4. Saponin derivative according to any one of the claims 1-3, wherein the saponin derivative has a molecular weight of less than 2,500 g/mol, preferably less than 2,300 g/mol, more preferably less than 2,150 g/mol.

5. Saponin derivative according to any one of the claims 1-4, wherein at least one of the following derivatisations is present, preferably one or two of the following derivatisations is present:
i. the aldehyde group at position C₂₃ of the quillaic acid has been derivatised by;
- reduction to an alcohol;
- transformation into a hydrazone bond through reaction with N-ε-maleimidocaproic acid hydrazide (EMCH), therewith providing SO1861-Ald-EMCH, wherein the maleimide group of the EMCH is optionally derivatised by formation of a thioether bond with mercaptoethanol;
- transformation into a hydrazone bond through reaction with N-[β-maleimidopropionic acid] hydrazide (BMPH) wherein the maleimide group of the BMPH is optionally derivatised by formation of a thioether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. the carboxyl group of said glucuronic acid moiety of the first saccharide chain has been derivatised by transformation into an amide bond through reaction with 2-amino-2-methyl-1,3-propanediol (AMPD) or N-(2-aminoethyl)maleimide (AEM), therewith providing a SO1861-Glu-AMPD or a SO1861-Glu-AEM; and
iii. the acetoxy group of the second saccharide moiety has been derivatised by transformation into a hydroxyl group (HO-) by deacetylation.

6. Saponin derivative of any one of the claims 1-5, wherein the saponin derivative is a SO1861 derivative comprising a single derivatisation, wherein the single derivatisation is transformation of a carboxyl group of a glucuronic acid moiety of SO1861 by binding 1-[Bis(dimethylamino)methylene]-1H-1,2,3-triazolo[4,5-b]pyridinium 3-oxid hexafluorophosphate (HATU) to the carboxyl group of the glucuronic acid moiety of SO1861 or by binding (benzotriazol-1-yloxy)tris(dimethylamino)phosphonium hexafluorophosphate (BOP) to the carboxyl group of the glucuronic moiety of SO1861, or wherein the saponin derivative is a SO1861 derivative represented by Molecule 2, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH): 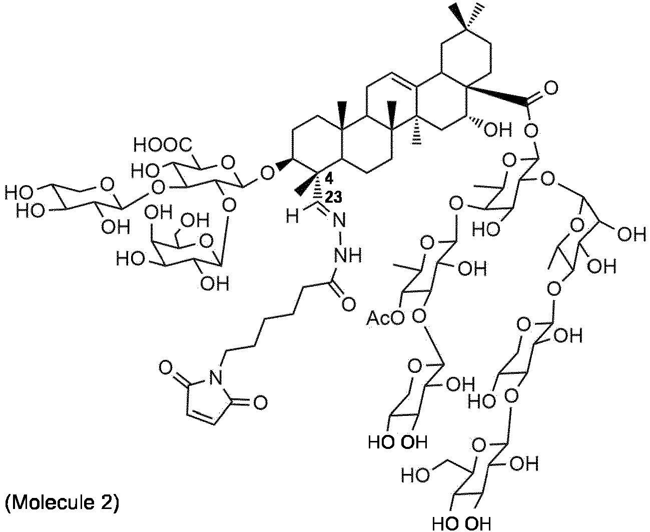 , or wherein the saponin derivative is a SO1861 derivative represented by Molecule 3, which represents a SO1861 derivative comprising an aldehyde group at indicated position C₂₃ of the quillaic acid aglycone core structure which has been derivatised by transformation into a hydrazone bond through reaction with N-ε-maleimidocaproic acid hydrazide (EMCH) wherein the maleimide group of the EMCH is derivatised with mercaptoethanol therewith forming a thioether bond:

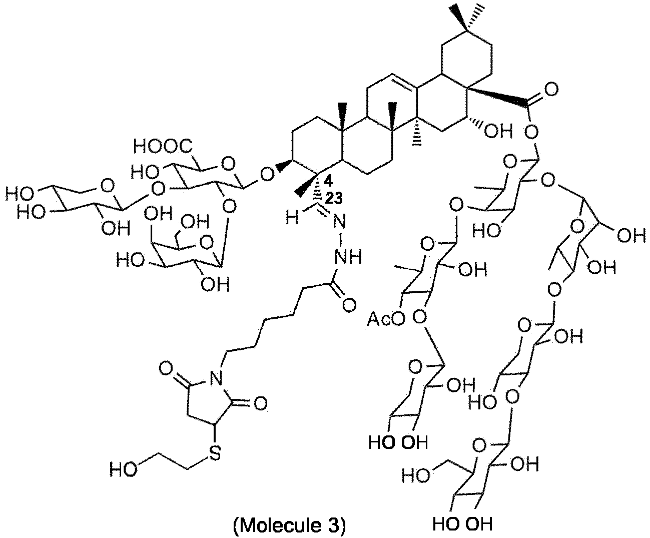

7. Saponin derivative according to any one of the claims 1-6, wherein
i. the saponin derivative comprises said aglycone core structure wherein the aglycone core structure comprises an aldehyde group which has been derivatised by:
- reduction to an alcohol;
- transformation into a hydrazone bond through reaction with N-ε-maleimidocaproic acid hydrazide (EMCH) wherein the maleimide group of the EMCH is optionally derivatised by formation of a thioether bond with mercaptoethanol;
- transformation into a hydrazone bond through reaction with N-[β-maleimidopropionic acid] hydrazide (BMPH) wherein the maleimide group of the BMPH is optionally derivatised by formation of a thioether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. the first saccharide chain comprises said carboxyl group, preferably a carboxyl group of a glucuronic acid moiety, which has been derivatised by transformation into an amide bond through reaction with 2-amino-2-methyl-1,3-propanediol (AMPD) or *N-(2-*aminoethyl)maleimide (AEM);
iii. the second saccharide chain comprises said acetoxy group (Me(CO)O-) which has been derivatised by transformation into a hydroxyl group (HO-) by deacetylation; or
iv. the saponin derivative comprises any combination of two or three derivatisations i., ii. and iii., preferably any combination of two derivatisations i., ii. and iii.;
preferably, the saponin derivative comprises said aglycone core structure wherein the aglycone core structure comprises an aldehyde group which has been derivatised by transformation into a hydrazone bond through reaction with EMCH wherein the maleimide group of the EMCH is optionally derivatised by formation of a thioether bond with mercaptoethanol.

8. Saponin derivative according to claim 7, wherein the saponin derivative comprises said aglycone core structure wherein the aglycone core structure comprises an aldehyde group and wherein the first saccharide chain comprises a carboxyl group, preferably a carboxyl group of a glucuronic acid moiety, which has been derivatised by transformation into an amide bond through reaction with N-(2-aminoethyl)maleimide (AEM).

9. Saponin derivative according to claim 8, with the proviso that when the aldehyde group in the aglycone core structure is derivatised by transformation into a hydrazone bond through reaction with N-ε-maleimidocaproic acid hydrazide (EMCH), at least one of the glucuronic acid and the acetoxy group (Me(CO)O-) is also derivatised, and with the proviso that when the glucuronic acid moiety of SO1861 is derivatised by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, at least one of the aldehyde group and the acetoxy group (Me(CO)O-) is also modified.

10. Saponin derivative of claim 9, with the proviso that when the aldehyde group in the aglycone core structure of the saponin derivative is derivatised through reaction with EMCH, at least one of the glucuronic acid and the acetoxy group (Me(CO)O-) is also derivatised, and with the proviso that when the carboxyl group of the glucuronic acid moiety of SO1861 is derivatised by bound HATU, at least one of the aldehyde group and the acetoxy group (Me(CO)O-) is also derivatised.

11. First pharmaceutical composition comprising the saponin derivative according to any one of the claims 1-10 and optionally a pharmaceutically acceptable excipient and/or diluent.

12. First pharmaceutical composition of claim 11, wherein the saponin derivative is the saponin derivative represented by Molecule 2: 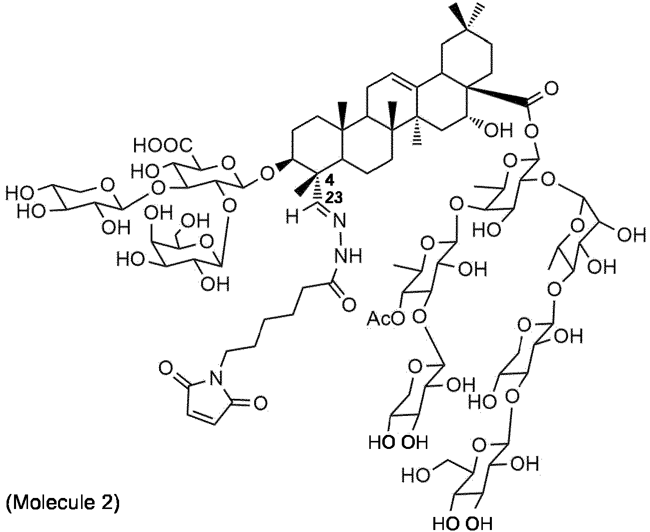 or SO1861 derivative comprising a single derivatisation, wherein the single derivatisation is transformation of the carboxyl group of the glucuronic acid moiety of SO1861 by reaction of 1-[Bis(dimethylamino)methylene]-1H-1,2,3-triazolo[4,5-b]pyridinium 3-oxid hexafluorophosphate (HATU) with the carboxyl group of the glucuronic acid moiety of SO1861, or the saponin derivative is the saponin derivative represented by Molecule 3:

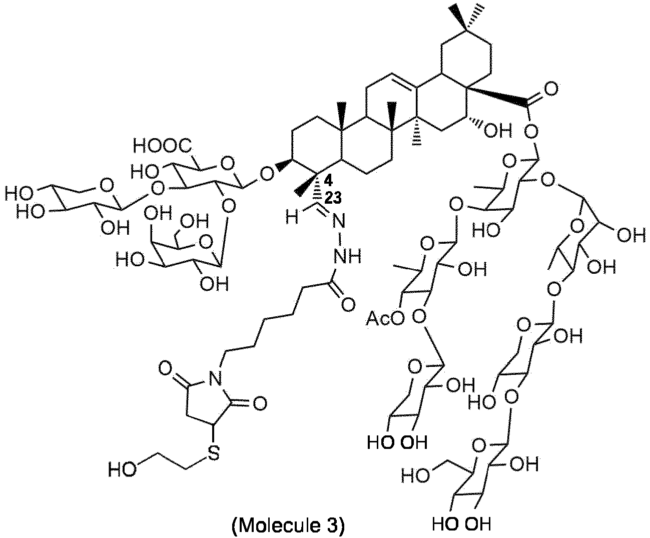

13. Pharmaceutical combination comprising:
• the first pharmaceutical composition of claim 11 or 12; and
• a second pharmaceutical composition comprising any one or more of an antibody-toxin conjugate, a receptor-ligand - toxin conjugate, an antibody-drug conjugate, a receptor-ligand - drug conjugate, an antibody-oligonucleotide conjugate or a receptor-ligand - oligonucleotide conjugate, and optionally comprising a pharmaceutically acceptable excipient and/or diluent.

14. Third pharmaceutical composition comprising the saponin derivative of any one of the claims 1-10 and further comprising any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate, and optionally comprising a pharmaceutically acceptable excipient and/or diluent.

15. Pharmaceutical combination of claim 13 or the third pharmaceutical composition of claim 14, wherein the second pharmaceutical composition or the third pharmaceutical composition comprises any one or more of an antibody-drug conjugate, a receptor-ligand - drug conjugate, an antibody-oligonucleotide conjugate or a receptor-ligand - oligonucleotide conjugate, wherein the drug is for example a toxin such as saporin and dianthin, and wherein the oligonucleotide is for example an siRNA or a BNA, for example for gene silencing of apolipoprotein B or HSP27.

16. Pharmaceutical combination of claim 13 or 15 or the third pharmaceutical composition of claim 14 or 15, wherein the antibody-drug conjugate is a conjugate of an antibody such as an IgG, a Fab, an scFv, an immunoglobulin, an immunoglobulin fragment, one or multiple VH domains, single-domain antibodies, a V_{HH} or a camelid V_{H}.

17. Pharmaceutical combination of claim 13 or 15-16or the third pharmaceutical composition of any one of the claims 14-16, wherein the saponin derivative is represented by Molecule 2 or Molecule 3.

18. First pharmaceutical composition of claim 11 or 12, pharmaceutical combination of any one of the claims 13 or 15-17 or the third pharmaceutical composition of any one of the claims 14-17, for use as a medicament.

19. First pharmaceutical composition of claim 11 or 12, pharmaceutical combination of any one of the claims 13 or 15-17 or the third pharmaceutical composition of any one of the claims 14-17, for use in the treatment or prophylaxis of a cancer, an infectious disease, viral infection, hypercholesterolemia, primary hyperoxaluria, haemophilia A, haemophilia B, alpha-1 antitrypsin related liver disease, acute hepatic porphyria, transthyretin-mediated amyloidosis, or an auto-immune disease.

20. *In vitro* or *ex vivo* method for transferring a molecule from outside a cell to inside said cell, preferably into the cytosol of said cell, comprising the steps of:
a) providing a cell;
b) providing the molecule for transferring from outside the cell into the cell provided in step a);
c) providing a saponin derivative according to any one of the claims 1-10;
d) contacting the cell of step a) *in vitro* or *ex vivo* with the molecule of step b) and the saponin derivative of step c), therewith establishing the transfer of the molecule from outside the cell into said cell.

21. The method of claim 20, wherein the cell is a human cell such as a T-cell, an NK-cell, a tumor cell and/or wherein the saponin derivative is the saponin derivative of any one of the claims 5-10, and/or wherein the molecule of step b) is any one of: an antibody-drug conjugate, a receptor-ligand - drug conjugate, an antibody-oligonucleotide conjugate or a receptor-ligand - oligonucleotide conjugate, wherein the drug is for example a toxin and wherein the oligonucleotide is for example an siRNA or a BNA.

## Patentansprüche

1. Saponinderivat basierend auf einem SO1861 Saponin, das eine Triterpen-Aglykon-Kernstruktur und eine erste Saccharidkette und eine zweite Saccharidkette, die mit der Aglykon-Kernstruktur verbunden sind, umfasst, wobei das Saponinderivat ein SO1861 Derivat ist, das eine Quillajasäure-Aglykon Kernstruktur aufweist, wobei das Saponinderivat dem durch Molekül 1 dargestellten Saponin entspricht: 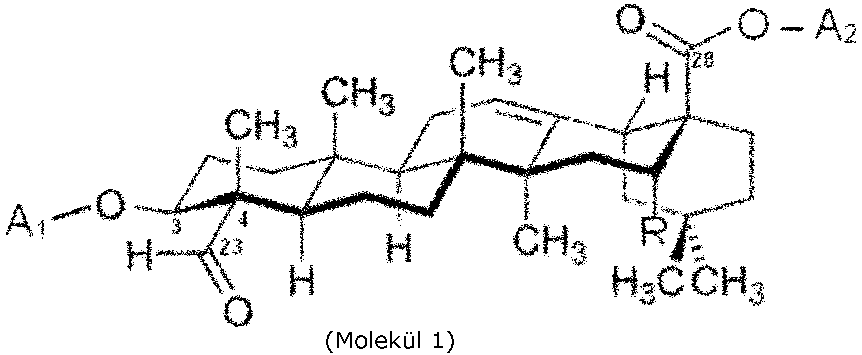 wobei die Aglykon-Kernstruktur von Molekül 1 Quillajasäure ist und R als Hydroxyl definiert ist; wobei die erste Saccharidkette A1 Gal-(1→2)-[Xyl-(1 →3)]1-GlcA ist und die zweite Saccharidkette A2 Glc-(1→3)-Xyl-(1→4)-Rha-(1→2)-[Xyl-(1→3)-4-OAc-Qui-(1→4)]-Fuc ist; und wobei:
i. das Saponinderivat besagte Aglykon-Kernstruktur umfasst, umfassend eine Aldehydgruppe in Position C23 der Quillajasäure, welche derivatisiert wurde; oder
ii. die erste Saccharidkette A1 eine Carboxylgruppe einer Glucuronsäureeinheit umfasst, welche derivatisiert wurde; oder
iii. die zweite Saccharidkette A2 eine Acetoxy (Me(CO)O-) Gruppe umfasst, welche derivatisiert wurde; oder
iv. das Saponinderivat eine beliebige Kombination der Derivatisierungen i., ii. und iii. umfasst, bevorzugt eine beliebige Kombination von zwei Derivatisierungen i., ii. und iii.

2. Saponinderivat nach Anspruch 1, wobei das Saponinderivat beides aus (ii) besagter erster Saccharidkette, welche derivatisiert wurde, und (iii) besagter zweite Saccharidkette, welche derivatisiert wurde, umfasst, bevorzugt umfasst das Saponinderivat beides aus besagter erster Saccharidkette, welche derivatisiert wurde, und besagter zweite Saccharidkette, welche derivatisiert wurde, und (i.) eine Aglykon-Kernstruktur, die Quillajasäure ist, umfassend eine Aldehydgruppe, welche derivatisiert wurde.

3. Saponinderivat nach Anspruch 1 oder 2, wobei mindestens eine der folgenden Derivatisierungen vorhanden ist, bevorzugt eine oder zwei der folgenden Derivatisierungen vorhanden ist, mehr bevorzugt eine:
i. die Aldehydgruppe in Position C23 der Quillajasäure wurde derivatisiert durch;
- Reduktion zu einem Alkohol;
- Transformation in eine Hydrazonbindung, bevorzugt mittels Reaktion mit einem Hydrazid;
ii. die Carboxylgruppe der Glucuronsäureeinheit wurde durch Transformation in eine Amidbindung derivatisiert, bevorzugt mittels Reaktion mit einem Amin; und
iii. die Acetoxygruppe wurde durch Transformation in eine Hydroxygruppe (HO-) durch Deacetylierung derivatisiert.

4. Saponinderivat nach einem der Ansprüche 1-3, wobei das Saponinderivat ein Molekulargewicht von weniger als 2500 g/mol, bevorzugt weniger als 2300 g/mol, mehr bevorzugt weniger als 2150 g/mol aufweist.

5. Saponinderivat nach einem der Ansprüche 1-4, wobei mindestens eine der folgenden Derivatisierungen vorhanden ist, bevorzugt eine oder zwei der folgenden Derivatisierungen vorhanden ist:
i. die Aldehydgruppe in Position C₂₃ der Quillajasäure wurde derivatisiert durch;
- Reduktion zu einem Alkohol;
- Transformation in eine Hydrazonbindung mittels Reaktion mit N-ε-Maleimidocapronsäurehydrazid (EMCH), dabei SO1861-AldEMCH bereitstellend, wobei die Maleimidgruppe des EMCH optional durch Bildung einer Thioetherbindung mit Mercaptoethanol derivatisiert ist;
- Transformation in eine Hydrazonbindung mittels Reaktion mit N-β-Maleimidopropionsäurehydrazid (BMPH), wobei die Maleimidgruppe des BHPH optional durch Bildung einer Thioetherbindung mit Mercaptoethanol derivatisiert ist; oder
- Trasnformation in eine Hydrazonbindung mittels Reaktion mit N-κ-Maleimidoundecansäurehydrazid (KMUH), wobei die Maleimidgruppe des KMUH optional durch Bildung einer Thioetherbindung mit Mercaptoethanol derivatisiert ist;
ii. die Carboxylgruppe der Glucuronsäureeinheit der ersten Saccharidkette wurde durch Transformation in eine Amidbindung mittels Reaktion mit 2-Amino-2-methyl-1,3-propandiol (AMPD) oder N-(2-aminomethyl)maleimid (AEM) derivatisiert, dabei ein SO1861-Glu-AMPD oder ein SO1861-Glu-AEM bereitstellend; und
iii. die Acetoxygruppe der zweiten Saccharideinheit wurde durch Transformation in eine Hydroxygruppe (HO-) durch Deacetylierung derivatisiert.

6. Saponinderivat nach einem der Ansprüche 1-5, wobei das Saponinderivat ein SO1861 Derivat ist, das eine einzelne Derivatisierung umfasst, wobei die einzelne Derivatisierung die Transformation einer Carboxylgruppe einer Glucuronsäureeinheit von SO1861 durch Bindung von 1-[Bis(dimethylamino)methylen]-1H-1,2,3-triazol[4,5-b]pyridinium-3-oxid-hexafluorophosphat (HATU) an die Carboxylgruppe des Glucuronsäureeinheit von SO1861 oder durch Bindung von (Benzotriazol-1-yloxy)tris(dimethylamino)phosphoniumhexafluorophosphat (BOP) an die Carboxylgruppe der Glucuronsäureeinheit von SO1861 ist, oder wobei das Saponinderivat ein durch Molekül 2 dargestelltes SO1861 Derivat ist, welches ein SO1861 Derivat darstellt, das eine Aldehydgruppe an angegebener Position C₂₃ der Quillajasäure-Aglycon-Kernstruktur umfasst, welche durch Transformation in eine Hydrazonbindung mittels Reaktion mit N-ε-Maleimidocapronsäurehydrazid (EMCH) derivatisiert wurde: 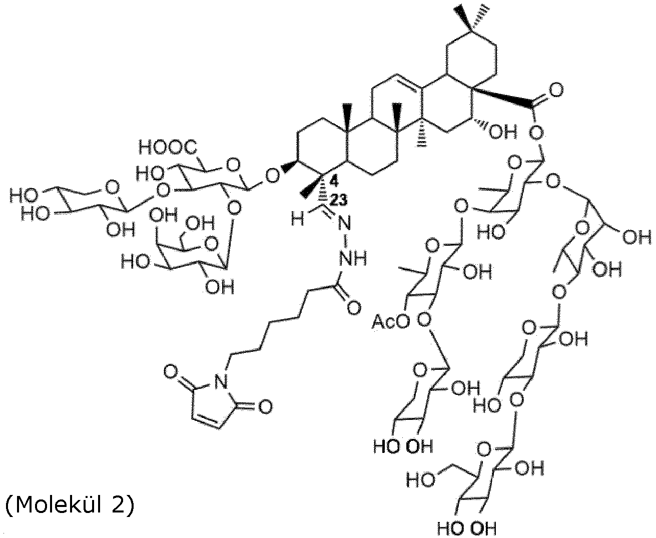 , oder wobei das Saponinderivat ein durch Molekül 3 dargestelltes SO1861 Derivat ist, welches ein SO1861 Derivat darstellt, das eine Aldehydgruppe an angegebener Position C₂₃ der Quillajasäure-Aglycon-Kernstruktur umfasst, welche durch Umwandlung in eine Hydrazonbindung mittels Reaktion mit N-ε-Maleimidocapronsäurehydrazid (EMCH) derivatisiert wurde, wobei die Maleimidgruppe des HMCHs mit Mercaptoethanol derivatisiert ist, damit eine Thioetherbindung bildend:

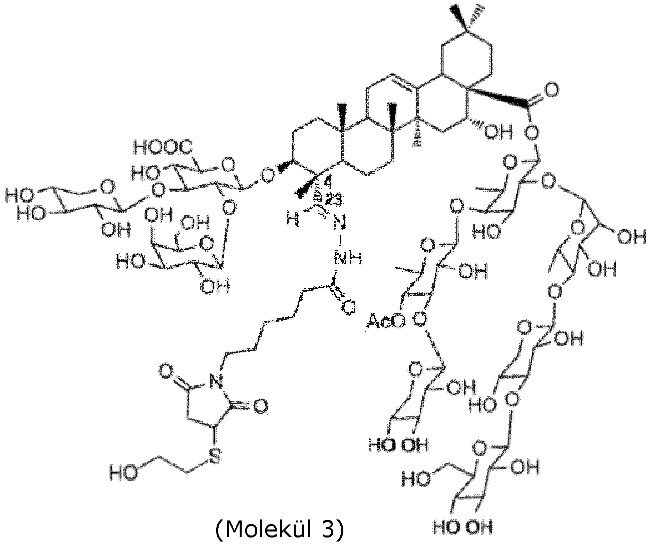

7. Saponinderivat nach einem der Ansprüche 1-6, wobei
i. das Saponinderivat besagte Aglykon-Kernstruktur umfasst, wobei die Aglykon-Kernstruktur eine Aldehydgruppe umfasst, welche derivatisiert wurde durch:
- Reduktion zu einem Alkohol;
- Transformation in eine Hydrazonbindung mittels Reaktion mit N-ε-Maleimidocapronsäurehydrazid (EMCH), wobei die Maleimidgruppe des EMCHs optional durch Bildung einer Thioetherbindung mit Mercaptoethanol derivatisiert ist;
- Transformation in eine Hydrazonbindung mittels Reaktion mit N-β-Maleimidopropionsäurehydrazid (BMPH), wobei die Maleimidgruppe des BMPHs optional durch Bildung einer Thioetherbindung mit Mercaptoethanol derivatisiert ist; oder
- Transformation in eine Hydrazonbindung mittels Reaktion mit N-κ-Maleimidoundecansäurehydrazid (KMUH), wobei die Maleimidgruppe des KMUHs optional durch Bildung einer Thioetherbindung mit Mercaptoethanol derivatisiert ist;
ii. die erste Saccharidkette besagte Carboxylgruppe umfasst, bevorzugt eine Carboxylgruppe einer Glucuronsäureeinheit, welche durch Transformation in eine Amidbindung mittels Reaktion mit 2-Amino-2-methyl-1,3-propandiol (AMPD) oder N-(2-aminomethyl)maleimid (AEM) derivatisiert wurde;
iii. die zweite Saccharidkette besagte Acetoxygruppe (Me(CO)O-) umfasst, welche durch Transformation in eine Hydroxygruppe (OH-) durch Deacetylierung derivatisiert wurde; oder
iv. das Saponinderivat eine beliebige Kombination aus zwei oder drei Derivatisierungen i., ii. und iii. umfasst, bevorzugt eine beliebige Kombination von zwei Derivatisierungen i., ii. und iii;
bevorzugt umfasst das Saponinderivat besagte Aglykon-Kernstruktur, wobei die Aglykon-Kernstruktur eine Aldehydgruppe umfasst, welche durch Transformation in eine Hydrazonbindung mittels Reaktion mit EMCH derivatisiert wurde, wobei die Maleimidgruppe des EMCHs optional durch Bildung einer Thioetherbindung mit Mercaptoethanol derivatisiert ist.

8. Saponinderivat nach Anspruch 7, wobei das Saponinderivat besagte Aglykon-Kernstruktur umfasst, wobei die Aglykon-Kernstruktur eine Aldehydgruppe umfasst, und wobei die erste Saccharidkette eine Carboxylgruppe umfasst, bevorzugt eine Carboxylgruppe einer Glucuronsäureeinheit, welche durch Transformation in eine Amidbindung mittels Reaktion mit N-(2-aminomethyl)maleimid (AEM) derivatisiert wurde.

9. Saponinderivat nach Anspruch 8, mit der Maßgabe, dass, wenn die Aldehydgruppe in der Aglykon-Kernstruktur durch Transformation in eine Hydrazonbindung mittels Reaktion mit N-ε-Maleimidocapronsäurehydrazid (EMCH) derivatisiert ist, mindestens eine der Glucuronsäure und der Acetoxygruppe (Me(CO)O-) ebenfalls derivatisiert ist, und mit der Maßgabe, dass, wenn die Glucuronsäureeinheit von SO1861 durch Reaktion von 1-[Bis(dimethylamino)methylen]-1H-1,2,3-triazol[4,5-b]pyridinium-3-oxid-hexafluorophosphat (HATU) mit der Carboxylgruppe der Glucuronsäureeinheit von SO1861 derivatisiert ist, mindestens eine der Aldehydgruppe und der Acetoxygruppe (Me(CO)O-) ebenfalls modifiziert ist.

10. Saponinderivat nach Anspruch 9, mit der Maßgabe, dass, wenn die Aldehydgruppe in der Aglykon-Kernstruktur des Saponinderivats mittels Reaktion mit EMCH derivatisiert ist, mindestens eine der Glucuronsäure und der Acetoxygruppe (Me(CO)O-) ebenfalls derivatisiert ist, und mit der Maßgabe, dass, wenn die Carboxylgruppe der Glucuronsäureeinheit von SO1861 durch gebundenes HATU derivatisiert ist, mindestens eine der Aldehydgruppe und der Acetoxygruppe (Me(CO)O-) ebenfalls derivatisiert ist.

11. Erste pharmazeutische Zusammensetzung, die das Saponinderivat nach einem der Ansprüche 1-10 und optional einen pharmazeutisch akzeptablen Hilfsstoff und/oder ein Verdünnungsmittel umfasst.

12. Erste pharmazeutische Zusammensetzung nach Anspruch 11, wobei das Saponinderivat ist das durch Molekül 2 dargestellte Saponinderivat 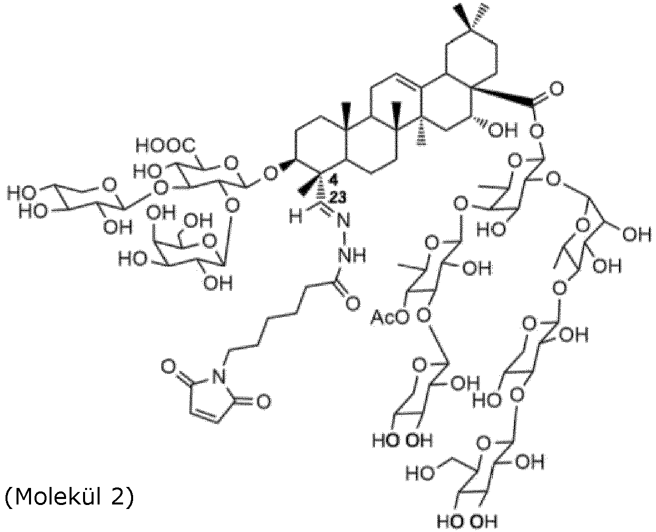 oder SO1861 Derivat, das eine einzelne Derivatisierung umfasst, wobei die einzelne Derivatisierung die Transformation der Carboxylgruppe der Glucuronsäureeinheit von SO1861 durch Reaktion von 1-[Bis(dimethylamino)methylen]-1H-1,2,3-triazol[4,5-b]pyridinium-3-oxid-hexafluorophosphat (HATU) mit der Carboxylgruppe der Glucuronsäureeinheit von SO1861 ist, oder das Saponinderivat das durch Molekül 3 dargestellte Saponinderivat ist:

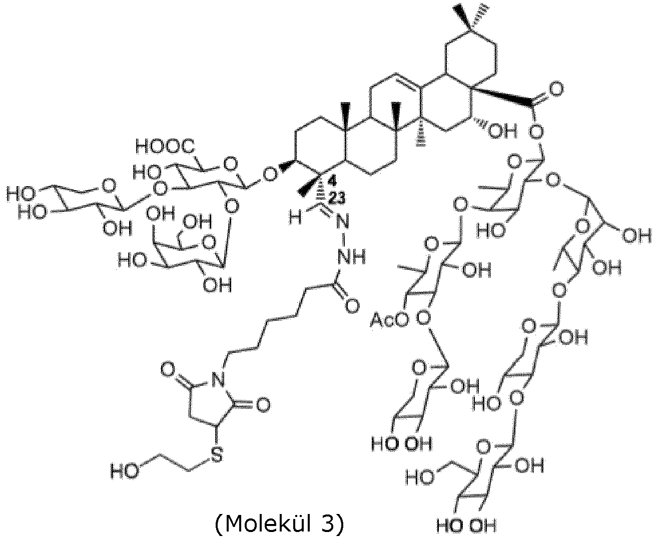

13. Pharmazeutische Kombination, umfassend:
• die erste pharmazeutische Zusammensetzung nach Anspruch 11 oder 12; und
• eine zweite pharmazeutische Zusammensetzung, die eines oder mehrere aus einem Antikörper-Toxin-Konjugat, einem Rezeptor-Ligand-Toxin-Konjugat, einem Antikörper-Arzneimittel-Konjugat, einem Rezeptor-Ligand-Arzneimittel-Konjugat, einem Antikörper-Oligonukleotid-Konjugat oder einem Rezeptor-Ligand-Oligonukleotid-Konjugat umfasst, und optional einen pharmazeutisch akzeptablen Hilfsstoff und/oder ein Verdünnungsmittel umfasst.

14. Dritte pharmazeutische Zusammensetzung, die das Saponinderivat nach einem der Ansprüche 1-10 umfasst und ferner eines oder mehrere umfasst von: einem Antikörper-Toxin-Konjugat, einem Rezeptor-Ligand-Toxin-Konjugat, einem Antikörper-Arzneimittel-Konjugat, einem Rezeptor-Ligand-Arzneimittel-Konjugat, einem Antikörper-Nukleinsäure-Konjugat oder einem Rezeptor-Ligand-Nukleinsäure-Konjugat, und optional einen pharmazeutisch akzeptablen Hilfsstoff und/oder ein Verdünnungsmittel umfasst.

15. Pharmazeutische Kombination nach Anspruch 13 oder die dritte pharmazeutische Zusammensetzung nach Anspruch 14, wobei die zweite pharmazeutische Zusammensetzung oder die dritte pharmazeutische Zusammensetzung eines oder mehrere aus einem Antikörper-Arzneimittel-Konjugat, einem Rezeptor-Ligand-Arzneimittel-Konjugat, einem Antikörper-Oligonukleotid-Konjugat oder einem Rezeptor-Ligand-Oligonukleotid-Konjugat umfasst, wobei das Arzneimittel zum Beispiel ein Toxin, wie etwa Saporin und Dianthin, ist, und wobei das Oligonukleotid zum Beispiel eine siRNA oder eine BNA ist, zum Beispiel zum Gen-Silencing von Apolipoprotein B oder HSP27.

16. Pharmazeutische Kombination nach Anspruch 13 oder 15 oder die dritte pharmazeutische Zusammensetzung nach Anspruch 14 oder 15, wobei das Antikörper-Arzneimittel-Konjugat ein Konjugat eines Antikörpers, wie etwa ein IgG, ein Fab, ein scFv, ein Immunoglobulin, ein Immunoglobulin-Fragment, eine oder mehrere VH Domänen, Einzeldomänenantikörper, ein V_{HH} oder ein Camelid V_{H} ist.

17. Pharmazeutische Kombination nach Anspruch 13 oder 15-16 oder die dritte pharmazeutische Zusammensetzung nach einem der Ansprüche 14-16, wobei das Saponinderivat durch Molekül 2 oder Molekül 3 dargestellt ist.

18. Erste pharmazeutische Zusammensetzung nach Anspruch 11 oder 12, pharmazeutische Kombination nach einem der Ansprüche 13 oder 15-17 oder die dritte pharmazeutische Zusammensetzung nach einem der Ansprüche 14-17, zur Verwendung als ein Medikament.

19. Erste pharmazeutische Zusammensetzung nach Anspruch 11 oder 12, pharmazeutische Kombination nach einem der Ansprüche 13 oder 15-17 oder die dritte pharmazeutische Zusammensetzung nach einem der Ansprüche 14-17 zur Verwendung bei der Behandlung oder Prophylaxe von einem Krebs, einer Infektionskrankheit, Virusinfektion, Hypercholesterinämie, primärer Hyperoxalurie, Hämophilie A, Hämophilie B, Alpha-1-Antitrypsin-bedingter Lebererkrankung, akuter hepatischer Porphyrie, Transthyretin-vermittelter Amyloidose oder einer Autoimmunerkrankung.

20. *In vitro-* oder ex vivo-Verfahren zum Transferieren eines Moleküls von außerhalb einer Zelle in das Innere besagter Zelle, bevorzugt in das Zytosol besagter Zelle, umfassend die Schritte von:
a) Bereitstellen einer Zelle;
b) Bereitstellen des Moleküls zum Transferieren von außerhalb der Zelle in das Innere der in Schritt a) bereitgestellten Zelle;
c) Bereitstellen des Saponinderivats nach einem der Ansprüche 1-10;
d) In-Kontakt-bringen der Zelle aus Schritt a) *in vitro* oder ex *vivo* mit dem Molekül aus Schritt b) und dem Saponin Derivat aus Schritt c), dabei den Transfer des Moleküls von außerhalb der Zelle in die besagte Zelle herstellend.

21. Verfahren nach Anspruch 20, wobei die Zelle eine menschliche Zelle, wie etwa eine T-Zelle, eine NK-Zelle, eine Tumorzelle, ist und/oder wobei das Saponinderivat das Saponinderivat nach einem der Ansprüche 5-10 ist, und/oder wobei das Molekül aus Schritt b) eines ist aus: einem Antikörper-Arzneimittel-Konjugat, einem Rezeptor-Ligand-Arzneimittel-Konjugat, einem Antikörper-Oligonukleotid-Konjugat oder einem Rezeptor-Ligand-Oligonukleotid-Konjugat, wobei das Arzneimittel zum Beispiel ein Toxin ist und wobei das Oligonukleotid zum Beispiel eine siRNA oder eine BNA ist.

## Revendications

1. Dérivé de saponine basé sur une saponine SO1861 comprenant une structure de base aglycone triterpénique et une première chaîne saccharidique et une deuxième chaîne saccharidique liées à la structure de base aglycone, où le dérivé de saponine est un dérivé de SO1861 ayant une structure de base aglycone d'acide quillaïque, où le dérivé de saponine correspond à la saponine représentée par la Molécule 1 : 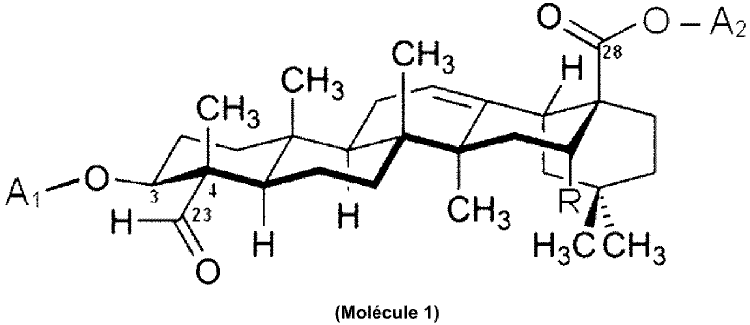
où la structure de base aglycone de la Molécule 1 est l'acide quillaïque et R est défini comme un hydroxyle ;
où la première chaîne saccharidique A1 est Gal-(1→2)-[Xyl-(1→3)]-GlcA- et la deuxième chaîne saccharidique A2 est Glc-(1→3)-Xyl-(1→4)-Rha-(1→2)-[Xyl-(1→3)-4-OAc-Qui-(1→4)]-Fuc- ; et où :
i. le dérivé de saponine comprend ladite structure de base aglycone comprenant un groupe aldéhyde en position C23 de l'acide quillaïque qui a été dérivatisé ; ou
ii. la première chaîne saccharidique A1 comprend un groupe carboxyle d'un fraction acide glucuronique qui a été dérivatisé ; ou
iii. la deuxième chaîne saccharidique A2 comprend un groupe acétoxy (Me(CO)O-) qui a été dérivatisé ; ou
iv. le dérivé de saponine comprend toute combinaison des dérivations i., ii. et iii., de préférence toute combinaison de deux dérivations i., ii. et iii.

2. Dérivé de saponine selon la revendication 1, où le dérivé de saponine comprend à la fois (ii.) ladite première chaîne saccharidique qui a été dérivatisée et (iii.) ladite deuxième chaîne saccharidique qui a été dérivatisée,
de préférence, le dérivé de saponine comprend à la fois ladite première chaîne saccharidique qui a été dérivatisée et ladite deuxième chaîne saccharidique qui a été dérivatisée et (i.) une structure de base aglycone étant l'acide quillaïque comprenant un groupe aldéhyde qui a été dérivatisé.

3. Dérivé de saponine selon la revendication 1 ou 2, où au moins l'une des dérivations suivantes est présente, de préférence une ou deux des dérivations suivantes est présente, plus préférablement une :
i. le groupe aldéhyde en position C23 de l'acide quillaïque a été dérivatisé par ;
- réduction en un alcool ;
- transformation en une liaison hydrazone, de préférence par réaction avec un hydrazide ;
ii. le groupe carboxyle du fraction acide glucuronique a été dérivatisé par transformation en une liaison amide, de préférence par réaction avec une amine ; et
iii. le groupe acétoxy a été dérivé par transformation en un groupe hydroxyle (HO-) par désacétylation.

4. Dérivé de saponine selon l'une quelconque des revendications 1 à 3, où le dérivé de saponine a un poids moléculaire inférieur à 2500 g/mol, de préférence inférieur à 2300 g/mol, plus préférablement inférieur à 2150 g/mol.

5. Dérivé de saponine selon l'une quelconque des revendications 1 à 4, où au moins l'une des dérivations suivantes est présente, de préférence une ou deux des dérivations suivantes est présente :
i. le groupe aldéhyde en position C₂₃ de l'acide quillaïque a été dérivatisé par ;
- réduction en un alcool ;
- transformation en une liaison hydrazone par réaction avec de l'hydrazide de l'acide N-ε-maléimidocaproïque (EMCH), donnant ainsi SO1861-Ald-EMCH, où le groupe maléimide de l'EMCH est éventuellement dérivatisé par formation d'une liaison thioéther avec du mercaptoéthanol ;
- transformation en une liaison hydrazone par réaction avec de l'hydrazide de l'acide N-[β-maléimidopropionique] (BMPH), où le groupe maléimide du **BMPH** est éventuellement dérivé par formation d'une liaison thioéther avec du mercaptoéthanol ; ou
- transformation en une liaison hydrazone par réaction avec de l'hydrazide de l'acide N-[κ-maléimidoundécanoïque] (KMUH), où le groupe maléimide du **KMUH** est éventuellement dérivatisé par formation d'une liaison thioéther avec du mercaptoéthanol ;
ii. le groupe carboxyle dudit fraction acide glucuronique de la première chaîne saccharidique a été dérivatisé par transformation en une liaison amide par réaction avec du 2-amino-2-méthyl-1,3-propanediol (AMPD) ou du *N-(2-*aminoéthyl)maléimide (AEM), fournissant ainsi SO1861-Glu-AMPD ou SO1861-Glu-AEM ; et
iii. le groupe acétoxy de la deuxième fraction saccharidique a été dérivatisé par transformation en un groupe hydroxyle (HO-) par désacétylation.

6. Dérivé de saponine de l'une quelconque des revendications 1 à 5, où le dérivé de saponine est un dérivé de SO1861 comprenant une dérivatisation unique, où la dérivatisation unique est la transformation d'un groupe carboxyle d'un fraction acide glucuronique de SO1861 par liaison de 1-[Bis(diméthylamino)méthylène]-1H-1,2,3-triazolo[4,5-b]pyridinium 3-oxid hexafluorophosphate (HATU) au groupe carboxyle du fraction acide glucuronique de SO1861 ou par liaison de (benzotriazol-1-yloxy)tris(diméthylamino)phosphonium hexafluorophosphate (BOP) au groupe carboxyle du fraction acide glucuronique de SO1861, ou où le dérivé de saponine est un dérivé de SO1861 représenté par la Molécule 2, qui représente un dérivé de SO1861 comprenant un groupe aldéhyde à la position indiquée C₂₃ de la structure de base aglycone d'acide quillaïque qui a été dérivatisé par transformation en une liaison hydrazone par réaction avec de l'hydrazide de l'acide N-ε-maléimidocaproïque (EMCH) : 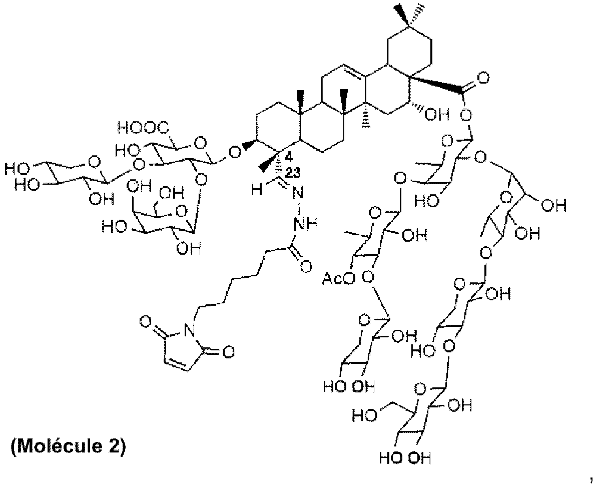 ou où le dérivé de saponine est un dérivé de SO1861 représenté par la Molécule 3, qui représente un dérivé de SO1861 comprenant un groupe aldéhyde à la position indiquée C₂₃ de la structure de base aglycone d'acide quillaïque qui a été dérivatisé par transformation en une liaison hydrazone par réaction avec de l'hydrazide de l'acide N-ε-maléimidocaproïque (EMCH) où le groupe maléimide de l'EMCH est dérivatisé avec du mercaptoéthanol, formant ainsi une liaison thioéther :

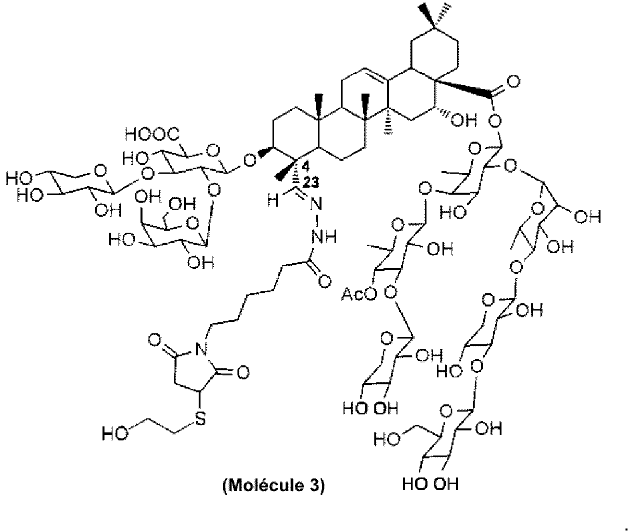

7. Dérivé de saponine selon l'une quelconque des revendications 1 à 6, où
i. le dérivé de saponine comprend ladite structure de base aglycone où la structure de base aglycone comprend un groupe aldéhyde qui a été dérivatisé par :
- réduction en un alcool ;
- transformation en une liaison hydrazone par réaction avec de l'hydrazide de l'acide N-ε-maléimidocaproïque (EMCH), où le groupe maléimide de l'EMCH est éventuellement dérivatisé par formation d'une liaison thioéther avec du mercaptoéthanol ;
- transformation en une liaison hydrazone par réaction avec de l'hydrazide de l'acide N-[β-maléimidopropionique] (BMPH) où le groupe maléimide du **BMPH** est éventuellement dérivatisé par formation d'une liaison thioéther avec du mercaptoéthanol ; ou
- transformation en une liaison hydrazone par réaction avec de l'hydrazide de l'acide N-[κ-maléimidoundécanoïque] (KMUH) où le groupe maléimide du **KMUH** est éventuellement dérivatisé par formation d'une liaison thioéther avec du mercaptoéthanol ;
ii. la première chaîne saccharidique comprend ledit groupe carboxyle, de préférence un groupe carboxyle d'un fraction acide glucuronique, qui a été dérivatisé par transformation en une liaison amide par réaction avec du 2-amino-2-méthyl-1,3-propanediol (AMPD) ou du N-(2-aminoéthyl)maléimide (AEM) ;
iii. la deuxième chaîne saccharidique comprend ledit groupe acétoxy (Me(CO)O-) qui a été dérivatisé par transformation en un groupe hydroxyle (HO-) par désacétylation ; ou
iv. le dérivé de saponine comprend toute combinaison de deux ou trois dérivations i., ii. et iii., de préférence toute combinaison de deux dérivations i., ii. et iii. ;
de préférence, le dérivé de saponine comprend ladite structure de base aglycone où la structure de base aglycone comprend un groupe aldéhyde qui a été dérivatisé par transformation en une liaison hydrazone par réaction avec de l'EMCH où le groupe maléimide de l'EMCH est éventuellement dérivatisé par formation d'une liaison thioéther avec du mercaptoéthanol.

8. Dérivé de saponine selon la revendication 7, où le dérivé de saponine comprend ladite structure de base aglycone où la structure de base aglycone comprend un groupe aldéhyde et où la première chaîne saccharidique comprend un groupe carboxyle, de préférence un groupe carboxyle d'un fraction acide glucuronique, qui a été dérivatisé par transformation en une liaison amide par réaction avec du N-(2-aminoéthyl)maléimide (AEM).

9. Dérivé de saponine selon la revendication 8, à condition que, lorsque le groupe aldéhyde dans la structure de base aglycone est dérivatisé par transformation en une liaison hydrazone par réaction avec de l'hydrazide de l'acide N-ε-maléimidocaproïque (EMCH), au moins l'un entre l'acide glucuronique et le groupe acétoxy (Me(CO)O-) soit également dérivatisé, et à condition que, lorsque le fraction acide glucuronique de SO1861 est dérivatisé par réaction de 1-[Bis(diméthylamino)méthylène]-1H-1,2,3-triazolo[4,5-b]pyridinium 3-oxid hexafluorophosphate (HATU) avec le groupe carboxyle du fraction acide glucuronique de SO1861, au moins l'un entre le groupe aldéhyde et le groupe acétoxy (Me(CO)O-) soit également modifié.

10. Dérivé de saponine de la revendication 9, à condition que, lorsque le groupe aldéhyde dans la structure de base aglycone du dérivé de saponine est dérivatisé par réaction avec de l'EMCH, au moins l'un entre l'acide glucuronique et le groupe acétoxy (Me(CO)O-) soit également dérivatisé, et à condition que, lorsque le groupe carboxyle du fraction acide glucuronique de SO1861 est dérivatisé par HATU lié, au moins l'un entre le groupe aldéhyde et le groupe acétoxy (Me(CO)O-) soit également dérivatisé.

11. Première composition pharmaceutique comprenant le dérivé de saponine selon l'une quelconque des revendications 1 à 10 et éventuellement un excipient et/ou un diluant pharmaceutiquement acceptable.

12. Première composition pharmaceutique de la revendication 11, où le dérivé de saponine est le dérivé de saponine représenté par la Molécule 2 : 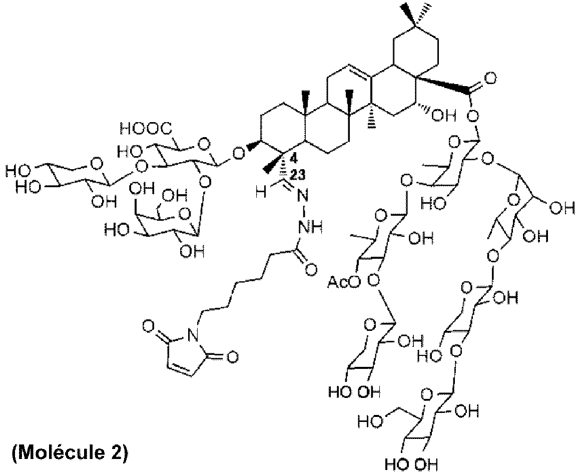 ou le dérivé de SO1861 comprenant une dérivatisation unique, où la dérivatisation unique est la transformation du groupe carboxyle du fraction acide glucuronique de SO1861 par réaction du 1-[Bis(diméthylamino)méthylène]-1H-1,2,3-triazolo[4,5-b]pyridinium 3-oxid hexafluorophosphate (HATU) avec le groupe carboxyle du fraction acide glucuronique de la SO1861, ou le dérivé de saponine est le dérivé de saponine représenté par la Molécule 3 :

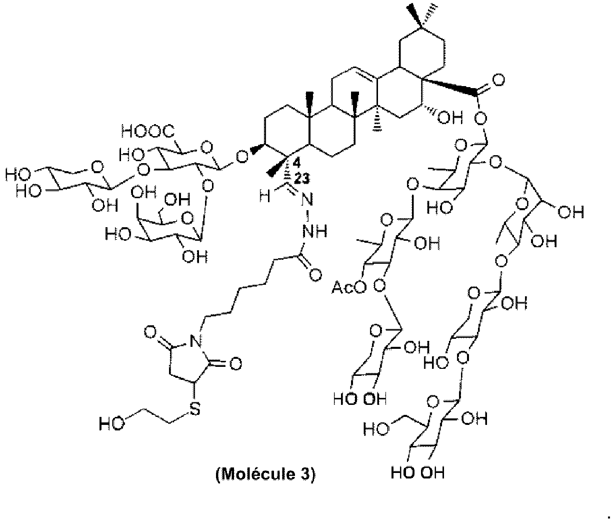

13. Combinaison pharmaceutique comprenant :
• la première composition pharmaceutique de la revendication 11 ou 12 ; et
• une deuxième composition pharmaceutique comprenant un quelconque ou plusieurs parmi un conjugué anticorps-toxine, un conjugué récepteur-ligand - toxine, un conjugué anticorps-médicament, un conjugué récepteur-ligand - médicament, un conjugué anticorps-oligonucléotide ou un conjugué récepteur-ligand - oligonucléotide, et comprenant éventuellement un excipient et/ou un diluant pharmaceutiquement acceptable.

14. Troisième composition pharmaceutique comprenant le dérivé de saponine de l'une quelconque des revendications 1 à 10 et comprenant en outre un quelconque ou plusieurs parmi : un conjugué anticorps-toxine, un conjugué récepteur-ligand - toxine, un conjugué anticorps-médicament, un conjugué récepteur-ligand - médicament, un conjugué anticorps-acide nucléique ou un conjugué récepteur-ligand - acide nucléique, et comprenant éventuellement un excipient et/ou un diluant pharmaceutiquement acceptable.

15. Combinaison pharmaceutique de la revendication 13 ou troisième composition pharmaceutique de la revendication 14, où la deuxième composition pharmaceutique ou la troisième composition pharmaceutique comprend un quelconque ou plusieurs parmi un conjugué anticorps-médicament, un conjugué récepteur-ligand - médicament, un conjugué anticorps-oligonucléotide ou un conjugué récepteur-ligand - oligonucléotide, où le médicament est par exemple une toxine telle que la saporine et la dianthine, et où l'oligonucléotide est par exemple un siRNA ou un BNA, par exemple pour le silençage génique de l'apolipoprotéine B ou de la HSP27.

16. Combinaison pharmaceutique de la revendication 13 ou 15 ou troisième composition pharmaceutique de la revendication 14 ou 15, où le conjugué anticorps-médicament est un conjugué d'un anticorps tel qu'une IgG, un Fab, un scFv, une immunoglobuline, un fragment d'immunoglobuline, un ou plusieurs domaines VH, des anticorps à domaine unique, un V_{HH} ou un V_{H} de camélidés.

17. Combinaison pharmaceutique de la revendication 13 ou 15 et 16 ou troisième composition pharmaceutique de l'une quelconque des revendications 14 à 16, où le dérivé de saponine est représenté par la Molécule 2 ou la Molécule 3.

18. Première composition pharmaceutique de la revendication 11 ou 12, combinaison pharmaceutique de l'une quelconque des revendications 13 ou 15 à 17 ou troisième composition pharmaceutique de l'une quelconque des revendications 14 à 17, pour utilisation comme médicament.

19. Première composition pharmaceutique de la revendication 11 ou 12, combinaison pharmaceutique de l'une quelconque des revendications 13 ou 15 à 17 ou troisième composition pharmaceutique de l'une quelconque des revendications 14 à 17, pour utilisation dans le traitement ou la prophylaxie d'un cancer, d'une maladie infectieuse, d'une infection virale, de l'hypercholestérolémie, de l'hyperoxalurie primaire, de l'hémophilie A, de l'hémophilie B, d'une maladie hépatique liée à l'alpha-1-antitrypsine, de la porphyrie hépatique aiguë, de l'amylose à transthyrétine ou d'une maladie auto-immune.

20. Procédé *in vitro* ou *ex vivo* pour transférer une molécule de l'extérieur d'une cellule à l'intérieur de ladite cellule, de préférence dans le cytosol de ladite cellule, comprenant les étapes consistant à :
a) fournir une cellule ;
b) fournir la molécule à transférer de l'extérieur de la cellule à l'intérieur de la cellule fournie à l'étape a) ;
c) fournir un dérivé de saponine selon l'une quelconque des revendications 1 à 10 ;
d) mettre en contact la cellule de l'étape a) *in vitro* ou *ex vivo* avec la molécule de l'étape b) et le dérivé de saponine de l'étape c), établissant ainsi le transfert de la molécule de l'extérieur de la cellule à l'intérieur de ladite cellule.

21. Procédé de la revendication 20, où la cellule est une cellule humaine telle qu'une cellule T, une cellule NK, une cellule tumorale et/ou où le dérivé de saponine est le dérivé de saponine de l'une quelconque des revendications 5 à 10, et/ou où la molécule de l'étape b) est l'une quelconque parmi: un conjugué anticorps-médicament, un conjugué récepteur-ligand - médicament, un conjugué anticorps-oligonucléotide ou un conjugué récepteur-ligand - oligonucléotide, où le médicament est par exemple une toxine et où l'oligonucléotide est par exemple un siRNA ou un BNA.
